# EUROPÄISCHE PATENTSCHRIFT

(11) **EP 1 730 049 B1**
(45) Veröffentlichungstag und Bekanntmachung des Hinweises auf die Patenterteilung: **12.09.2018**
(21) Anmeldenummer: 04720866.5
(22) Anmeldetag: 16.03.2004
(51) Int. Cl.: B65D 65/46, A61K 8/00, B65D 65/00, A61K 8/02, B01F 13/00, B01F 15/00, B65D 81/32, B65D 47/10, A61Q 5/08, A61Q 5/10, B01F 15/02

(54) **KOSMETISCHE PORTION**
COSMETIC PORTION
DOSE COSMETIQUE

(30) Priorität: 03.03.2004 DE 102004010975
(43) Veröffentlichungstag der Anmeldung: 13.12.2006
(73) Patentinhaber: Henkel AG & Co. KGaA, 40589 Düsseldorf (DE)
(72) Erfinder: SEILER, Martina, 47228 Duisburg (DE); SCHMIEDEL, Peter, 40599 Düsseldorf (DE); PAULI, Kristin, 42119 Wuppertal (DE); BERNECKER, Ullrich, 52393 Hürtgenwald (DE); VELDMAN, Gerard, 41372 Niederkrüchten (DE)
(86) Internationale Anmeldenummer: PCT/EP2004/002693
(87) Internationale Veröffentlichungsnummer: WO 2005/085089

(56) Entgegenhaltungen:
- EP-A- 0 479 404
- GB-A- 666 244
- GB-A- 2 118 961
- GB-A- 2 356 842
- US-A- 5 063 057
- US-A- 5 116 388
- US-A- 5 270 054
- US-A- 5 484 598

## Beschreibung

Die Erfindung betrifft eine Portion umfassend eine kosmetische Zubereitung wasserlöslicher und/oder wasserdispergierbarer Umhüllung, ein Verfahren zur Herstellung solcher Portionen, ein Kit, enthaltend eine oder mehrere Portionen, ein Verfahren zum Blondieren oder Färben keratiner Fasern, die Verwendung einer Portion zur Behandlung keratiner Fasern sowie die Verwendung einer wasserlöslichen und/oder wasserdispergierbaren Umhüllung zur Portionierung kosmetischer Zubereitungen.

Auf dem Gebiet der Kosmetik besteht ein großer Bedarf an Produkten die einerseits effektiv wirksam sein sollen und andererseits für den Verbraucher einfach und vor allem gefahrlos handhabbar und anwendbar sein sollen. Insbesondere auf dem Gebiet der Haarkosmetik haben sich in den vergangenen Jahren Blondier- und Haarfärbesysteme entwickelt, die äußerst effektiv wirken, jedoch bei unsachgemäßer Handhabung, beispielsweise bei Kontaminierung mit Hautpartien oder Augen zu Irritationen oder in Extremfällen sogar zum Auslösen von Allergien führen können. Es bestand daher ein großer Bedarf, die Sicherheit der Handhabung solcher kosmetischer Formulierungen zu gewährleisten und darüber hinaus dem Verbraucher ein einfach zu dosierendes Verpackungssystem an die Hand zu geben, das auch ein Anmischen bzw. eine Zusammenstellung der benötigten Komponenten vor Ort beim Verbraucher ermöglicht.

Im Stand der Technik werden in wasserlösliche Beutel-verpackte Haarkosmetikformulierungen bereits offenbart. DE 196 13 941 A1 beschreibt ein Verfahren zur Herstellung von nichtstaubenden pulverförmigen Mitteln zum Blondieren von menschlichen Haaren. Die Blondiermittel weisen mindestens eine Peroxidverbindung auf, die mit geeigneten Verdickungsmitteln versetzt und anschließend für den Transport und die Weiterverarbeitung portionsweise in wasserlösliche Beutel verpackt werden.

EP-A1-1037589 offenbart ein Mittel zur Behandlung keratinischer Fasern, bestehend aus mindestens einer wässerigen Zubereitung A und mindestens einer davon räumlich getrennt vorliegenden Zubereitung B, die einen in der Zubereitung A nicht lagerstabilen Bestandteil, ausgewählt aus der Gruppe, die von Parfümölen sowie Vitaminen, Provitaminen und deren Derivaten gebildet wird, enthält, wobei die Umhüllung der Zubereitung B aus einem Material, das bei Zugabe der Zubereitung B zu einer Zubereitung A eine Vermischung der Komponenten beider Zubereitungen bei 38 °C innerhalb von 5 Minuten ermöglicht.

US 5,116,388 offenbart Haarfärbemittel auf Basis von Oxidationsfarbstoffen sowie Bleichmittel zum Blondieren von Haaren, die in Polyvinylalkohol-Verpackungen eingearbeitet werden, um die durch Pulverstaub verursachten Irritationen zu vermindern.

Die im Stand der Technik offenbarten portionierten kosmetischen Formulierungen bieten zwar eine verbesserte Handhabbarkeit und eine Verringerung der Staubkontamination der verpackten kosmetischen Zubereitungen, jedoch weisen die in wasserlöslichen Foliensystemen verpackten Portionen den Nachteil auf, dass sie sich in Wasser nur langsam lösen. Darüber hinaus weisen die im Stand der Technik offenbarten wasserlöslichen Kosmetikportionen, insbesondere auf dem Gebiet der Haarkosmetik, den Nachteil auf, dass die Portionen den Verbrauchern aus den Händen gleiten können und gegebenenfalls dabei zerbersten. Nicht selten haben Verbraucher, die haarkosmetische Produkte anwenden, feuchte Hände bzw. Finger, beispielsweise deshalb, weil die Haare kurz vor der Anwendung gewaschen wurden, was die Gefahr des Abrutschens der Kosmetikportionen extrem erhöht. Viele der im Stand der Technik offenbarten kosmetischen Einzelportionen werden häufig in einer weiteren wasserundurchlässigen Umverpackung in den Handel gebracht. Die Umverpackungen bestehen dabei häufig aus glatten Folienbeuteln oder metallbeschichteten glatten Verpackungssystemen, so dass die im Stand der Technik beschriebenen wasserlöslichen PVA-Kosmetikportionen plan auf den Oberflächen der Umverpackungsmaterialien anliegen und durch hohe Adhäsionskräfte nicht ohne Weiteres aus dem Umverpackungsbehältnis gleiten.

Aufgabe der vorliegenden Erfindung ist es, portionierte kosmetische Zubereitung in wasserlöslicher und/oder wasserdispergierbarer Umhüllung bereitzustellen, die die vorgenannten Probleme des Standes der Technik nicht aufweisen.

Es wurde gefunden, dass die vorgenannten Probleme durch die spezielle Ausgestaltung einer Portion gelöst werden.

Gegenstand der vorliegenden Erfindung ist somit eine Portion umfassend eine kosmetische Zubereitung und eine wasserlösliche und/oder wasserdispergierbare Umhüllung, wobei die Umhüllung die kosmetische Zubereitung umhüllt und die Oberfläche der Umhüllung einen quadratischen Mittelwert der Rauheit von wenigstens 10 µm aufweist, wobei sowohl Innenals auch Außenseite der Umhüllung ein durch Erhitzen und Pressen aufgeprägtes, dreidimensionales Muster aufweisen.

Die erfindungsgemäßen Portionen weisen kosmetische Zubereitungen auf, die in wasserlöslichen und/oder wasserdispergierbaren Umhüllungen umhüllt sind. Bevorzugt sind jedoch in Wasser vollständig lösliche Umhüllungen. Im Rahmen der vorliegenden Erfindung wird der Begriff "Portion" oder "Kosmetikportion" synonym mit dem Begriff "portionierte kosmetische Zubereitung in wasserlöslicher und/oder wasserdispergierbarer Umhüllung verwandt. Die erfindungsgemäßen Portionen weisen eine Umhüllung auf, die bevorzugt einen quadratischen Mittelwert der Rauheit von wenigstens 10 µm aufweist. Vorzugsweise zeigt die Oberfläche der Umhüllung einen quadratischen Mittelwert der Rauheit von 10 bis 100 µm, besonders bevorzugt von 10 bis 50 µm und insbesondere von 30 bis 35 µm auf. Der Begriff "Oberfläche" bezeichnet im Rahmen dieser Erfindung die flächigen Bereiche der Umhüllung, also nicht die Schnittflächen einer Umhüllung, beispielsweise einer Polymerfolie.

Die Bestimmung des quadratischen Mittelwerts der Rauheit der Umhüllung erfolgte gemäß DIN 4762/1 mit handelsüblichen Oberflächenabtastgeräten.

Kommerziell erhältlich sind Umhüllungsmaterialien auf Basis von Polyvinylalkohol, beispielsweise als Polymerfolien, die die oben angegebenen Rauheitswerte aufweisen, von der Firma Syntana unter dem Markennamen Solublon® PVAL-Folie, Typ SA 20.

Dadurch, dass die erfindungsgemäß einzusetzenden Umhüllungen im Vergleich zu den im Stand der Technik auf diesem Gebiet eingesetzten Umhüllungen eine wesentlich rauere Oberfläche aufweisen, vergrößert sich auch die dreidimensionale makroskopische Oberfläche der Umhüllung. Die dreidimensionale makroskopische Oberfläche berücksichtigt zusätzlich die Flächen, die sich durch Unregelmäßigkeiten auf der Folienoberfläche aufspannen. Für den Fall einer ideal glatten Oberfläche entspricht die dreidimensional makroskopische Oberfläche der zweidimensionalen geometrischen Oberfläche. In einer bevorzugten Ausführungsform der vorliegenden Erfindung ist die dreidimensionale makroskopische Oberfläche der Umhüllung der Portion mindestens 10 %, bevorzugt mindestens 20 %, weiter bevorzugt zwischen 20 und 100 %, äußerst bevorzugt zwischen 30 und 50 % größer als die zweidimensionale geometrische Oberfläche.

Gegenstand der vorliegenden Erfindung sind somit Portionen umfassend eine kosmetische Zubereitung und eine wasserlösliche und/oder wasserdispergierbare Umhüllung, wobei die Umhüllung die kosmetische Zubereitung umhüllt und die dreidimensionale makroskopische Oberfläche der Umhüllung der Portion mindestens 10 %, bevorzugt mindestens 20 %, weiter bevorzugt zwischen 20 und 100 %, äußerst bevorzugt zwischen 30 und 50 % größer als die zweidimensionale geometrische Oberfläche.
Die Bestimmung der dreidimensionalen Oberfläche wird ausgehend von der Bezugsoberfläche bestimmt, die die Form der geometrischen Oberfläche hat und in ihrer Lage im Raum mit der Hauptrichtung der wirklichen Oberfläche übereinstimmt.

Die im Vergleich zur zweidimensionalen geometrischen Oberfläche vergrößerte dreidimensionale makroskopische Oberfläche trägt unter anderem zu einer verbesserten Wasserlöslichkeit der erfindungsgemäßen Portionen bei.

Die Außen- und Innenfläche der Umhüllung können dabei zu mindestens 50 %, vorzugsweise mindestens 70 %, weiter bevorzugt mindestens zu 90 % und insbesondere im Wesentlichen vollständig mit einem dreidimensionalen, aufgeprägtenMuster versehen sein.

Im Rahmen der vorliegenden Erfindung bezeichnet die Innenfläche der Umhüllung den flächigen Bereich, der mit der kosmetischen Zubereitung in Kontakt treten kann. Bei Vorliegen eines Folienbeutels ist somit die Oberfläche der Folie im Beutelinneren die Innenfläche und die Folienoberfläche außerhalb des Beutelinneren die Außenfläche. Die Außenfläche steht nicht mit der kosmetischen Zubereitung der erfindungsgemäßen Portion in Kontakt.

Bei dem auf einer Oberfläche der Umhüllung aufgeprägten Muster handelt es sich in einer bevorzugten Ausführungsform um ein regelmäßiges Musters.

Die aufgeprägte Struktur weist auf der Oberfläche in einer bevorzugten Ausführungsform ein regelmäßiges dreidimensionales Muster auf. Das regelmäßige Muster kann dabei jegliche erdenkliche Form aufweisen, beispielsweise Karos, Rauten, eingestanzte Zylinder, Ovale, etc. In einer bevorzugten Ausführungsform besteht das regelmäßige Muster in einer periodisch wiederkehrenden Anordnung von Erhöhungen und Vertiefungen der Umhüllungsoberfläche.

Das aufgeprägte Muster kann dabei sowohl die haptischen Eigenschaften der erfindungsgemäßen Portion als auch dessen Auflösegeschwindigkeit beeinflussen. Es hat sich daher als vorteilhaft erwiesen, dass das aufgeprägte Muster mindestens 4, vorzugsweise mindestens 6, besonders bevorzugt zwischen 8 und 50, weiter bevorzugt zwischen 10 und 25 Vertiefungen oder Erhöhungen pro 1 cm² der zweidimensionalen Oberfläche aufweist. Aufgeprägte Vertiefungen oder Erhöhungen weisen im Rahmen der vorliegenden Erfindung in ihrer größten Ausdehnung mindestens einen Durchmesser von 2 µm und eine Tiefe bzw. Höhe von mindestens 2 µm vorzugsweise mindestens 5 µm auf.

In einer weiteren bevorzugten Ausführungsform weist die Oberfläche der Umhüllung kreisförmige und/oder dreieckige und/oder viereckige und/oder mehreckige Vertiefungen auf.

Die Oberflächen der Umhüllungen können aber auch in einer weiteren Ausführungsform quaderförmige, runde, eckige, ovale, sägezahnförmige oder dreieckig spitz zur Oberfläche laufende Erhöhungen aufweisen.

In einer bevorzugten Ausführungsform weist die Oberfläche der Umhüllung ein gitterförmiges oder wabenförmiges dreidimensionales strukturiertes Muster auf. Diese Muster sind vorzugsweise aufgeprägt oder auf die Hülloberfläche aufgestanzt und verleihen somit der Oberfläche ein dreidimensionales Profil. In einer bevorzugten Ausführungsform weist die Umhüllung durch Aufprägen eines gitterförmigen oder wabenförmigen Musters Gitternetzlinien auf. Bevorzugt werden die Gitternetzlinien durch eine Aneinanderreihung von die Vertiefungen begrenzenden Rändern gebildet.

Die bevorzugten gitterförmigen Muster werden vorzugsweise derart auf die Oberflächen der Umhüllungen aufgeprägt, so dass das Verhältnis der durchschnittlichen Breite von Gitternetzlinie zur maximalen Ausdehnung der Ebene der Vertiefung kleiner 20:1, vorzugsweise kleiner 10:1, besonders bevorzugt kleiner 1:1, weiter bevorzugt kleiner 0,5:1 und insbesondere kleiner 0,25:1 ist.

Insbesondere bei aufgeprägten Mustern hat es sich als vorteilhaft herausgestellt, dass das Verhältnis von durchschnittlichem Durchmesser der Vertiefung zur Tiefe der Vertiefung unterhalb 20:1, vorzugsweise 10:1 bis 1:10, insbesondere 8:1 bis 1:1, im Speziellen 6:1 bis 4:1 beträgt.

Vorzugsweise weisen die erfindungsgemäßen Portionen Umhüllungen auf, deren mittlere Stärke 10 bis 100 µm, vorzugsweise 15 bis 50 µm und insbesondere 20 bis 40 µm beträgt. Die gewählten Umhüllungsstärken tragen insbesondere, wenn es sich bei den Umhüllungen um wasserlösliche und/oder wasserdispergierbare Folien handelt, zu einer optimalen Wasserauflösegeschwindigkeit und zudem zu einer guten Verarbeitbarkeit der Folien bei. So hat sich gezeigt, dass insbesondere in dem Bereich von einer mittleren Folienstärken zwischen 10 und 100 µm das thermische Versiegeln, insbesondere flüssigkeitsdichtes Versiegeln, problemlos durchgeführt werden kann. Die Folienstärke kann sich dabei auf Teilbereiche oder bevorzugt auf das gesamte Hüllmaterial beziehen. Die mittlere Folienstärke bezieht sich auf das Querschnittsprofil und wurde über die Erhöhungen und Vertiefungen entlang einer 1 cm langen Profilstrecke gemittelt. Ein 1 Quadratzentimeter großes Folienstück (1 cm x 1 cm) des Hüllmaterials wird in 5 äquidistante Streifen (jeweils 2 mm) zerlegt und jeweils die mittlere Folienstärke entlang des Profils ermittelt. Der Mittelwert aus den 5 Messungen bildet die mittlere Folienstärke. Die Bestimmung der mittleren Folienstärke entlang des Querschnittprofils mittels Videolichtmikroskopie bestimmt.

Das Material der wasserlöslichen und/oder wasserdispergierbaren Umhüllung der erfindungsgemäßen Portionen besteht in einer bevorzugten Ausführungsform ganz oder teilweise aus einem Thermoplast, ausgewählt aus der Gruppe umfassend Polyvinylalkohol (PVA), acetalisierter Polyvinylalkohol, Polyvinylpyrrolidon, Polyethylenoxid, Gelatine, Cellulose, Stärke und Derivate der vorgenannten Stoffe und/oder Mischungen der vorgenannten Polymere, wobei Polyvinylalkohol besonders bevorzugt ist.
Die vorstehend beschriebenen Polyvinylalkohole sind kommerziell verfügbar, beispielsweise unter dem Warenzeichen Mowiol® (Clariant). Im Rahmen der vorliegenden Erfindung besonders geeignete Polyvinylalkohole sind beispielsweise Mowiol® 3-83, Mowiol® 4-88, Mowiol® 5-88, Mowiol® 8- 88 sowie Clariant L648.

Weitere als Material für die Umhüllung geeignete Polyvinylalkohole sind ELVANOL® 51-05, 52-22, 50-42, 85-82, 75-15, T-25, T-66, 90-50 (Warenzeichen der Du Pont), ALCOTEX® 72.5, 78, B72, F80/40, F88/4, F88/26, F88/40, F88/47 (Warenzeichen der Harlow Chemical Co.), Gohsenol® NK-05, A-300, AH-22, C-500, GH-20, GL-03, GM-14L, KA-20, KA-500, KH-20, KP-06, N- 300, NH-26, NM11Q, KZ-06 (Warenzeichen der Nippon Gohsei K. K.).

In einer weiteren bevorzugten Ausführungsform weist das Umhüllungsmaterial zusätzlich Polymere ausgewählt aus der Gruppe, umfassend Acrylsäure-haltige Polymere, Polyacrylamide, Oxazolin-Polymere, Polystyrolsulfonate, Polyurethane, Polyester, Polyether und/oder Mischungen der vorstehenden Polymere, auf.
Bevorzugt ist, wenn das Hüllmaterial der erfindungsgemäßen Portion ein teilacetalisierter Polyvinylalkohol mit einem Verseifungsgrad von 70 bis 100 Mol-%, vorzugsweise 80 bis 96 Mol-%, besonders bevorzugt 82 bis 94 Mol-% und insbesondere 85 bis 89 Mol-% ausmacht. Weiter bevorzugt ist, dass der verwendete wasserlösliche Thermoplast ein Polyvinylacetat umfasst, dessen mittleres Molekulargewicht im Bereich von 10000 bis 100000 gmol⁻¹, vorzugsweise von 11000 bis 90000 gmol⁻¹, besonders bevorzugt von 12000 bis 80000 gmol⁻¹, insbesondere von 13000 bis 70000 gmol⁻¹ und im Speziellen von 20000 bis 40000 gmol⁻¹ ist. Die mittleren Molekulargewichte wurden mittels Gelpermeationschromatographie bestimmt. Es wurde überraschend gefunden, dass über die spezielle Auswahl des Molekulargewichts die Auflösegeschwindigkeit erheblich verbessert werden kann.

In einer weiteren Ausführungsform, umfasst das Hüllmaterial die genannten Thermoplasten in Mengen von mindestens 50 Gew.-%, vorzugsweise von mindestens 70 Gew.-%, besonders bevorzugt von mindestens 80 Gew.-% und insbesondere von mindestens 90 Gew.-%, jeweils bezogen auf das Gewicht des gesamten Hüllmaterials.

Die erfindungsgemäßen Portionen können in unterschiedlichen Darbietungsformen vorliegen, die beispielsweise durch das Verfahren zur Herstellung der Portion vorgegeben ist. In bevorzugten Ausführungsformen weist die Umhüllung der erfindungsgemäßen Portionen die Form von Beutel, Kapsel, Spritzguss oder Tiefziehformkörper oder Blassformkörpers auf, besonders bevorzugt sind jedoch aus Polymerfolien hergestellte Beutel, insbesondere mittels eines Tiefziehverfahrens oder eines Schlauchbeutelsiegelverfahrens erhaltene Beutel.

Die erfindungsgemäßen Portionen weisen in einer bevorzugten Ausführungsform der vorliegenden Erfindung ein Innenvolumen von 5 bis 500 cm³, vorzugsweise von 10 bis 200 cm³, besonders bevorzugt von 20 bis 100 cm³ und insbesondere von 30 bis 70 cm³ auf. Insbesondere auf dem Gebiet der Haarbehandlungsmittel haben sich Portionsgrößen mit einem Innenvolumen von 30 bis 70 cm³ als besonders geeignet herausgestellt, da sie einerseits für den Endverbraucher einfach zu handhaben sind und andererseits durch das begrenzte Innenvolumen keine Probleme bezüglich der durch die Schwerkraft der kosmetischen Zubereitung bedingte Schäden an der Umhüllung auftreten.

Das Innenvolumen der Portion bezeichnet den Raum der Portion, der befähigt ist, die kosmetische Zubereitung aufzunehmen.

In einer weiteren Ausführungsform der vorliegenden Erfindung löst sich die Umhüllung in Wasser bei 20 °C innerhalb von weniger als 5 Minuten, vorzugsweise weniger als 4 Minuten und weiter bevorzugt weniger als 3 Minuten und insbesondere zwischen 2 und 0,5 Minuten in Wasser auf. Die Bestimmung der Auflösegeschwindigkeit erfolgte durch Zugabe von 0,07 g Hüllmaterial in ein Becherglas mit 7 ml Wasser. Das Wasser wurde während der Auflösungsphase mit Hilfe eines Magnetrührers gerührt (60 U/min). Die Auflösegeschwindigkeit wurde mittels optischer Methoden bestimmt, beginnend vom Zugabezeitpunkt des Hüllmaterials zur gerührten wässrigen Lösung bis zur vollständigen Auflösung (Trübungsmessung).

Einzeldosierungen werden von dem Verbraucher als bequem empfunden. Der Verbraucher nimmt das entsprechende Produkt, dosiert es und braucht sich über die Abmessung geeigneter Mengen keine Gedanken zu machen. Es kann jedoch Situationen geben, in denen diese Angebotsformen kritisch sind, da eine situationsabhängige Abänderung der Dosierung nicht mehr möglich ist und somit beispielsweise mit einer Dosiereinheit zu wenig, mit zwei Einheiten aber zuviel dosiert wird. Dieses Problem kann gelöst werden, in dem Mehrkammerbehälter zur Verfügung gestellt werden.

Ein Mehrkammerbehälter kann aus zwei oder mehrstückigen Einzelportionen, die über Stege miteinander verbunden sind, bestehen. Stege können dabei auch gemeinsame Siegelflächen zweier benachbarten Portionen sein.

Mehrkammerbehälter können derart vorliegen, dass sie zusammenhängend zwei oder drei Einzelportionen aufweisen, die bevorzugt jeweils unterschiedliche kosmetische Zubereitungen aufweisen. Ein Mehrkammersystem kann somit auch ein System sein, beispielsweise zweier Farbstoffkomponenten (Entwickler und Kuppler), die sich in einem Mehrkammerbehälter getrennt voneinander befinden. Bevorzugt weisen die Mehrkammerbehälter zwei oder mehr Kompartimente auf, in denen sich vorzugsweise unterschiedliche kosmetische Zubereitungen, insbesondere Haarfärbemittelkomponenten befinden. Solche Mehrkammerbehälter vereinfachen die Dosierung für den Verbraucher weiter, da in einem solchen Fall lediglich eine einzige Verpackungseinheit in der entsprechenden Anwendungslösung aufgelöst werden muss.

### Kosmetische Zubereitungen

An die Konsistenz der kosmetischen Zubereitung, die von der wasserlöslichen und oder wasserdispergierbaren Umhüllung umhüllt wird, werden keine besonderen Anforderungen gestellt. Bevorzugt liegen die kosmetischen Zubereitungen in Form von Pulvern, Pasten, Emulsionen oder Gelen vor.

Die erfindungsgemäßen Portionen haben sich als besonders vorteilhaft auf dem Gebiet der Haarbehandlungsmittel herausgestellt. Die kosmetischen Zubereitungen sind daher in einer bevorzugten Ausführungsform Haarbehandlungsmittel, insbesondere Blondier- oder Haarfärbemittel.

Der Wassergehalt der kosmetischen Zubereitung ist ein kritischer Wert und sollte daher vorzugsweise unterhalb von 20 Gew.-%, vorzugsweise unterhalb von 12 Gew.-%, besonders bevorzugt unterhalb von 8 Gew.-%, weiter bevorzugt unterhalb von 4 Gew.-%, insbesondere unterhalb von 2 Gew.-%, jeweils bezogen auf die gesamte kosmetische Zubereitung, liegen.

### Blondiermittel:

Die erfindungsgemäßen Portionen enthalten in einer bevorzugten Ausführungsform ein oder mehrere Blondiermittel als kosmetische Zubereitung.

Die Grundlagen der Blondierverfahren sind dem Fachmann bekannt und in einschlägigen Monographien, z.B. von K. Schrader, Grundlagen und Rezepturen der Kosmetika, 2. Auflage, 1989, Dr. Alfred Hüthig Verlag, Heidelberg, oder W. Umbach (Hrg.), Kosmetik, 2. Auflage, 1995, Georg Thieme Verlag, Stuttgart, New York, zusammenfassend beschrieben.

Zum Blondieren menschlicher Haare - insbesondere für die Strähnchenapplikation -werden üblicherweise feste oder pastenförmige Zubereitungen mit festen Oxidationsmitteln unmittelbar vor der Anwendung mit einer verdünnten Wasserstoffperoxidlösung vermischt. Diese Mischung wird dann auf das Haar aufgebracht und nach einer bestimmten Einwirkzeit wieder ausgespült.

Die genannten Zubereitungen, die vor der Anwendung üblicherweise mit einer Wasserstoffperoxidlösung vermischt werden, werden im weiteren als "Blondiermittel" bezeichnet. Alle aufgeführten Mengenangaben beziehen sich, soweit nicht anders ausgeführt, ausschließlich auf diese Zubereitungen und werden in Gewichtsprozent bezogen auf die Zubereitung angegeben.

Blondiermittel enthalten üblicherweise eine feste Peroxoverbindung. Die Auswahl dieser Peroxoverbindung unterliegt prinzipiell keinen Beschränkungen; übliche, dem Fachmann bekannte Peroxoverbindungen sind beispielsweise Ammoniumperoxidisulfat, Kaliumperoxidisulfat, Natriumperoxidisulfat, Ammoniumpersulfat, Kaliumpersulfat, Natriumpersulfat, Kaliumperoxidiphosphat, Percarbonate wie Magnesiumpercarbonat, Peroxide wie Bariumperoxid sowie Perborate, Harnstoffperoxid und Melaminperoxid. Unter diesen Peroxoverbindungen, die auch in Kombination eingesetzt werden können, sind erfindungsgemäß die anorganischen Verbindungen bevorzugt. Besonders bevorzugt sind die Peroxidisulfate, insbesondere Kombinationen aus mindestens zwei Peroxidisulfaten.

Die Peroxoverbindungen sind in den Blondiermitteln bevorzugt in Mengen von 20-80 Gew.-%, insbesondere in Mengen von 40-70 Gew.-%, jeweils bezogen auf das gesamte Blondiermittel, enthalten.

Bevorzugt enthalten die Blondiermittel ein Alkalisierungsmittel, das zur Einstellung des alkalischen pH-Wertes der Anwendungsmischung dient. Es können die dem Fachmann ebenfalls für Blondiermittel bekannten, üblichen Alkalisierungsmittel wie Ammonium-, Alkalimetall- und Erdalkalimetallhydroxyde, -carbonate, carbamate, -hydrogencarbonate, -hydroxycarbonate, -silikate, insbesondere -metasilikate, sowie Alkaliphosphate verwendet werden. In einer bevorzugten Ausführungsform enthalten die Blondiermittel mindestens zwei unterschiedliche Alkalisierungsmittel. Dabei können Mischungen beispielsweise aus einem Metasilikat und einem Hydroxycarbonat bevorzugt sein.

Die Blondiermittel enthalten Alkalisierungsmittel bevorzugt in Mengen von 10-30 Gew.-%, insbesondere 15-25 Gew.-%.

Weiterhin können Blondiermittel Amine und/oder Diamine, beispielsweise Monoethanolamin, Triethanolamin sowie das 2-Amino-2-methyl-1-propanol enthalten.

Weitere Amine sind ethoxylierte Kokosamine und Derivate des Sojaamins, insbesondere das PEG-3-Cocamin und das Dihydroxyethyl Soyamin-dioleat.

Weiterhin hat es sich als vorteilhaft erwiesen, wenn die Blondiermittel nichtionogene grenzflächenaktive Stoffe enthalten. Dabei sind solche grenzflächenaktive Stoffe, die einen HLB-Wert von 5,0 und größer aufweisen, bevorzugt. Für die Definition des HLB-Wertes wird ausdrücklich auf die Ausführungen in Hugo Janistyn, Handbuch der Kosmetika und Riechstoffe, III. Band: Die Körperpflegemittel, 2. Auflage, Dr. Alfred Hüthig Verlag Heidelberg, 1973, Seiten 68-78 und Hugo Janistyn, Taschenbuch der modernen Parfümerie und Kosmetik, 4. Auflage, Wissenschaftliche Verlagsgesellschaft m.b.H. Stuttgart, 1974, Seiten 466-474, sowie die darin zitierten Originalarbeiten Bezug genommen.

Besonders bevorzugte nichtionogene oberflächenaktive Substanzen sind dabei wegen der einfachen Verarbeitbarkeit Substanzen, die kommerziell als Feststoffe oder Flüssigkeiten in reiner Form erhältlich sind. Die Definition für Reinheit bezieht sich in diesem Zusammenhang nicht auf chemisch reine Verbindungen. Vielmehr können, insbesondere wenn es sich um Produkte auf natürlicher Basis handelt, Mischungen verschiedener Homologen eingesetzt werden, beispielsweise mit verschiedenen Alkylkettenlängen, wie sie bei Produkten auf Basis natürlicher Fette und Öle erhalten werden. Auch bei alkoxylierten Produkten liegen üblicherweise Mischungen unterschiedlicher Alkoxylierungsgrade vor. Der Begriff Reinheit bezieht sich in diesem Zusammenhang vielmehr auf die Tatsache, dass die gewählten Substanzen bevorzugt frei von Lösungsmitteln, Stellmitteln und anderen Begleitstoffen sein sollen.

Bevorzugte nichtionogene grenzflächenaktive Stoffe sind
- alkoxylierte Fettalkohole mit 8 bis 22, insbesondere 10 bis 16, Kohlenstoffatomen in der Fettalkylgruppe und 1 bis 30, insbesondere 1 bis 15, Ethylenoxid- und/oder Propylenoxid-Einheiten. Bevorzugte Fettalkylgruppen sind beispielsweise Lauryl-, Myristyl-, Cetyl-, aber auch Stearyl-, Isostearyl- und Oleylgruppen. Besonders bevorzugte Verbindungen dieser Klasse sind beispielsweise Laurylalkohol mit 2 bis 4 Ethylenoxid-Einheiten, Oleyl- und Cetylalkohol mit jeweils 5 bis 10 Ethylenoxideinheiten, Cetyl- und Stearylalkohol sowie deren Mischungen mit 10 bis 30 Ethylenoxideinheiten sowie das Handelsprodukt Aethoxal®B (Henkel), ein Laurylalkohol mit jeweils 5 Ethylenoxid- und Propylenoxideinheiten. Neben den üblichen alkoxylierten Fettalkoholen können auch sogenannte "endgruppenverschlossene" Verbindungen erfindungsgemäß eingesetzt werden. Bei diesen Verbindungen weist die Alkoxygruppe am Ende keine OH-Gruppe auf, sondern ist in Form eines Ethers, insbesondere eines C₁-C₄-Alkyl-Ethers, "verschlossen", Ein Beispiel für eine solche Verbindung ist das Handelsprodukt Dehypon®LT 054, ein C₁₂₋₁₈-Fettalkoholol + 4,5 Ethylenoxid-butylether.
- alkoxylierte Fettsäuren mit 8 bis 22, insbesondere 10 bis 16, Kohlenstoffatomen in der Fettsäuregruppe und 1 bis 30, insbesondere 1 bis 15, Ethylenoxid- und/oder Propylenoxid-Einheiten. Bevorzugte Fettsäuren sind beispielsweise Laurin-, Myristin-, Palmitin-, Stearin-, Isostearin- und Ölsäure.
- alkoxylierte, bevorzugt propoxylierte und insbesondere ethoxylierte, Mono-, Di- und Triglyceride. Beispiele für bevorzugte Verbindungen sind Glycerinmonolaurat + 20 Ethylenoxid und Glycerinmonostearat + 20 Ethylenoxid.
- Polyglycerinester und alkoxylierte Polyglycerinester. Bevorzugte Verbindungen dieser Klasse sind beispielsweise Poly(3)glycerindiisostearat (Handelsprodukt: Lameform®TGI (Henkel)) und Poly(2)glycerinpolyhydroxy-stearat (Handelsprodukt: Dehymuls®PGPH (Henkel)).
- Sorbitan-Fettsäureester und alkoxylierte Sorbitan-Fettsäureester wie beispielsweise Sorbitanmonolaurat und Sorbitanmonolaurat + 20 Ethylenoxid (EO).
- Alkylphenole und Alkylphenolalkoxylate mit 6 bis 21, insbesondere 6 bis 15, Kohlenstoffatomen in der Alkylkette und 0 bis 30 Ethylenoxid- und/oder Propylenoxid-Einheiten. Bevorzugte Vertreter dieser Klasse sind beipielsweise Nonylphenol + 4 EO, Nonylphenol + 9 EO, Octylphenol + 3 EO und Octylphenol + 8 EO.

Besonders bevorzugte Klassen an nichtionogenen grenzflächenaktiven Stoffen stellen die alkoxylierten Fettalkohole, die alkoxylierten Fettsäuren sowie die Alkylphenole und Alkylphenolalkoxylate dar.

Als besonders vorteilhaft haben sich Mittel erwiesen, die nichtionogene grenzflächenaktive Substanzen in Mengen von 0,5 - 10 Gew.-% enthalten.

Weiterhin können die Blondiermittel alle in solchen Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische und kationische Tenside geeignet sind. In vielen Fällen hat es sich aber als vorteilhaft erwiesen, die Tenside aus anionischen, kationischen oder nichtionischen Tensiden auszuwählen. Anionische Tenside können dabei ganz besonders bevorzugt sein.

Bevorzugte anionische Tenside sind Alkylsulfate, Ethercarbonsäuresalze mit 10 bis 18 C-Atomen in der Alkylgruppe und bis zu 12 Glykolethergruppen im Molekül wie C₁₂H₂₅-(C₂H₄O)₆-CH₂-COONa sowie insbesondere Salze von gesättigten und speziell ungesättigten C₃-C₂₂-Carbonsäuren wie Ölsäure, Stearinsäure, Isostearinsäure und Palmitinsäure.

Diese anionischen Tenside sollten bevorzugt in fester, insbesondere Pulverform vorliegen. Ganz besonders bevorzugt sind dabei bei Raumtemperatur feste Seifen, insbesondere Natriumstearat. Diese liegen bevorzugt in Mengen von 5 bis 20 Gew.-%, insbesondere 10 bis 15 Gew.-.%, vor.

Als nichtionische Tenside eignen sich insbesondere C₈-C₂₂-Alkylmono- und oligoglycoside und deren ethoxylierte Analoga. Insbesondere die nichtethoxylierten Verbindungen, die zudem in Pulverform kommerziell erhältlich sind, haben sich als besonders geeignet erwiesen.

Beispiele für die in den Haarbehandlungsmitteln verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quaternisierten Proteinhydrolysate dar.

Alkylamidoamine, insbesondere Fettsäureamidoamine wie das unter der Bezeichnung Tego Amid®S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quaternäre Esterverbindungen, sogenannte "Esterquats", wie das unter der Bezeichnung Dehyquart®F 75 in Abmischung mit Cetearylalkohol erhältliche Distearoylethylhydroxyethylammoniummethosulfat.

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/VinylacetatCopolymere und Polysiloxane,
- kationische Polymere wie quaternisierte Celluloseether und andere, als Feststoff stabile bzw. im Handel erhältliche Verbindungen,
- zwitterionische und amphotere Polymere, die als Feststoffe stabil bzw. bevorzugt als Handelsprodukte erhältlich sind,
- anionische Polymere wie beispielsweise Polyacrylsäuren, vernetzte Polyacrylsäuren und Vinylacetat/Crotonsäure-Copolymere, sofern diese als Feststoffe stabil bzw. bevorzugt im Handel erhältlich sind,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Glucose, Maleinsäure und Milchsäure,
- haarkonditionierende Verbindungen wie Phospholipide, beispielsweise Sojalecithin, Ei-Lecitin und Kephaline, sowie Silikonöle
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Farbstoffe zum Einfärben der Zubereitungen,
- Wirkstoffe wie Panthenol, Pantothensäure, Allantoin, Pyrrolidoncarbonsäuren und deren Salze,
- Cholesterin,
- Fette und Wachse wie Walrat, Bienenwachs, Montanwachs, Paraffine,
- Fettalkohole und Fettsäureester,
- Fettsäurealkanolamide,
- Komplexbildner wie EDTA, NTA und Phosphonsäuren,
- Quell- und Penetrationshilfsstoffe wie Carbonate, Hydrogencarbonate, Guanidine, Harnstoffe sowie primäre, sekundäre und tertiäre Phosphate.

Die Auswahl dieser weiteren Stoffe wird der Fachmann gemäß der gewünschten Eigenschaften der Mittel treffen.

Die Herstellung der Blondiermittel kann nach den üblichen, dem Fachmann bekannten Verfahren erfolgen.

Ein Verfahren besteht darin, die anorganischen, als Feststoff vorliegenden Komponenten, gegebenenfalls nach Mischung z.B. in einem Drais-Mischer, vorzulegen und mit dem grenzflächenaktiven Mittel zu besprühen. Dies erfolgt bevorzugt bei Raumtemperatur, d. h. bei Temperaturen unterhalb von ca. 30 °C; lediglich wenn die gewählten staubbindenden Komponenten bei diesen Temperaturen nicht als Flüssigkeit vorliegen, wird man erhöhte Temperaturen anwenden.

Ein weiteres Herstellungsverfahren für die Blondiermittel ist das Vermahlen aller Komponenten in einer Kugelmühle, einer Ringwalzenmühle oder insbesondere einer Spindelmühle.

Schließlich ist es möglich, die pulverförmigen Blondiermittel durch Mischen aller Komponenten und die anschließende Behandlung, bevorzugt bei erhöhten Temperaturen, im Wirbelbett herzustellen.

Die Blondiermittel können in flüssiger, gelförmiger, pastöser oder in Pulverform vorliegen.

Bevorzugte kosmetische Zubereitungen sind Blondiermittel in Pulverform. Es hat sich gezeigt, dass für die erfindungsgemäßen Portionen insbesondere Blondierpulver geeignet sind, die eine mittlere Teilchengröße von unterhalb 250 µm, vorzugsweise zwischen 50 und 150 µm aufweisen.

Gemessen wurden die Teilchengrößen mittels eines Coulter Counters. Die erfindungsgemäßen Portionen, die Blondiermittel als kosmetische Zubereitung enthalten, werden üblicherweise unmittelbar vor dem Auftragen mit einer Wasserstoffperoxid-Lösung vermischt und in dieser aufgelöst.

Die Konzentration dieser Wasserstoffperoxid-Lösung wird einerseits von den gesetzlichen Vorgaben und andererseits von dem gewünschten Effekt bestimmt; in der Regel werden 6- bis 12-prozentige Lösungen in Wasser verwendet. Die Mengenverhältnisse von Blondiermittel und Wasserstoffperoxid-Lösung liegen dabei üblicherweise im Bereich 1:1 bis 1:2, wobei ein Überschuss an Wasserstoffperoxid-Lösung insbesondere dann gewählt wird, wenn keine zu ausgeprägte Blondierwirkung erwünscht ist.

### Haarfärbemittel:

In einer weiteren Ausführungsform enthalten die erfindungsgemäßen Portionen Haarfärbemittel als kosmetischen Zubereitung. Die Haarfärbemittel sind vorzugsweise ausgewählt aus der Gruppe der temporären Färbemittel, der semipermanenten Färbemittel und der permanenten Haarfärbemittel, insbesondere ausgewählt aus der Gruppe der Färbemittel mit reaktiven Carbonylverbindungen (Oxofärbemittel), Oxidationsfärbemittel, im Speziellen ausgewählt aus Entwicklerkomponenten oder Kupplerkomponenten oder den Komponenten A oder B eines Oxofärbemittels.

Temporäre Haarfärbemittel sind geeignet, um temporär Färbungen auf keratinen Fasern hervorzurufen.
Für temporäre Färbungen werden üblicherweise Färbe- oder Tönungsmittel verwendet, die als färbende Komponente sogenannte Direktzieher enthalten. Hierbei handelt es sich um Farbstoffmoleküle, die direkt auf das Haar aufziehen und keinen oxidativen Prozess zur Ausbildung der Farbe benötigen. Zu diesen Farbstoffen gehört beispielsweise das bereits aus dem Altertum zur Färbung von Körper und Haaren bekannte Henna. Diese Färbungen sind gegen Shampoonieren in der Regel deutlich empfindlicher als die oxidativen Färbungen, so dass dann sehr viel schneller eine vielfach unerwünschte Nuancenverschiebung oder gar eine sichtbare "Entfärbung" eintritt.

Semipermanente Haarfärbemittel zeichnen sich aus durch stärker ausgeprägte und dauerhaftere Farbnuancen aus.

Sie sind beständig gegen bis zu 5-6 Haarwäschen. Die verwendeten Farbstoffe müssen demgemäss eine hohe Affinität zum Keratin besitzen und relativ tief in die Oberfläche der Haarfaser eindringen. Wichtigste Vertreter dieser Farbstoff-Gruppe sind 2-Nitro-1,4-phenylendiamin- und Nitroanilin-Derivate. Bei den ebenfalls verwendeten sog. "Arianor-Farbstoffen" handelt es sich um Azo- oder Chinonimin-Farbstoffe mit quartären AmmoniumGruppen. Die Gegenwart von Glykolethern, Cyclohexanol oder Benzylalkohol im Lösungsmittelsystem fördert die Keratin-Affinität der Farbstoffe.

Permanente Haarfärbemittel haben weite Verbreitung gefunden. Die permanente Haarfärbung ist weitestgehend beständig gegen Licht- und Witterungseinflüsse sowie gegen alle üblichen Haarbehandlungsmethoden und braucht nur ca. monatlich erneuert zu werden, bedingt durch das Nachwachsen der Haare.

Die Definitionen für Haarfärbemittel finden sich im Römpp Lexikon Chemie, Version 2.0, Stuttgart/New York: Georg Thieme Verlag 1999.

In einer besonders bevorzugten Ausführungsform enthalten die erfindungsgemäßen Portionen Oxidationsfärbemittel. Oxidationsfärbemittel werden überlicherweise für dauerhafte, intensive Färbungen verwendet.

Solche Färbemittel enthalten üblicherweise Oxidationsfarbstoffvorprodukte, sogenannte Entwicklerkomponenten und Kupplerkomponenten. Die Entwicklerkomponenten bilden unter dem Einfluss von Oxidationsmitteln oder von Luftsauerstoff untereinander oder unter Kupplung mit einer oder mehreren Kupplerkomponenten die eigentlichen Farbstoffe aus. Für natürlich wirkende Färbungen wird üblicherweise eine Mischung aus einer größeren Zahl von Oxidationsfarbstoffvorprodukten eingesetzt werden; in vielen Fällen werden weiterhin direktziehende Farbstoffe zur Nuancierung verwendet.

Die die Entwickler- und Kupplerkomponenten enthaltende Färbezubereitung und das Oxidationsmittel - zumeist eine Wasserstoffperoxidzubereitungwerden üblicherweise kurz vor ihrer Applikation miteinander vermischt. In einer bevorzugten Ausführungsform der vorliegenden Erfindung befindet sich in einer erfindungsgemäßen Portion mindestens eine Färbezubereitung oder eine Oxidationsmittelzubereitung. Bevorzugt liegen die Portionen separat nebeneinander vor, also als Färbemittelzubereitung, vorzugsweise Entwickler- und/oder Kupplerkomponenten enthaltende Färbezubereitung, enthaltende Portion und als Oxidationsmittel enthaltende Portion.

Besonders bevorzugt liegt das Oxidationsfärbemittel in einem Mehrkammerbehälter vor, in dem sich in mindestens einer Kammer eine Färbezubereitung, vorzugsweise Entwickler- und/oder Kupplerkomponenten enthaltende Färbezubereitung, und in mindestens einer weiteren Kammer des Behälters mindestens eine Oxidationsmittelzubereitung befindet.

### Entwicklerkomponente:

Geeignete Entwicklerkomponenten sind beispielsweise p-Phenylendiaminderivate oder eines seiner physiologisch verträglichen Salze der Formel (E1) 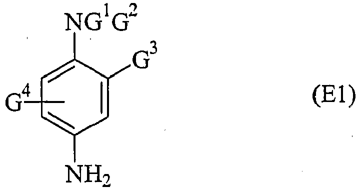 wobei
- G¹ steht für ein Wasserstoffatom, einen C₁-C₄-Alkylrest, einen C₁-C₃-Monohydroxyalkylrest, einen C₂-C₆-Polyhydroxyalkylrest, einen (C₁-C₄)-Alkoxy-(C₁-C₄)-alkylrest, einen 4-Aminophenylrest oder einen C₁-C₄-Alkylrest, der mit einer stickstoffhaltigen Gruppe, einem Phenyl- oder einem 4-Aminophenylrest substituiert ist;
- G² steht für ein Wasserstoffatom, einen C₁-C₄-Alkylrest, einen C₁-C₃-Monohydroxyalkylrest, einen C₂-C₆-Polyhydroxyalkylrest, einen (C₁-C₄)-Alkoxy-(C₁-C₄)-alkylrest oder einen C₁-C₄-Alkylrest, der mit einer stickstoffhaltigen Gruppe substituiert ist;
- G³ steht für ein Wasserstoffatom, ein Halogenatom, wie ein Chlor-, Brom-, Iod- oder Fluoratom, einen C₁-C₄-Alkylrest, einen C₁-C₄-Monohydroxyalkylrest, einen C₂-C₆-Polyhydroxyalkylrest, einen C₁-C₄-Hydroxyalkoxyrest, einen C₁-C₄-Acetylaminoalkoxyrest, einen C₁-C₄-Mesylaminoalkoxyrest oder einen C₁-C₄-Carbamoylaminoalkoxyrest;
- G⁴ steht für ein Wasserstoffatom, ein Halogenatom oder einen C₁-C₄-Alkylrest oder
- wenn G³ und G⁴ in ortho-Stellung zueinander stehen, können sie gemeinsam eine verbrückende α,ω-Alkylendioxogruppe, wie beispielsweise eine Ethylendioxygruppe bilden.

Beispiele für die als Substituenten in den erfindungsgemäßen Verbindungen genannten C₁-C₃-Alkylreste sind die Gruppen Methyl, Ethyl, Propyl und Isopropyl. Ethyl und Methyl sind allgemein bevorzugte Alkylreste. Erfindungsgemäß bevorzugte C₁-C₄-Alkoxyreste sind beispielsweise eine Methoxy- oder eine Ethoxygruppe. Weiterhin können als bevorzugte Beispiele für eine C₁-C₄-Monohydroxyalkylgruppe eine Hydroxymethyl-, eine 2-Hydroxyethyl-, eine 3-Hydroxypropyl- oder eine 4-Hydroxybutylgruppe genannt werden. Eine 2-Hydroxyethylgruppe ist besonders bevorzugt. Eine besonders bevorzugte C₂-C₄-Polyhydroxyalkylgruppe ist die 1,2-Dihydroxyethylgruppe. Beispiele für Halogenatome sind erfindungsgemäß F-, Cl- oder Br-Atome, Cl-Atome sind ganz besonders bevorzugt. Die weiteren verwendeten Begriffe leiten sich erfindungsgemäß von den hier gegebenen Definitionen ab. Beispiele für stickstoffhaltige Gruppen der Formel (E1) sind insbesondere die Aminogruppen, C₁-C₄-Monoalkylaminogruppen, C₁-C₄-Dialkylaminogruppen, C₁-C₄-Trialkylammoniumgruppen, C₁-C₄-Monohydroxyalkylaminogruppen, Imidazolinium und Ammonium.

Besonders bevorzugte p-Phenylendiamine der Formel (E1) sind ausgewählt aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)-anilin, N,N-Bis-(β-hydroxyethyl)-p-phenylen-diamin, 4-N,N-Bis-(β-hydroxyethyl)-amino-2-methylanilin, 4-N,N-Bis-(β-hydroxyethyl)-amino-2-chloranilin, 2-(β-Hydroxyethyl)-p-phenylendiamin, 2-(α,β-Dihydroxyethyl)-p-phenylendiamin, 2-Fluor-p-phenylendiamin, 2-Iso-propyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl,β-hydroxyethyl)-p-phenylendiamin, N-(β,γ-Dihydroxypropyl)-p-phenylendiamin, N-(4-Aminophenyl)-p-phenylendiamin, N-Phenyl-p-phenylendiamin, 2-(β-Hydroxyethyloxy)-p-phenylendiamin, 2-(β-Acetylaminoethyloxy)-p-phenylendiamin, N-(β-Methoxyethyl)-p-phenylendiamin und 5,8-Diaminobenzo-1,4-dioxan sowie ihren physiologisch verträglichen Salzen.

Erfindungsgemäß ganz besonders bevorzugte p-Phenylendiaminderivate der Formel (E1) sind p-Phenylendiamin, p-Toluylendiamin, 2-(β-Hydroxyethyl)-p-phenylendiamin, 2-(α,β-Dihydroxyethyl)-p-phenylendiamin und N,N-Bis-(β-hydroxyethyl)-p-phenylendiamin.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind.

Unter den zweikernigen Entwicklerkomponenten, die in den Färbezusammensetzungen gemäß der Erfindung verwendet werden können, kann man insbesondere die Verbindungen nennen, die der folgenden Formel (E2) entsprechen, sowie ihre physiologisch verträglichen Salze: 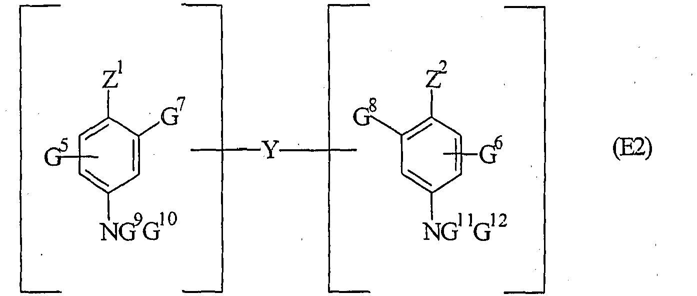 wobei:
- Z¹ und Z² stehen unabhängig voneinander für einen Hydroxyl- oder NH₂-Rest, der gegebenenfalls durch einen C₁-C₄-Alkylrest, durch einen C₁-C₄-Hydroxyalkylrest und/oder durch eine Verbrückung Y substituiert ist oder der gegebenenfalls Teil eines verbrückenden Ringsystems ist,
- die Verbrückung Y steht für eine Alkylengruppe mit 1 bis 14 Kohlenstoffatomen, wie beispielsweise eine lineare oder verzweigte Alkylenkette oder einen Alkylenring, die von einer oder mehreren stickstoffhaltigen Gruppen und/oder einem oder mehreren Heteroatomen wie Sauerstoff-, Schwefel- oder Stickstoffatomen unterbrochen oder beendet sein kann und eventuell durch einen oder mehrere Hydroxyl- oder C₁-C₈-Alkoxyreste substituiert sein kann, oder eine direkte Bindung,
- G⁵ und G⁶ stehen unabhängig voneinander für ein Wasserstoff- oder Halogenatom, einen C₁-C₄-Alkylrest, einen C₁-C₄-Monohydroxyalkylrest, einen C₂-C₆-Polyhydroxyalkylrest, einen C₁-C₄-Aminoalkylrest oder eine direkte Verbindung zur Verbrückung Y,
- G⁷, G⁸, G⁹, G¹⁰, G¹¹ und G¹² stehen unabhängig voneinander für ein Wasserstoffatom, eine direkte Bindung zur Verbrückung Y oder einen C₁-C₄-Alkylrest,
   mit den Maßgaben, dass
- die Verbindungen der Formel (E2) nur eine Verbrückung Y pro Molekül enthalten und
- die Verbindungen der Formel (E2) mindestens eine Aminogruppe enthalten, die mindestens ein Wasserstoffatom trägt.

Die in Formel (E2) verwendeten Substituenten sind erfindungsgemäß analog zu den obigen Ausführungen definiert.

Bevorzugte zweikernige Entwicklerkomponenten der Formel (E2) sind insbesondere: N,N'-Bis-(β-hydroxyethyl)-N,N'-bis-(4-aminophenyl)-1,3-diamino-propan-2-ol, N,N'-Bis-(β-hydroxyethyl)-N,N'-bis-(4-aminophenyl)-ethylendiamin, N,N'-Bis-(4-aminophenyl)-tetramethylendiamin, N,N'-Bis-(β-hydroxyethyl)-N,N'-bis-(4-aminophenyl)-tetramethylendiamin, N,N'-Bis-(4-methyl-aminophenyl)-tetramethylendiamin, N,N'-Diethyl-N,N'-bis-(4-amino-3-methylphenyl)-ethylendiamin, Bis-(2-hydroxy-5-aminophenyl)-methan, N,N'-Bis-(4-aminophenyl)-1,4-diazacycloheptan, N,N'-Bis-(2-hydroxy-5-aminobenzyl)-piperazin, N-(4-Aminophenyl)-p-phenylendiamin und 1,10-Bis-(2,5-diaminophenyl)-1,4,7,10-tetraoxadecan und ihre physiologisch verträglichen Salze.

Ganz besonders bevorzugte zweikernige Entwicklerkomponenten der Formel (E2) sind N,N'-Bis-(β-hydroxyethyl)-N,N'-bis-(4-aminophenyl)-1,3-diamino-propan-2-ol, Bis-(2-hydroxy-5-aminophenyl)-methan, N,N'-Bis-(4-amino-phenyl)-1,4-diazacycloheptan und 1,10-Bis-(2,5-diaminophenyl)-1,4,7,10-tetraoxadecan oder eines ihrer physiologisch verträglichen Salze.

Weiterhin kann es erfindungsgemäß bevorzugt sein, als Entwicklerkomponente ein p-Aminophenolderivat oder eines seiner physiologisch verträglichen Salze einzusetzen. Besonders bevorzugt sind p-Aminophenolderivate der Formel (E3) 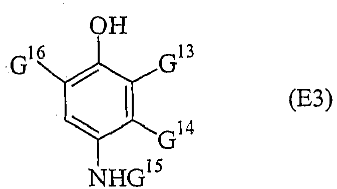 wobei:
- G¹³ steht für ein Wasserstoffatom, ein Halogenatom, einen C₁-C₄-Alkylrest, einen C₁-C₄-Monohydroxyalkylrest, einen C₂-C₆-Polyhydroxyalkylrest, einen (C₁-C₄)-Alkoxy-(C₁-C₄)-alkylrest, einen C₁-C₄-Aminoalkylrest, einen Hydroxy-(C₁-C₄)-alkylaminorest, einen C₁-C₄-Hydroxyalkoxyrest, einen C₁-C₄-Hydroxyalkyl-(C₁-bis C₄)-aminoalkylrest oder einen (Di-C₁-C₄-Alkylamino)-(C₁-C₄)-alkylrest, und
- G¹⁴ steht für ein Wasserstoff- oder Halogenatom, einen C₁-C₄-Alkylrest, einen C₁-C₄-Monohydroxyalkylrest, einen C₂-C₆-Polyhydroxyalkylrest, einen (C₁-C₄)-Alkoxy-(C₁-C₄)-alkylrest, einen C₁-C₄-Aminoalkylrest oder einen C₁-C₄-Cyanoalkylrest,
- G¹⁵ steht für Wasserstoff, einen C₁-C₄-Alkylrest, einen C₁-C₄-Monohydroxyalkylrest, einen C₂-C₆-Polyhydroxyalkylrest, einen Phenylrest oder einen Benzylrest, und
- G¹⁶ steht für Wasserstoff oder ein Halogenatom.

Die in Formel (E3) verwendeten Substituenten sind erfindungsgemäß analog zu den obigen Ausführungen definiert.

Bevorzugte p-Aminophenole der Formel (E3) sind insbesondere p-Aminophenol, N-Methyl-p-aminophenol, 4-Amino-3-methyl-phenol, 4-Amino-3-fluorphenol, 2-Hydroxymethylamino-4-aminophenol, 4-Amino-3-hydroxymethylphenol, 4-Amino-2-(β-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(β-hydroxyethyl-aminomethyl)-phenol, 4-Amino-2-(α,β-dihydroxyethyl)-phenol, 4-Amino-2-fluorphenol, 4-Amino-2-chlorphenol, 4-Amino-2,6-dichlorphenol, 4-Amino-2-(diethyl-aminomethyl)-phenol sowie ihre physiologisch verträglichen Salze.

Ganz besonders bevorzugte Verbindungen der Formel (E3) sind p-Aminophenol, 4-Amino-3-methylphenol, 4-Amino-2-aminomethylphenol, 4-Amino-2-(α,β-dihydroxyethyl)-phenol und 4-Amino-2-(diethyl-aminomethyl)-phenol.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol.

Weiterhin kann die Entwicklerkomponente ausgewählt sein aus heterocyclischen Entwicklerkomponenten, wie beispielsweise den Pyridin-, Pyrimidin-, Pyrazol-, Pyrazolopyrimidin-Derivaten und ihren physiologisch verträglichen Salzen.

Bevorzugte Pyridin-Derivate sind insbesondere die Verbindungen, die in den Patenten GB 1 026 978 und GB 1 153 196 beschrieben werden, wie 2,5-Diamino-pyridin, 2-(4-Methoxyphenyl)-amino-3-amino-pyridin, 2,3-Diamino-6-methoxy-pyridin, 2-(β-Methoxyethyl)-amino-3-amino-6-methoxy-pyridin und 3,4-Diamino-pyridin.

Bevorzugte Pyrimidin-Derivate sind insbesondere die Verbindungen, die im deutschen Patent DE 2 359 399, der japanischen Offenlegungsschrift JP 02019576 A2 oder in der Offenlegungsschrift WO 96/15765 beschrieben werden, wie 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin.

Bevorzugte Pyrazol-Derivate sind insbesondere die Verbindungen, die in den Patenten DE 3 843 892, DE 4 133 957 und Patentanmeldungen WO 94/08969, WO 94/08970, EP-740 931 und DE 195 43 988 beschrieben werden, wie 4,5-Diamino-1-methylpyrazol, 4,5-Diamino-1-(β-hydroxyethyl)-pyrazol, 3,4-Diaminopyrazol, 4,5-Diamino-1-(4-chlorbenzyl)-pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert.-butyl-l-methylpyrazol, 4,5-Diamino-1-tert.-butyl-3-methylpyrazol, 4,5-Diamino-1-(β-hydroxyethyl)-3-methylpyrazol, 4,5-Diamino-1-ethyl-3-methylpyrazol, 4,5-Diamino-1-ethyl-3-(4-methoxyphenyl)-pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(β-aminoethyl)-amino-1,3-dimethylpyrazol, 3,4,5-Triaminopyrazol, 1-Methyl-3,4,5-triaminopyrazol, 3,5-Diamino-1-methyl-4-methylaminopyrazol und 3,5-Diamino-4-(β-hydroxyethyl)-amino-l-methylpyrazol.

Bevorzugte Pyrazolopyrimidin-Derivate sind insbesondere die Derivate des Pyrazolo[1,5-a]pyrimidin der folgenden Formel (E4) und dessen tautomeren Formen, sofern ein tautomeres Gleichgewicht besteht: 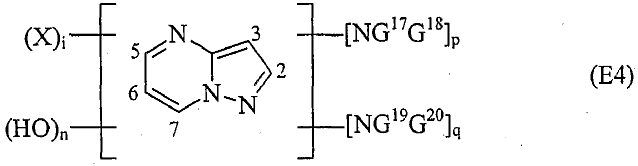 wobei:
- G¹⁷, G¹⁸, G¹⁹ und G²⁰ unabhängig voneinander stehen für ein Wasserstoffatom, einen C₁-C₄-Alkylrest, einen Aryl-Rest, einen C₁-C₄-Hydroxyalkylrest, einen C₂-C₄-Polyhydroxyalkylrest einen (C₁-C₄)-Alkoxy-(C₁-C₄)-alkylrest, einen C₁-C₄-Aminoalkylrest, der gegebenenfalls durch ein Acetyl-Ureid- oder einen Sulfonyl-Rest geschützt sein kann, einen (C₁-C₄)-Alkylamino-(C₁-C₄)-alkylrest, einen Di-[(C₁-C₄)-alkyl]-(C₁-C₄)-aminoalkylrest, wobei die Dialkyl-Reste gegebenenfalls einen Kohlenstoffzyklus oder einen Heterozyklus mit 5 oder 6 Kettengliedern bilden, einen C₁-C₄-Hydroxyalkyl- oder einen Di-(C₁-C₄)-[Hydroxyalkyl]-(C₁-C₄)-aminoalkylrest,
- die X-Reste stehen unabhängig voneinander für ein Wasserstoffatom, einen C₁-C₄-Alkylrest, einen Aryl-Rest, einen C₁-C₄-Hydroxyalkylrest, einen C₂-C₄-Polyhydroxyalkylrest, einen C₁-C₄-Aminoalkylrest, einen (C₁-C₄)-Alkylamino-(C₁-C₄)-alkylrest, einen Di-[(C₁-C₄)alkyl]- (C₁-C₄)-aminoalkylrest, wobei die Dialkyl-Reste gegebenenfalls einen Kohlenstoffzyklus oder einen Heterozyklus mit 5 oder 6 Kettengliedern bilden, einen C₁-C₄-Hydroxyalkyl- oder einen Di-(C₁-C₄-hydroxyalkyl)-aminoalkylrest, einen Aminorest, einen C₁-C₄-Alkyl- oder Di-(C₁-C₄-hydroxyalkyl)-aminorest, ein Halogenatom, eine Carboxylsäuregruppe oder eine Sulfonsäuregruppe,
- i hat den Wert 0, 1, 2 oder 3,
- p hat den Wert 0 oder 1,
- q hat den Wert 0 oder 1 und
- n hat den Wert 0 oder 1,
   mit der Maßgabe, dass
- die Summe aus p + q ungleich 0 ist,
- wenn p + q gleich 2 ist, n den Wert 0 hat, und die Gruppen NG¹⁷G¹⁸ und NG¹⁹G²⁰ belegen die Positionen (2,3); (5,6); (6,7); (3,5) oder (3,7);
- wenn p + q gleich 1 ist, n den Wert 1 hat, und die Gruppen NG¹⁷G¹⁸ (oder NG¹⁹G²⁰) und die Gruppe OH belegen die Positionen (2,3); (5,6); (6,7); (3,5) öder (3,7);

Die in Formel (E4) verwendeten Substituenten sind erfindungsgemäß analog zu den obigen Ausführungen definiert.

Wenn das Pyrazolo[1,5-a]pyrimidin der obenstehenden Formel (E4) eine Hydroxygruppe an einer der Positionen 2, 5 oder 7 des Ringsystems enthält, besteht ein tautomeres Gleichgewicht, das zum Beispiel im folgenden Schema dargestellt wird:

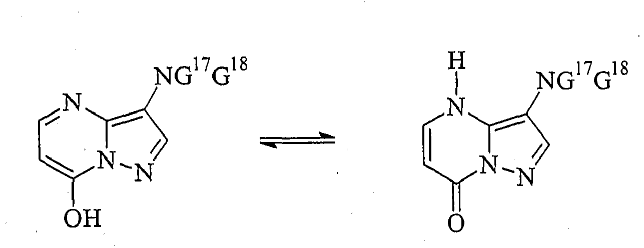

Unter den Pyrazolo[1,5-a]pyrimidinen der obenstehenden Formel (E4) kann man insbesondere nennen:
- Pyrazolo[1,5-a]pyrimidin-3,7-diamin;
- 2,5-Dimethyl-pyrazoto[1,5-a]pyrimidin-3,7-diamin;
- Pyrazolo[1,5-a]pyrimidin-3,5-diamin;
- 2,7-Dimethyl-pyrazolo[1,5-a]pyrimidin-3,5-diamin;
- 3-Aminopyrazolo[1,5-a]pyrimidin-7-ol;
- 3-Aminopyrazolo[1,5-a]pyrimidin-5-ol;
- 2-(3-Aminopyrazolo[1,5-a]pyrimidin-7-ylamino)-ethanol;
- 2-(7-Aminopyrazolo[1,5-a]pyrimidin-3-ylamino)-ethanol;
- 2-[(3-Aminopyrazolo[1,5-a]pyrimidin-7-yl)-(2-hydroxy-ethyl)-amino]-ethanol;
- 2-[(7-Aminopyrazolo[1,5-a]pyrimidin-3-yl)-(2-hydroxy-ethyl)-amino]-ethanol;
- 5,6-Dimethylpyrazolo[1,5-a]pyrimidin-3,7-diamin;
- 2,6-Dimethylpyrazolo[1,5-a]pyrimidin-3,7-diamin;
- 3-Amino-7-dimethylamino-2,5-dimethylpyrazolo[1,5-a]pyrimidin;
sowie ihre physiologisch verträglichen Salze und ihre tautomeren Formen, wenn ein tautomers Gleichgewicht vorhanden ist.

Die Pyrazolo[1,5-a]pyrimidine der obenstehenden Formel (E4) können wie in der Literatur beschrieben durch Zyklisierung ausgehend von einem Aminopyrazol oder von Hydrazin hergestellt werden.

### Kupplerkomponenten:

Vorzugsweise enthalten die erfindungsgemäßen Portionen mindestens eine Kupplerkomponente. Als Kupplerkomponenten werden in der Regel m-Phenylendiaminderivate, Naphthole, Resorcin und Resorcinderivate, Pyrazolone und m-Aminophenolderivate verwendet. Als Kupplersubstanzen eignen sich insbesondere 1-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 1-Phenyl-3-methyl-pyrazol-5-on, 2,4-Dichlor-3-aminophenol, 1,3-Bis-(2,4-diaminophenoxy)-propan, 2-Chlor-resorcin, 4-Chlor-resorcin, 2-Chlor-6-methyl-3-aminophenol, 2-Amino-3-hydroxypyridin, 2-Methylresorcin, 5-Methylresorcin und 2-Methyl-4-chlor-5-aminophenol.

Erfindungsgemäß bevorzugte Kupplerkomponenten sind
- m-Aminophenol und dessen Derivate wie beispielsweise 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2-Hydroxyethyl)-amino-2-methylphenol, 3-(Diethylamino)-phenol, N-Cyclopentyl-3-aminophenol, 1,3-Dihydroxy-5-(methylamino)-benzol, 3-Ethylamino-4-methylphenol und 2,4-Dichlor-3-aminophenol,
- o-Aminophenol und dessen Derivate,
- m-Diaminobenzol und dessen Derivate wie beispielsweise 2,4-Diaminophenoxyethanol, 1,3-Bis-(2,4-diaminophenoxy)-propan, 1-Methoxy-2-amino-4-(2-hydroxyethylamino)benzol, 1,3-Bis-(2,4-diaminophenyl)-propan, 2,6-Bis-(2-hydroxyethylamino)-1-methylbenzol und 1-Amino-3-bis-(2-hydroxyethyl)-aminobenzol,
- o-Diaminobenzol und dessen Derivate wie beispielsweise 3,4-Diaminobenzoesäure und 2,3-Diamino-1-methylbenzol,
- Di- beziehungsweise Trihydroxybenzolderivatewie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Amino-5-chlor-3-hydroxypyridin, 3-Amino-2-methyl-amino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxy-naphthalin, 1,6-Dihydroxynaphthalin, 1,7-Dihydroxynaphthalin, 1,8-Dihydroxynaphthalin, 2,7-Dihydroxynaphthalin und 2,3-Dihydroxy-naphthalin,
- Morpholinderivate wie beispielsweise 6-Hydroxybenzomorpholin und 6-Amino-benzomorpholin,
- Chinoxalinderivate wie beispielsweise 6-Methyl-1,2,3,4-tetra-hydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol,
- Pyrimidinderivate, wie beispielsweise 4,6-Diaminopyrimidin, 4-Amino-2,6-dihydroxypyrimidin, 2,4-Diamino-6-hydroxypyrimidin, 2,4,6-Trihydroxy-pyrimidin, 2-Amino-4-methylpyrimidin, 2-Amino-4-hydroxy-6-methylpyrimidin und 4,6-Dihydroxy-2-methylpyrimidin, oder
- Methylendioxybenzolderivate wie beispielsweise 1-Hydroxy-3,4-methylendioxybenzol, 1-Amino-3,4-methylendioxybenzol und 1-(2-Hydroxyethyl)-amino-3,4-methylendioxybenzol.

Erfindungsgemäß besonders bevorzugte Kupplerkömponenten sind 1-Naphthol, 1,5-, 2,7- und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin und 2,6-Dihydroxy-3,4-dimethylpyridin.

In einer weiteren bevorzugten Ausführungsform enthalten die erfindungsgemäßen Portionen naturanaloge Haarfarbstoffvorstufen. Das Färben mit naturanalogen Farbstoffen hat eine große Beachtung gefunden.

Bei diesem Verfahren werden Vorstufen des natürlichen Haarfarbstoffes Melanin auf das Haar aufgebracht; diese bilden dann im Rahmen oxidativer Prozesse im Haar naturanaloge Farbstoffe aus. Ein solches Verfahren mit 5,6-Dihydroxyindolin als Farbstoffvorprodukt wurde in der EP-B1-530 229 beschrieben. Bei, insbesondere mehrfacher, Anwendung von Mitteln mit 5,6-Dihydroxyindolin ist es möglich, Menschen mit ergrauten Haaren die natürliche Haarfarbe wiederzugeben. Die Ausfärbung kann dabei mit Luftsauerstoff als einzigem Oxidationsmittel erfolgen, so dass auf keine weiteren Oxidationsmittel zurückgegriffen werden muss. Bei Personen mit ursprünglich mittelblondem bis braunem Haar kann das Indolin als alleinige Farbstoffvorstufe eingesetzt werden. Für die Anwendung bei Personen mit ursprünglich roter und insbesondere dunkler bis schwarzer Haarfarbe können dagegen befriedigende Ergebnisse häufig nur durch Mitverwendung weiterer Farbstoffkomponenten, insbesondere spezieller Oxidationsfarbstoffvorprodukte, erzielt werden. Als Vorstufen naturanaloger Farbstoffe werden bevorzugt solche Indole und Indoline eingesetzt, die mindestens eine Hydroxy- oder Aminogruppe, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. In einer zweiten bevorzugten Ausführungsform enthalten die Färbemittel mindestens ein Indol- und/oder Indolinderivat.

Besonders gut als Vorstufen naturanaloger Haarfarbstoffe geeignet sind Derivate des 5,6-Dihydroxyindolins der Formel (VIIa), 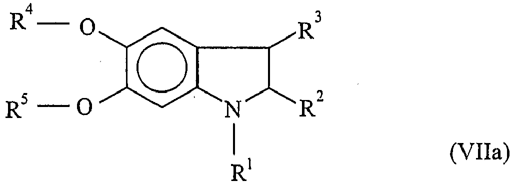 in der unabhängig voneinander
- R¹ steht für Wasserstoff, eine C₁-C₄-Alkylgruppe oder eine C₁-C₄-Hydroxy-alkylgruppe,
- R² steht für Wasserstoff oder eine -COOH-Gruppe, wobei die -COOH-Gruppe auch als Salz mit einem physiologisch verträglichen Kation vorliegen kann,
- R³ steht für Wasserstoff oder eine C₁-C₄-Alkylgruppe,
- R⁴ steht für Wasserstoff, eine C₁-C₄-Alkylgruppe oder eine Gruppe -CO-R⁶, in der R⁶ steht für eine C₁-C₄-Alkylgruppe, und
- R⁵ steht für eine der unter R⁴ genannten Gruppen,
sowie physiologisch verträgliche Salze dieser Verbindungen mit einer organischen oder anorganischen Säure.

Besonders bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure sowie das 6-Hydroxyindolin, das 6-Aminoindolin und das 4-Aminoindolin.

Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin.

Als Vorstufen naturanaloger Haarfarbstoffe hervorragend geeignet sind weiterhin Derivate des 5,6-Dihydroxyindols der Formel (VIIb), 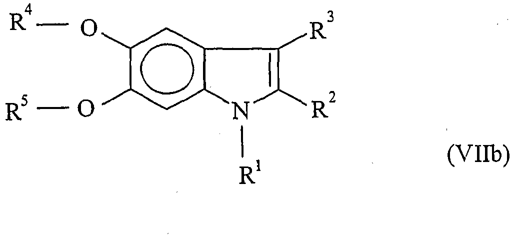 in der unabhängig voneinander
- R¹ steht für Wasserstoff, eine C₁-C₄-Alkylgruppe oder eine C₁-C₄-Hydroxy-alkylgruppe,
- R² steht für Wasserstoff oder eine -COOH-Gruppe, wobei die -COOH-Gruppe auch als Salz mit einem physiologisch verträglichen Kation vorliegen kann,
- R³ steht für Wasserstoff oder eine C₁-C₄-Alkylgruppe,
- R⁴ steht für Wasserstoff, eine C₁-C₄-Alkylgruppe oder eine Gruppe -CO-R⁶, in der R⁶ steht für eine C₁-C₄-Alkylgruppe, und
- R⁵ steht für eine der unter R⁴ genannten Gruppen,
sowie physiologisch verträgliche Salze dieser Verbindungen mit einer organischen oder anorganischen Säure.

Besonders bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxy-indol, 6-Aminoindol und 4-Aminoindol.

Innerhalb dieser Gruppe hervorzuheben sind N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.

Die Indolin- und Indol-Derivate können in den im Rahmen des erfindungsgemäßen Verfahrens eingesetzten Färbemitteln sowohl als freie Basen als auch in Form ihrer physiologisch verträglichen Salze mit anorganischen oder organischen Säuren, z. B. der Hydrochloride, der Sulfate und Hydrobromide, eingesetzt werden. Die Indol- oder Indolin-Derivate sind in diesen üblicherweise in Mengen von 0,05-10 Gew.-%, vorzugsweise 0,2-5 Gew.-% enthalten.

In einer weiteren Ausführungsform kann es erfindungsgemäß bevorzugt sein, das Indolin- oder Indolderivat in Haarfärbemitteln in Kombination mit mindestens einer Aminosäure oder einem Oligopeptid einzusetzen. Die Aminosäure ist vorteilhafterweise eine α-Aminosäure; ganz besonders bevorzugte α-Aminosäuren sind Arginin, Ornithin, Lysin, Serin und Histidin, insbesondere Arginin.

### Direktziehende Farbstoffe:

Die erfindungsgemäßen Portionen enthalten in einer weiteren Ausführungsform zusätzlich oder einzeln mindestens einen direktziehenden Farbstoff. Direktziehende Farbstoffe dienen häufig der Nuancierung der Haarfarben und werden daher vorteilhaft den permanenten Haarfarbstoffen, wie beispielsweise den vorgenannten Entwicklerkomponenten, Kupplerkomponenten, naturanalogen Haarfarbstoff-Vorstufen oder den Komponenten der Oxofärbemittel zugesetzt.

Direktziehende Farbstoffe sind üblicherweise Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 12, Disperse Blue 3, HC Violet 1, Disperse Violet 1, Disperse Violet 4, Acid Violet 43, Disperse Black 9 und Acid Black 52 bekannten Verbindungen sowie 1,4-Diamino-2-nitrobenzol, 2-Amino-4-nitrophenol, 1,4-Bis-(β-hydroxyethyl)-amino-2-nitrobenzol, 3-Nitro-4-(β-hydroxyethyl)-aminophenol, 2-(2'-Hydroxyethyl)amino-4,6-dinitrophenol, 1-(2'-Hydroxyethyl)amino-4-methyl-, 2-nitrobenzol, 1-Amino-4-(2'-hydroxyethyl)-amino-5-chlor-2-nitrobenzol, 4-Amino-3-nitrophenol, 1-(2'-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol.

Ferner können die erfindungsgemäßen Mittel einen kationischen direktziehenden Farbstoff enthalten. Besonders bevorzugt sind dabei
(a) kationische Triphenylmethanfarbstoffe, wie beispielsweise Basic Blue 7, Basic Blue 26, Basic Violet 2 und Basic Violet 14,
(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie
(c) direktziehende Farbstoffe, die einen Heterocyclus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden.

Bevorzugte kationische direktziehende Farbstoffe der Gruppe (c) sind insbesondere die folgenden Verbindungen:

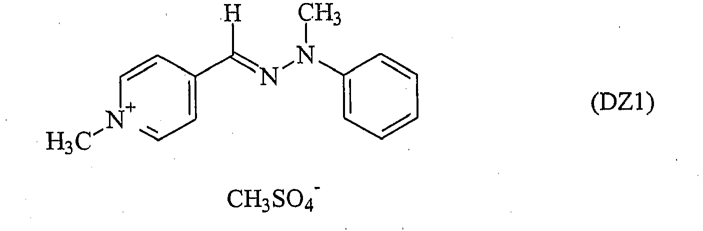

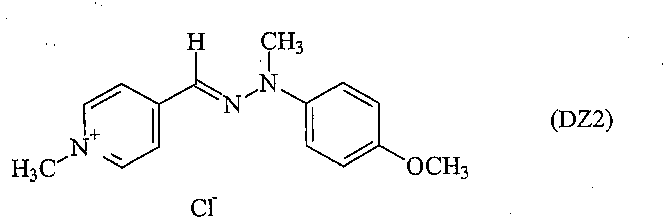

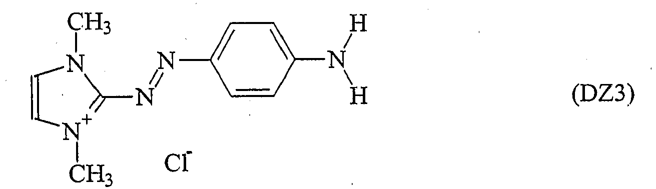

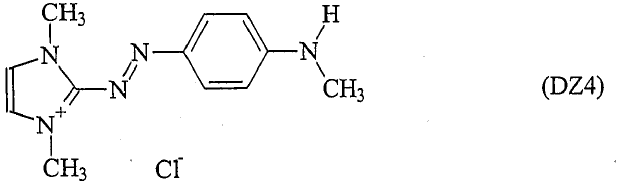

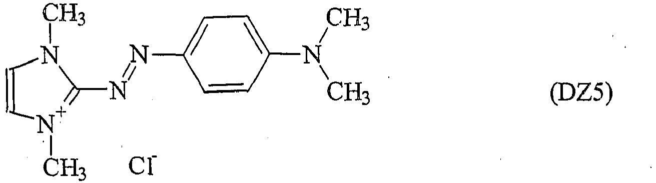

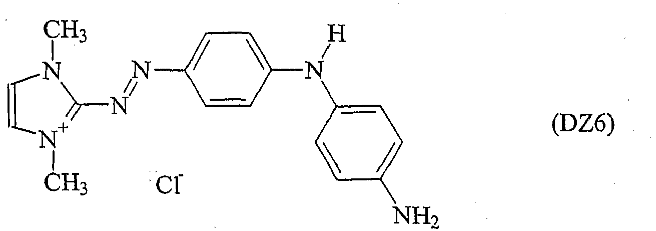

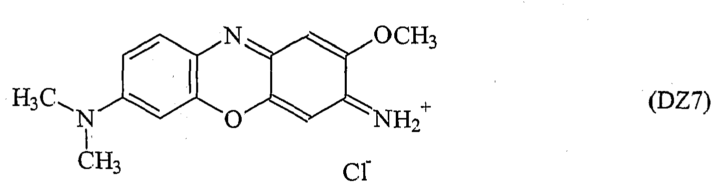

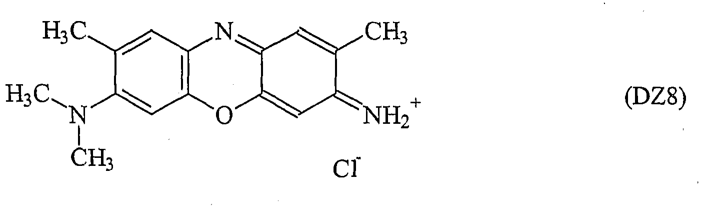

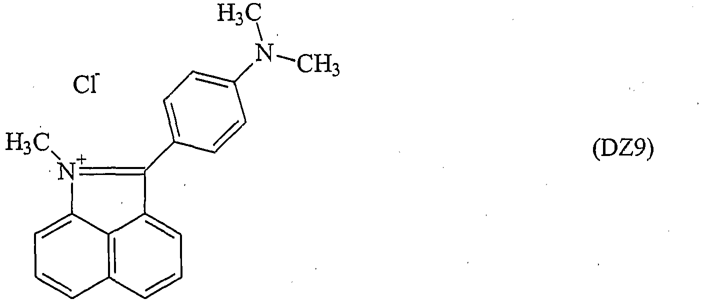

Die Verbindungen der Formeln (DZ1), (DZ3) und (DZ5) sind ganz besonders bevorzugte kationische direktziehende Farbstoffe der Gruppe (c). Die kationischen direktziehenden Farbstoffe, die unter dem Warenzeichen Arianor® vertrieben werden, sind erfindungsgemäß besonders bevorzugte direktziehende Farbstoffe.

In einer weiteren Ausführungsform enthalten die erfindungsgemäßen Potionen in der Natur vorkommende Farbstoffe, wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu, Sedre und Alkannawurzel enthalten sind.

### Oxofärbung:

In einer besonders bevorzugten Ausführungsform enthalten die erfindungsgemäßen Portionen Komponenten (Komponente A oder B), die für die Oxofärbung eingesetzt werden.

Oxofärbemittel bieten die Möglichkeit keratinhaltige Fasern zu färben, mittels Verwendung einer Kombination aus
Komponente A): Verbindungen, die eine reaktive Carbonylgruppe enthalten,
mit
Komponente B): Verbindungen, ausgewählt aus (a) CH-aciden Verbindungen, (b) Verbindungen mit primärer oder sekundärer Aminogruppe oder Hydroxygruppe, ausgewählt aus primären oder sekundären aromatischen Aminen, stickstoffhaltigen heterozyklischen Verbindungen und aromatischen Hydroxyverbindungen, (c) Aminosäuren, (d) aus 2 bis 9 Aminosäuren aufgebauten Oligopeptiden enthalten.

Die entsprechende Färbemethode (im folgenden Oxofärbung genannt) wird beispielweise in den Druckschriften WO-A1-99/18916, WO-A1-00/38638, WO-A1-01/34106 und WO-A1-01/47483 beschrieben. Die resultierenden Färbungen besitzen teilweise Farbechtheiten auf der keratinhaltigen Faser, die mit denen der Oxidationsfärbung vergleichbar sind. Das mit der schonenden Oxofärbung erzielbare Nuancenspektrum ist sehr breit und die erhaltene Färbung weist oftmals eine akzeptable Brillanz und Farbtiefe auf. Die vorgenannten Komponenten A und B, im weiteren als Oxofarbstoffvorprodukte bezeichnet, sind im allgemeinen selbst keine Farbstoffe, und eignen sich daher jede für sich genommen allein nicht zur Färbung keratinhaltiger Fasern. In Kombination bilden sie in einem nichtoxidativen Prozess Farbstoffe aus. Unter Verbindungen der Komponente B können allerdings auch entsprechende Oxidationsfarbstoffvorprodukte vom Entwickler- und/oder Kupplertyp mit oder ohne Einsatz eines Oxidationsmittels Verwendung finden. Somit läßt sich die Methode der Oxofärbung ohne weiteres mit dem oxidativen Färbesystem kombinieren.

Im Rahmen der Oxofärbung werden als Komponente A reaktive Carbonylverbindungen eingesetzt, die insbesondere nach Reaktion mit einer Komponente B, den eigentlichen Farbstoff im Haar ausbildet. Bevorzugte reaktive Carbonylverbindungen sind Aldehyde und Ketone, in denen die reaktive Carbonylgruppe entweder als Carbonylgruppe vorliegt oder derart derivatisiert bzw. maskiert ist, daß die Reaktivität des Kohlenstoffatoms der derivatisierten Carbonylgruppe gegenüber den Verbindungen der Komponente B stets vorhanden ist. Diese Derivate sind bevorzugt Additionsverbindungen
a) von Aminen und deren Derivate unter Bildung von Iminen oder Oximen als Additionsverbindung
b) von Alkoholen unter Bildung von Acetalen oder Ketalen als Additionsverbindung
c) von Wasser unter Bildung von Hydraten als Additionsverbindung (Komponente A leitet sich in diesem Fall c) von einem Aldehyd ab)
an das Kohlenstoffatom der Carbonylgruppe der reaktiven Carbonylverbindung.

Die Komponente A ist bevorzugt ausgewählt aus Verbindungen gemäß Formel (Ox1), 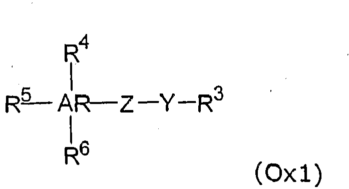 wobei
- AR steht für Benzol, Naphthalin, Pyridin, Pyrimidin, Pyrazin, Pyridazin, Carbazol, Pyrrol, Pyrazol, Furan, Thiophen, 1,2,3-Triazin, 1,3,5-Triazin, Chinolin, Isochinolin, Indol, Indolin, Indolizin, Indan, Imidazol, 1,2,4-Triazol, 1,2,3-Triazol, Tetrazol, Benzimidazol, 1,3-Thiazol, Benzothiazol, Indazol, Benzoxazol, Chinoxalin, Chinazolin, Chinolizin, Cinnolin, Acridin, Julolidin, Acenaphthen, Fluoren, Biphenyl, Diphenylmethan, Benzophenon, Diphenylether, Azobenzol, Chromon, Cumarin, Diphenylamin, Stilben, wobei die N-Heteroaromaten auch quaterniert sein können,
- R³ steht für ein Wasserstoffatom, eine C₁-C₆-Alkyl-, C₂-C₆-Acyl-, C₂-C₆-Alkenyl-, C₁-C₄-Perfluoralkyl-, eine ggf. substituierte Aryl- oder Heteroarylgruppe,
- R⁴, R⁵ und R⁶ stehen unabhängig voneinander für ein Wasserstoffatom, ein Halogenatom, eine C₁-C₆-Alkyl-, C₁-C₆-Alkoxy-, C₁-C₆-Aminoalkyl-, C₁-C₆-Hydroxyalkylgruppe, eine C₁-C₆-Alkoxy-C₁-C₆-alkyloxygruppe, eine C₂-C₆-Acylgruppe, eine Acetyl-, Carboxyl-, Carboxylato-, Carbamoyl-, Sulfo-, Sulfato-, Sulfonamid-, Sulfonamido-, C₂-C₆-Alkenyl-, eine Aryl-, eine Aryl-C₁-C₆-alkylgruppe, eine Hydroxy-, eine Nitro-, eine Pyrrolidino-, eine Morpholino-, eine Piperidino-, eine Amino- bzw. Ammonio- oder eine 1-Imidazol(in)iogruppe, wobei die letzten drei Gruppen mit einer oder mehrerer C₁-C₆-Alkyl-, C₁-C₆-Carboxyalkyl-, C₁-C₆-Hydroxyalkyl-, C₂-C₆-Alkenyl-, C₁-C₆-Alkoxy-C₁-C₆-alkyl-, mit ggf. substituierten Benzylgruppen, mit Sulfo-(C₁-C₄)-alkyl- oder Heterozyklus-(C₁-C₄)-alkylgruppen substituiert sein können,
wobei auch zwei der Reste aus R⁴, R⁵, R⁶ und -Z-Y-R³ zusammen mit dem Restmolekül einen ankondensierten gegebenenfalls substituierten 5-, 6- oder 7-Ring, der ebenfalls einen ankondensierten aromatischen Ring tragen kann, bilden können, wobei das System AR in Abhängigkeit von der Größe des Ringes weitere Substituenten tragen kann, die unabhängig voneinander für die gleichen Gruppen stehen können wie R⁴, R⁵ und R⁶,
- Z steht für eine direkte Bindung, eine Carbonyl-, eine Carboxy-(C₁-C₄)-alkylen-, eine gegebenenfalls substituierte C₂-C₆-Alkenylen-, C₄-C₆-Alkadienylen-, Furylen-, Thienylen-, Arylen-, Vinylenarylen-, Vinylenfurylen-, Vinylenthienylengruppe, wobei Z zusammen mit der -Y-R³-Gruppe auch einen gegebenenfalls substituierten 5-, 6- oder 7-Ring bilden kann,
- Y steht für eine Gruppe, die ausgewählt ist aus Carbonyl, einer Gruppe gemäß Formel (Ox2) und einer Gruppe gemäß Formel (Ox3), 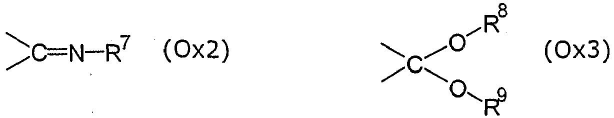
wobei
- R⁷ steht für ein Wasserstoffatom, eine Hydroxygruppe, eine C₁-C₄-Alkoxygruppe, eine C₁-C₆-Alkylgruppe, eine C₁-C₆-Hydroxyalkylgruppe, eine C₂-C₆-Polyhydroxyalkylgruppe, eine C₁-C₆-Alkoxy-C₁-C₆-alkylgruppe,
- R⁸ und R⁹ stehen unabhängig voneinander für ein Wasserstoffatom, eine C₁-C₆-Alkylgruppe, eine Arylgruppe oder bilden gemeinsam zusammen mit dem Strukturelement O-C-O der Formel (Ox3) einen 5- oder 6-gliederigen Ring.

Die Komponente A wird besonders bevorzugt ausgewählt aus der Gruppe bestehend aus Acetophenon, Propiophenon, 2-Hydroxyacetophenon, 3-Hydroxyacetophenon, 4-Hydroxyacetophenon, 2-Hydroxypropiophenon, 3-Hydroxypropiophenon, 4-Hydroxypropiophenon, 2-Hydroxybutyrophenon, 3-Hydroxybutyrophenon, 4-Hydroxybutyrophenon, 2,4-Dihydroxyacetophenon, 2,5-Dihydroxyacetophenon, 2,6-Dihydroxyacetophenon, 2,3,4-Trihydroxyacetophenon, 3,4,5-Trihydroxyacetophenon, 2,4,6-Trihydroxyacetophenon, 2,4,6-Trimethoxyacetophenon, 3,4,5-Trimethoxyacetophenon, 3,4,5-Tri-methoxy-acetophenon-diethylketal, 4-Hydroxy-3-methoxy-acetophenon, 3,5-Dimethoxy-4-hydroxyacetophenon, 4-Aminoacetophenon, 4-Dimethylaminoacetophenon, 4-Morpholinoacetophenon, 4-Piperidino-acetophenon, 4-Imidazolinoacetophenon, 2-Hydroxy-5-brom-acetophenon, 4-Hydroxy-3-nitroacetophenon, Acetophenon-2-carbonsäure, Acetophenon-4-carbonsäure, Benzophenon, 4-Hydroxybenzophenon, 2-Aminobenzophenon, 4,4'-Dihydroxybenzophenon, 2,4-Dihydroxy-benzophenon, 2,4,4'-Trihydroxybenzophenon, 2,3,4-Trihydroxybenzophenon, 2-Hydroxy-1-acetonaphthon, 1-Hydroxy-2-acetonaphthon, Chromon, Chromon-2-carbonsäure, Flavon, 3-Hydroxyflavon, 3,5,7-Trihydroxyflavon, 4,5,7-Trihydroxyflavon,5,6,7-Trihydroxyflavon, Quercetin, 1-Indanon, 9-Fluorenon, 3-Hydroxyfluorenon, Anthron, 1,8-Dihydroxyanthron,
Vanillin, Coniferylaldehyd, 2-Methoxybenzaldehyd, 3-Methoxybenzaldehyd, 4-Methoxybenzaldehyd, 2-Ethoxybenzaldehyd, 3-Ethoxybenzaldehyd, 4-Ethoxybenzaldehyd, 4-Hydroxy-2,3-dimethoxy-benzaldehyd, 4-Hydroxy-2,5-dimethoxy-benzaldehyd, 4-Hydroxy-2,6-dimethoxy-benzaldehyd, 4-Hydroxy-2-methyl-benzaldehyd, 4-Hydroxy-3-methyl-benzaldehyd, 4-Hydroxy-2,3-dimethyl-benzaldehyd, 4-Hydroxy-2,5-dimethyl-benzaldehyd, 4-Hydroxy-2,6-dimethyl-benzaldehyd, 4-Hydroxy-3,5-dimethoxy-benzaldehyd, 4-Hydroxy-3,5-dimethyl-benzaldehyd, 3,5-Diethoxy-4-hydröxy-benzaldehyd, 2,6-Diethoxy-4-hydroxy-benzaldehyd, 3-Hydroxy-4-methoxy-benzaldehyd, 2-Hydroxy-4-methoxy-benzaldehyd, 2-Ethoxy-4-hydroxy-benzaldehyd, 3-Ethoxy-4-hydroxy-benzaldehyd, 4-Ethoxy-2-hydroxy-benzaldehyd, 4-Ethoxy-3-hydroxy-benzaldehyd, 2,3-Dimethoxybenzaldehyd, 2,4-Dimethoxybenzaldehyd, 2,5-Dimethoxybenzaldehyd, 2,6-Dimethoxybenzaldehyd, 3,4-Dimethoxybenzaldehyd, 3,5-Dimethoxybenzaldehyd, 2,3,4-Trimethoxybenzaldehyd, 2,3,5-Trimethoxybenzaldehyd, 2,3,6-Trimethoxybenzaldehyd, 2,4,6-Trimethoxybenzaldehyd, 2,4,5-Trimethoxybenzaldehyd, 2,5,6-Trimethoxybenzaldehyd, 2-Hydroxybenzaldehyd, 3-Hydroxybenzaldehyd, 4-Hydroxybenzaldehyd, 2,3-Dihydroxybenzaldehyd, 2,4-Dihydroxybenzaldehyd, 2,4-Dihydroxy-3-methyl-benzaldehyd, 2,4-Dihydroxy-5-methyl-benzaldehyd, 2,4-Dihydroxy-6-methyl-benzaldehyd, 2,4-Dihydroxy-3-methoxy-benzaldehyd, 2,4-Dihydroxy-5-methoxy-benzaldehyd, 2,4-Dihydroxy-6-methoxy-benzaldehyd, 2,5-Dihydroxybenzaldehyd, 2,6-Dihydroxybenzaldehyd, 3,4-Dihydroxybenzaldehyd, 3,4-Dihydroxy-2-methyl-benzaldehyd, 3,4-Dihydroxy-5-methyl-benzaldehyd, 3,4-Dihydroxy-6-methyl-benzaldehyd, 3,4-Dihydroxy-2-methoxy-benzaldehyd, 3,4-Dihydroxy-5-methoxy-benzaldehyd, 3,5-Dihydroxybenzaldehyd, 2,3,4-Trihydroxybenzaldehyd, 2,3,5-Trihydroxybenzaldehyd, 2,3,6-Trihydroxybenzaldehyd, 2,4,6-Trihydroxybenzaldehyd, 2,4,5-Trihydroxybenzaldehyd, 3,4,5-Trihydroxybenzaldehyd, 2,5,6-Trihydroxybenzaldehyd, 4-Hydroxy-2-methoxybenzaldehyd, 4-Dimethylaminobenzaldehyd, 4-Diethylaminobenzaldehyd, 4-Dimethylamino-2-hydroxybenzaldehyd, 4-Diethylamino-2-hydroxybenzaldehyd, 4-Pyrrolidinobenzaldehyd, 4-Morpholinobenzaldehyd, 2-Morpholinobenzaldehyd, 4-Piperidinobenzaldehyd, 2-Methoxy-1-naphthaldehyd, 4-Methoxy-1-naphthaldehyd, 2-Hydroxy-1-naphthaldehyd, 2,4-Dihydroxy-1-napthaldehyd, 4-Hydroxy-3-methoxy-1-naphthaldehyd, 2-Hydroxy-4-methoxy-1-naphthaldehyd, 3-Hydroxy-4-methoxy-1-naphthaldehyd, 2,4-Dimethoxy-1-naphthaldehyd, 3,4-Dimethoxy-1-naphthaldehyd, 4-Hydroxy-1-naphthaldehyd, 4-Dimethylamino-1-naphthaldehyd, 4-Dimethylaminozimtaldehyd, 2-Dimethylaminobenzaldehyd, 2-Chlor-4-dimethylaminobenzaldehyd, 4-Dimethylamino-2-methylbenzaldehyd, 4-Diethylamino-zimtaldehyd, 4-Dibutylamino-benzaldehyd, 4-Diphenylamino-benzaldehyd, 4-Dimethylamino-2-methoxybenzaldehyd, 4-(1-Imidazolyl)-benzaldehyd, Piperonal, 2,3,6,7-Tetrahydro-1H,5H-benzo[ij]chinolizin-9-carboxaldehyd, 2,3,6,7-Tetrahydro-8-hydroxy-1H,5H-benzo[ij]chinolizin-9-carboxaldehyd, N-Ethylcarbazol-3-aldehyd, 2-Formylmethylen-1,3,3-trimethylindolin (Fischers Aldehyd oder Tribasen Aldehyd),
2-Indolaldehyd, 3-Indolaldehyd, 1-Methylindol-3-aldehyd, 2-Methylindol-3-aldehyd, 1-Acetylindol-3-aldehyd, 3-Acetylindol, 1-Methyl-3-acetylindol, 2-(1',3',3'-Trimethyl-2-indolinyliden)-acetaldehyd, 1-Methylpyrrol-2-aldehyd, 1-Methyl-2-acetylpyrrol, 4-Pyridinaldehyd, 2-Pyridinaldehyd, 3-Pyridinaldehyd, 4-Acetylpyridin, 2-Acetylpyridin, 3-Acetylpyridin, Pyridoxal, Chinolin-3-aldehyd, Chinolin-4-aldehyd, Antipyrin-4-aldehyd, Furfural, 5-Nitrofurfural, 2-Thenoyl-trifluor-aceton, Chromon-3-aldehyd, 3-(5'-Nitro-2'-furyl)-acrolein, 3-(2'-Furyl)-acrolein und Imidazol-2-aldehyd,
1,3-Diacetylbenzol, 1,4-Diacetylbenzol, 1,3,5-Triacetylbenzol, 2-Benzoyl-acetophenon, 2-(4'-Methoxybenzoyl)-acetophenon, 2-(2'-Furoyl)-acetophenon, 2-(2'-Pyridoyl)-acetophenon und 2-(3'-Pyridoyl)-acetophenon, Benzylidenaceton, 4-Hydroxybenzylidenaceton, 2-Hydroxybenzylidenaceton, 4-Methoxybenzylidenaceton, 4-Hydroxy-3-methoxybenzylidenaceton, 4-Dimethylaminobenzylidenaceton, 3,4-Methylendioxybenzylidenaceton, 4-Pyrrolidinobenzylidenaceton, 4-Piperidinobenzylidenaceton, 4-Morpholinobenzylidenaceton, 4-Diethylaminobenzylidenaceton, 3-Benzyliden-2,4-pentandion, 3-(4'-Hydroxybenzyliden)-2,4-pentandion, 3-(4'-Dimethylaminobenzyliden)-2,4-pentandion, 2-Benzylidencyclohexanon, 2-(4'-Hydroxybenzyliden)-cyclohexanon, 2-(4'-Dimethylaminobenzyliden)-cyclohexanon, 2-Benzyliden-1,3-cyclohexandion, 2-(4'-Hydroxybenzyliden)-1,3-cyclohexandion, 3-(4'-Dimethylaminobenzyliden)-1,3'cyclohexandion, 2-Benzyliden-5,5-dimethyl-1,3-cyclohexandion, 2-(4'-Hydroxybenzyliden)-5,5-dimethyl-1,3-cyclohexandion, 2-(4'-Hydroxy-3-methoxybenzyliden)-5,5-dimethyl-1,3-cyclohexandion, 2-(4-Dimethylaminobenzyliden)-5,5-dimethyl-1,3-cyclohexandion, 2-Benzylidencyclopentanon, 2'-(4-Hydroxybenzyliden)-cyclopentanon, 2-(4'-Dimethylaminobenzyliden)-cyclopentanon, 5-(4-Dimethylaminophenyl)penta-2,4-dienal, 5-(4-Diethylaminophenyl)penta-2,4-dienal, 5-(4-Methoxyphenyl)penta-2,4-dienal, 5-(3,4-Dimethoxyphenyl)penta-2,4-dienal, 5-(2,4-Dimethoxyphenyl)penta-2,4-dienal, 5-(4-Piperidinophenyl)penta-2,4-dienal, 5-(4-Morpholinophenyl)penta-2,4-dienal, 5-(4-Pyrrolidinophenyl)penta-2,4-dienal, 6-(4-Dimethylaminophenyl)hexa-3,5-dien-2-on, 6-(4-Diethylaminophenyl)hexa-3,5-dien-2-on, 6-(4-Methoxyphenyl)hexa-3,5-dien-2-on, 6-(3,4-Dimethoxyphenyl)hexa-3,5-dien-2-on, 6-(2,4-Dimethoxyphenyl)hexa-3,5-dien-2-on, 6-(4-Piperidinophenyl)hexa-3,5-dien-2-on, 6-(4-Morpholinophenyl)hexa-3,5-dien-2-on, 6-(4-Pyrrolidinophenyl)hexa-3,5-dien-2-on,
5-(4-Dimethylamino-1-naphthyl)penta-3,5-dienal, 2-Nitrobenzaldehyd, 3-Nitrobenzaldehyd, 4-Nitrobenzaldehyd, 4-Methyl-3-nitrobenzaldehyd, 3-Hydroxy-4-nitrobenzaldehyd, 4-Hydroxy-3-nitrobenzaldehyd, 5-Hydroxy-2-nitrobenzaldehyd, 2-Hydroxy-5-nitrobenzaldehyd, 2-Hydroxy-3-nitrobenzaldehyd, 2-Fluor-3-nitrobenzaldehyd, 3-Methoxy-2-nitrobenzaldehyd, 4-Chlor-3-nitrobenzaldehyd, 2-Chlor-6-nitrobenzaldehyd, 5-Chlor-2-nitrobenzaldehyd, 4-Chlor-2-nitrobenzaldehyd, 2,4-Dinitrobenzaldehyd, 2,6-Dinitrobenzaldehyd, 2-Hydroxy-3-methoxy-5-nitrobenzaldehyd, 4,5-Dimethoxy-2-nitrobenzaldehyd, 6-Nitropiperonal, 2-Nitropiperonal, 5-Nitrovanillin, 2,5-Dinitrosalicylaldehyd, 5-Brom-3-nitrosalicylaldehyd, 3-Nitro-4-formylbenzolsulfonsäure, 4-Nitro-1-naphthaldehyd, 2-Nitrozimtaldehyd, 3-Nitrozimtaldehyd, 4-Nitrozimtaldehyd, 9-Methyl-3-carbazolaldehyd, 9-Ethyl-3-carbazolaldehyd, 3-Acetylcarbazol, 3,6-Diacetyl-9-ethylcarbazol, 3-Acetyl-9-methylcarbazol, 1,4-Dimethyl-3-carbazolaldehyd, 1,4,9-Trimethyl-3-carbazolaldehyd,
4-Formyl-1-methylpyridinium-, 2-Formyl-1-methylpyridinium-, 4-Formyl-1-ethylpyridinium-, 2-Formyl-1-ethylpyridinium-, 4-Formyl-1-benzylpyridinium-, 2-Formyl-1-benzylpyridinium-, 4-Formyl-1,2-dimethylpyridinium-, 4-Formyl-1,3-dimethylpyridinium-, 4-Formyl-1-methylchinolinium-, 2-Formyl-1-methylchinolinium-, 4-Acetyl-1-methylpyridinium-, 2-Acetyl-1-methylpyridinium-, 4-Acetyl-1-methylchinolinium-, 5-Formyl-1-methylchinolinium-, 6-Formyl-1-methylchinolinium-, 7-Formyl-1-methylchinolinium-, 8-Formyl-1-methylchinolinium, 5-Formyl-1-ethylchinolinium-, 6-Formyl-1-ethylchinolinium-, 7-Formyl-1-ethylchinolinium-, 8-Formyl-1-ethylchinolinium, 5-Formyl-1-benzylchinolinium-, 6-Formyl-1-benzylchinolinium-, 7-Formyl-1-benzylchinolinium-, 8-Formyl-1-benzylchinolinium, 5-Formyl-l-allylchinolinium-, 6-Formyl-1-allylchinolinium-, 7-Formyl-1-allylchinolinium- und 8-Formyl-1-allylchinolinium-, 5-Acetyl-1-methylchinolinium-, 6-Acetyl-1-methylchinolinium-, 7-Acetyl-1-methylchinolinium-, 8-Acetyl-1-methylchinolinium, 5-Acetyl-1-ethylchinolinium-, 6-Acetyl-1-ethylchinolinium-, 7-Acetyl-1-ethylchinolinium-, 8-Acetyl-1-ethylchinolinium, 5-Acetyl-1-benzylchinolinium-, 6-Acetyl-1-benzylchinolinium-, 7-Acetyl-1-benzylchinolinium-, 8-Acetyl-1-benzylchinolinium, 5-Acetyl-1-allylchinolinium-, 6-Acetyl-1-allylchinolinium-, 7-Acetyl-1-allylchinolinium- und 8-Acetyl-1-allylchinolinium, 9-Formyl-10-methylacridinium-, 4-(2'-Formylvinyl)-1-methylpyridinium-, 1,3-Dimethyl-2-(4'-formylphenyl)-benzimidazolium-, 1,3-Dimethyl-2-(4'-formylphenyl)-imidazolium-, 2-(4'-Formylphenyl)-3-methylbenzothiazolium-, 2-(4'-Acetylphenyl)-3-methylbenzothiazolium-, 2-(4'-Formylphenyl)-3-methylbenzoxazolium-, 2-(5'-Formyl-2'-furyl)-3-methylbenzothiazolium-, 2-(5'-Formyl-2'-furyl)-3-methylbenzothiazolium-, 2-(5'-Formyl-2'-thienyl)-3-methylbenzothiazolium-, 2-(3'-Formylphenyl)-3-methylbenzothiazolium-, 2-(4'-Formyl-1-naphthyl)-3-methylbenzothiazolium-, 5-Chlor-2-(4'-formylphenyl)-3-methylbenzothiazolium-, 2-(4'-Formylphenyl)-3,5-dimethylbenzothiazolium-benzolsulfonat, -p-toluolsulfonat, -methansulfonat, - perchlorat, -sulfat, -chlorid, -bromid, -iodid, -tetrachlorozinkat, -methylsulfat-, trifluormethansulfonat, -tetrafluoroborat,
Isatin, 1-Methyl-isatin, 1-Allyl-isatin, 1-Hydroxymethyl-isatin, 5-Chlor-isatin, 5-Methoxy-isatin, 5-Nitroisatin, 6-Nitro-isatin, 5-Sulfo-isatin, 5-Carboxyisatin, Chinisatin, 1-Methylchinisatin, sowie beliebigen Gemischen der voranstehenden Verbindungen.

Ganz besonders bevorzugt werden in den erfindungsgemäßen Mitteln Benzaldehyd, Zimtaldehyd und Naphthaldehyd sowie deren Derivate, insbesondere mit einem oder mehreren Hydroxy-, Alkoxy- oder Aminosubstituenten, als Komponente A verwendet. Dabei werden wiederum die Verbindungen gemäß Formel (Ox4) bevorzugt, 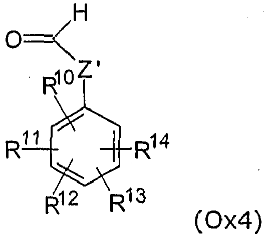 worin
- R¹⁰, R¹¹ und R¹² stehen unabhängig voneinander für ein Wasserstoffatom, ein Halogenatom, eine C₁-C₆-Alkylgruppe, eine Hydroxygruppe, eine C₁-C₆-Alkoxygruppe, eine Aminogruppe, eine C₁-C₆-Dialkylaminogruppe, eine Di(C₂-C₆-hydroxyalkyl)aminogruppe, eine Di(C₁-C₆-alkoxy-C₁-C₆-alkyl)aminoguppe, eine C₁-C₆-Hydroxyalkyloxygruppe, eine Sulfonylgruppe, eine Carboxylgruppe, eine Sulfonsäuregruppe, eine Sulfonamidogruppe, eine Sulfonamidgruppe, eine Carbamoylgruppe, eine C₂-C₆-Acylgruppe, eine Acetylgruppe oder eine Nitrogruppe,
- Z' steht für eine direkte Bindung oder eine Vinylengruppe,
- R¹³ und R¹⁴ stehen für ein Wasserstoffatom oder bilden gemeinsam, zusammen mit dem Restmolekül einen 5- oder 6-gliederigen aromatischen oder aliphatischen Ring.

Ganz besonders bevorzugte Verbindungen der Komponente A werden ausgewählt aus der Gruppe bestehend aus Vanillin, Coniferylaldehyd, 2-Methoxybenzaldehyd, 3-Methoxybenzaldehyd, 4-Methoxybenzaldehyd, 2-Ethoxybenzaldehyd, 3-Ethoxybenzaldehyd, 4-Ethoxybenzaldehyd, 4-Hydroxy-2,3-dimethoxy-benzaldehyd, 4-Hydroxy-2,5-dimethoxy-benzaldehyd, 4-Hydroxy-2,6-dimethoxy-benzaldehyd, 4-Hydroxy-2-methyl-benzaldehyd, 4-Hydroxy-3-methyl-benzaldehyd, 4-Hydroxy-2,3-dimethyl-benzaldehyd, 4-Hydroxy-2,5-dimethyl-benzaldehyd, 4-Hydroxy-2,6-dimethyl-benzaldehyd, 4-Hydroxy-3,5-dimethoxy-benzaldehyd, 4-Hydroxy-3,5-dimethyl-benzaldehyd, 3,5-Diethoxy-4-hydroxy-benzaldehyd, 2,6-Diethoxy-4-hydroxy-benzaldehyd, 3-Hydroxy-4-methoxy-benzaldehyd, 2-Hydroxy-4-methoxy-benzaldehyd, 2-Ethoxy-4-hydroxy-benzaldehyd, 3-Ethoxy-4-hydroxy-benzaldehyd, 4-Ethoxy-2-hydroxy-benzaldehyd, 4-Ethoxy-3-hydroxy-benzaldehyd, 2,3-Dimethoxybenzaldehyd, 2,4-Dimethoxybenzaldehyd, 2,5-Dimethoxybenzaldehyd, 2,6-Dimethoxybenzaldehyd, 3,4-Dimethoxybenzaldehyd, 3,5-Dimethoxybenzaldehyd, 2,3,4-Trimethoxybenzaldehyd, 2,3,5-Trimethoxybenzaldehyd, 2,3,6-Trimethoxybenzaldehyd, 2,4,6-Trimethoxybenzaldehyd, 2,4,5-Trimethoxybenzaldehyd, 2,5,6-Trimethoxybenzaldehyd, 2-Hydroxybenzaldehyd, 3-Hydroxybenzaldehyd, 4-Hydroxybenzaldehyd, 2,3-Dihydroxybenzaldehyd, 2,4-Dihydroxybenzaldehyd, 2,4-Dihydroxy-3-methyl-benzaldehyd, 2,4-Dihydroxy-5-methyl-benzaldehyd, 2,4-Dihydroxy-6-methyl-benzaldehyd, 2,4-Dihydroxy-3-methoxy-benzaldehyd, 2,4-Dihydroxy-5-methoxy-benzaldehyd, 2,4-Dihydroxy-6-methoxy-benzaldehyd, 2,5-Dihydroxybenzaldehyd, 2,6-Dihydroxybenzaldehyd, 3,4-Dihydroxybenzaldehyd, 3,4-Dihydroxy-2-methylbenzaldehyd, 3,4-Dihydroxy-5-methyl-benzaldehyd, 3,4-Dihydroxy-6-methylbenzaldehyd, 3,4-Dihydroxy-2-methoxy-benzaldehyd, 3,4-Dihydroxy-5-methoxy-benzaldehyd, 3,5-Dihydroxybenzaldehyd, 2,3,4-Trihydroxybenzaldehyd, 2,3,5-Trihydroxybenzaldehyd, 2,3,6-Trihydroxybenzaldehyd, 2,4,6-Trihydroxybenzaldehyd, 2,4,5-Trihydroxybenzaldehyd, 2,5,6-Trihydroxybenzaldehyd, 3,4,5-Trihydroxybenzaldehyd, 4-Hydroxy-2-methoxybenzaldehyd, 4-Dimethylaminobenzaldehyd, 4-Diethylaminobenzaldehyd, 4-Dimethylamino-2-hydroxybenzaldehyd, 4-Diethylamino-2-hydroxybenzaldehyd, 4-Pyrrolidinobenzaldehyd, 4-Morpholinobenzaldehyd, 2-Morpholinobenzaldehyd, 4-Piperidinobenzaldehyd, 2-Methoxy-1-naphthaldehyd, 4-Methoxy-1-naphthaldehyd, 2-Hydroxy-1-naphthaldehyd, 2,4-Dihydroxy-1-napthaldehyd, 4-Hydroxy-3-methoxy-1-naphthaldehyd, 2-Hydroxy-4-methoxy-1-naphthaldehyd, 3-Hydroxy-4-methoxy-l-naphthaldehyd, 2,4-Dimethoxy-1-naphthaldehyd, 3,4-Dimethoxy-1-naphthaldehyd, 4-Hydroxy-1-naphthaldehyd, 4-Dimethylamino-1-naphthaldehyd, 4-Dimethylaminozimtaldehyd, 2-Dimethylaminobenzaldehyd, 2-Chlor-4-dimethylaminobenzaldehyd, 4-Dimethylamino-2-methylbenzaldehyd, 4-Diethylamino-zimtaldehyd, 4-Dibutylamino-benzaldehyd, 4-Diphenylamino-benzaldehyd, 4-Dimethylamino-2-methoxybenzaldehyd, 4-(1-Imidazolyl)-benzaldehyd und Piperonal.

In einer zweiten Ausführungsform kann es zur Erweiterung des Farbspektrums sowie zur Verbesserung der Echtheitseigenschaften vorteilhaft sein, den Mitteln neben der reaktiven Carbonylverbindung (Komponente A) mindestens eine weitere Verbindung als Komponente B, ausgewählt aus (a) CH-aciden Verbindungen und (b) Verbindungen mit primärer oder sekundärer Amino- oder Hydroxygruppe, ausgewählt aus aromatischen Hydroxyverbindungen, primären oder sekundären aromatischen Aminen und stickstoffhaltigen heterozyklischen Verbindungen, zuzusetzen. CH-acide Verbindungen besitzen ein an ein Kohlenstoffatom gebundenes acides Wasserstoffatom, welches sich unter Verwendung einer Base vom Kohlenstoffatom abstrahieren lässt.

Die CH-aciden Verbindungen der Komponente B sind bevorzugt ausgewählt aus der Gruppe bestehend aus 1,2,3,3-Tetramethyl-3H-indoliumiodid, 1,2,3,3-Tetramethyl-3H-indolium-p-toluolsulfonat, 1,2,3,3-Tetramethyl-3H-indolium-methansulfonat, 1,3,3-Trimethyl-2-methylenindolin (Fischersche Base), 2,3-Dimethyl-benzothiazoliumiodid, 2,3-Dimethyl-benzothiazolium-p-toluolsulfonat, 2,3-Dimethyl-naphtho[1,2-d]thiazolium-p-toluolsulfonat, 3-Ethyl-2-methyl-naphtho[1,2-d]thiazolium-p-toluolsulfonat, Rhodanin, Rhodanin-3-essigsäure, 1,4-Dimethylchinolinium-iodid, 1,2-Dimethylchinolinium-iodid, Barbitursäure, Thiobarbitursäure, 1,3-Dimethylthiobarbitursäure, 1,3-Diethylthiobarbitursäure, 1,3-Diethylbarbitursäure, Oxindol, 3-Indoxylacetat, 2-Cumaranon, 5-Hydroxy-2-cumaranon, 6-Hydroxy-2-cumaranon, 3-Methyl-1-phenyl-pyrazolin-5-on, Indan-1,2-dion, Indan-1,3-dion, Indan-1-on, Benzoylacetonitril, 3-Dicyanmethylenindan-1-on, 2-Amino-4-imino-1,3-thiazolin-hydrochlorid, 5,5-Dimethylcyclohexan-1,3-dion, 2H-1,4-Benzoxazin-4H-3-on, 3-Ethyl-2-methyl-benzoxazoliumiodid, 3-Ethyl-2-methyl-benzothiazoliumiodid, 1-Ethyl-4-methyl-chinoliniumiodid, 1-Ethyl-2-methylchinoliniumiodid, 1,2,3-Trimethylchinoxaliniumiodid, 3-Ethyl-2-methyl-benzoxazolium-p-toluolsulfonat, 3-Ethyl-2-methyl-benzothiazolium-p-toluolsulfonat, 1-Ethyl-4-methyl-chinolinium-p-toluolsulfonat, 1-Ethyl-2-methylchinolinium-p-toluolsulfonat, 1,2,3-Trimethylchinoxalinium-p-toluolsulfonat, 1,2-Dihydro-1,3,4,6-tetramethyl-2-oxo-pyrimidinium-chlorid, 1,2-Dihydro-1,3,4,6-tetramethyl-2-oxo-pyrimidinium-hydrogensulfat, 1,2-Dihydro-1,3,4-trimethyl-2-oxo-pyrimidinium-chlorid, 1,2-Dihydro-4,6-dimethyl-1,3-dipropyl-2-oxo-pyrimidinium-chlorid, 1,2-Dihydro-1,3,4,6-tetramethyl-2-thioxo-pyrimidinium-hydrogensulfat und 2-Dihydro-1,3,4,5,6-pentamethyl-2-oxo-pyrimidinium-chlorid.

Die primären und sekundären aromatischen Amine der Komponente B sind bevorzugt ausgewählt aus der Gruppe bestehend aus N,N-Dimethyl-p-phenylendiamin, N,N-Diethyl-p-phenylendiamin, N-(2-Hydroxyethyl)-N-ethyl-p-phenylendiamin, N,N-Bis-(2-hydroxyethyl)-p-phenylendiamin, N-(2-Methoxyethyl)-p-phenylendiamin, 2,3-Dichlor-p-phenylendiamin, 2,4-Dichlor-p-phenylendiamin, 2,5-Dichlor-p-phenylendiamin, 2-Chlor-p-phenylendiamin, 2,5-Dihydroxy-4-morpholinoanilin, 2-Aminophenol, 3-Aminophenol, 4-Amino-phenol, 2-Aminomethyl-4-aminophenol, 2-Hydroxymethyl-4-aminophenol, o-Phenylendiamin, m-Phenylendiamin, p-Phenylendiamin, 2,5-Diaminotoluol, 2,5,-Diaminophenol, 2,5-Diaminoanisol, 2,5,Diaminophenethol, 4-Amino-3-methylphenol, 2-(2,5-Diaminophenyl)-ethanol, 2,4-Diaminophenoxyethanol, 2-(2,5-Diaminophenoxy)-ethanol, 3-Amino-4-(2-hydroxyethyloxy)phenol, 3,4-Methylendioxyphenol, 3,4-Methylendioxyanilin, 3-Amino-2,4-dichlorphenol, 4-Methylaminophenol, 2-Methyl-5-aminophenol, 3-Methyl-4-aminophenol, 2-Methyl-5-(2-hydroxyethylamino)phenol, 3-Amino-2-chlor-6-methylphenol, 2-Methyl-5-amino-4-chlorphenol, 5-(2-Hydroxyethylamino)-4-methoxy-2-methylphenol, 4-Amino-2-hydroxymethylphenol, 2-(Diethylaminomethyl)-4-aminophenol, 4-Amino-1-hydroxy-2-(2-hydroxyethylaminomethyl)-benzol, 1-Hydroxy-2-amino-5-methyl-benzol, 1-Hydroxy-2-amino-6-methyl-benzol, 2-Amino-5-acetamido-phenol, 1,3-Dimethyl-2,5-diaminobenzol, 5-(3-Hydroxypropylamino-)2-methylphenol, 5-Amino-4-methoxy-2-methylphenol, N,N-Dimethyl-3-aminophenol, N-Cyclopentyl-3-aminophenol, 5-Amino-4-fluor-2-methylphenol, 2,4-Diamino-5-fluortoluol, 2,4-Diamino-5-(2-hydroxyethoxy)-toluol, 2,4-Diamino-5-methylphenetol, 3,5-Diamino-2-methoxy-1-methylbenzol, 2-Amino-4-(2-hydroxyethylamino)-anisol, 2,6-Bis-(2-hydroxyethylamino)-1-methylbenzol, 1,3-Diamino-2,4-dimethoxybenzol, 3,5-Diamino-2-methoxy-toluol, 2-Aminobenzoesäure, 3-Aminobenzoesäure, 4-Aminobenzoesäure, 2-Aminophenylessigsäure, 3-Aminophenylessigsäure, 4-Aminophenylessigsäure, 2,3-Diaminobenzoesäure, 2,4-Diaminobenzoesäure, 2,5-Diaminobenzoesäure, 3,4-Diaminobenzoesäure 3,5-Diaminobenzoesäure, 4-Aminosalicylsäure, 5-Aminosalicylsäure, 3-Amino-4-hydroxy-benzoesäure, 4-Amino-3-hydroxybenzoesäure, 2-Aminobenzolsulfonsäure, 3-Aminobenzolsulfonsäure, 4-Aminobenzolsulfonsäure, 3-Amino-4-hydroxybenzolsulfonsäure, 4-Amino-3-hydroxynaphthalin-1-sulfonsäure, 6-Amino-7-hydroxynaphthalin-2-sulfonsäure, 7-Amino-4-hydroxynaphthalin-2-sulfonsäure, 4-Amino-5-hydroxynaphthalin-2,7-disulfonsäure, 3-Amino-2-naphthoesäure, 3-Aminophthalsäure, 5-Aminoisophthalsäure, 1,3,5-Triaminobenzol, 1,2,4-Triaminobenzol, 1,2,4,5-Tetraaminobenzol, 2,4,5-Triaminophenol, Pentaaminobenzol, Hexaaminobenzol, 2,4,6-Triaminoresorcin, 4,5-Diaminobrenzcatechin, 4,6-Diaminopyrogallol, 1-(2-Hydroxy-5-aminobenzyl)-2-imidazolidinon, 4-Amino-2-((4-[(5-amino-2-hydroxyphenyl)methyl]-piperazinyl)methyl)phenol, 3,5-Diamino-4-hydroxybrenzcatechin, 1,4-Bis-(4-aminophenyl)-1,4-diazacyclo-heptan, aromatische Nitrile, wie 2-Amino-4-hydroxybenzonitril, 4-Amino-2-hydroxybenzonitril, 4-Aminobenzonitril, 2,4-Diaminobenzonitril, Nitrogruppen-haltige Aminoverbindungen, wie 3-Amino-6-methylamino-2-nitro-pyridin, Pikraminsäure, [8-[(4-Amino-2-nitrophenyl)-azo]-7-hydroxy-naphth-2-yl]-trimethylammoniumchlorid, [8-((4-Amino-3-nitrophenyl)-azo)-7-hydroxy-naphth-2-yl]-trimethylammoniumchlorid (Basic Brown 17), 1-Hydroxy-2-amino-4,6-dinitrobenzol, 1-Amino-2-nitro-4-[bis-(2-hydroxyethyl)amino]-benzol, 1-Amino-2-[(2-hydroxyethyl)amino]-5-nitrobenzol (HC Yellow Nr. 5), 1-Amino-2-nitro-4-[(2-hydroxyethyl)amino]-benzol (HC Red Nr. 7), 2-Chlor-5-nitro-N-2-hydroxyethyl-1,4-phenylendiamin, 1-[(2-Hydroxyethyl)amino]-2-nitro-4-amino-benzol (HC Red Nr. 3), 4-Amino-3-nitrophenol, 4-Amino-2-nitrophenol, 6-Nitro-o-toluidin, 1-Amino-3-methyl-4-[(2-hydroxyethyl)amino]-6-nitrobenzol (HC Violet Nr. 1), 1-Amino-2-nitro-4-[(2,3-dihydroxypropyl)amino]-5-chlor-benzol (HC Red Nr. 10), 2-(4-Amino-2-nitroanilino)-benzoesäure, 6-Nitro-2,5-diaminopyridin, 2-Amino-6-chlor-4-nitrophenol, 1-Amino-2-(3-nitrophenylazo)-7-phenylazo-8-naphthol-3,6-disulfonsäure Dinatriumsalz (Acid blue Nr. 29), 1-Amino-2-(2-hydroxy-4-nitrophenylazo)-8-naphthol-3,6-disulfonsäure Dinatriumsalz (Palatinchrome green), 1-Amino-2-(3-chlor-2-hydroxy-5-nitrophenylazo)-8-naphthol-3,6-disulfonsäure Dinatriumsalz (Gallion), 4-Amino-4'-nitrostilben-2,2'-disulfonsäure Dinatriumsalz, 2,4-Diamino-3',5'-dinitro-2'-hydroxy-5-methyl-azobenzol (Mordant brown 4), 4'-Amino-4-nitrodiphenylamin-2-sulfonsäure, 4'-Amino-3'-nitrobenzophenon-2-carbonsäure, 1-Amino-4-nitro-2-(2-nitrobenzylidenamino)-benzol, 2-[2-(Diethylamino)ethylamino]-5-nitroanilin, 3-Amino-4-hydroxy-5-nitrobenzolsulfonsäure, 3-Amino-3'-nitrobiphenyl, 3-Amino-4-nitro-acenaphthen, 2-Amino-1-nitronaphthalin, 5-Amino-6-nitrobenzo-1,3-dioxol, Aniline, insbesondere Nitrogruppen-haltige Aniline, wie 4-Nitroanilin, 2-Nitroanilin, 1,4-Diamino-2-nitrobenzol, 1,2-Diamino-4-nitrobenzol, 1-Amino-2-methyl-6-nitrobenzol, 4-Nitro-1,3-phenylendiamin, 2-Nitro-4-amino-1-(2-hydroxyethylamino)-benzol, 2-Nitro-1-amino-4-[bis-(2-hydroxyethyl)-amino]-benzol, 4-Amino-2-nitrodiphenylamin-2'-carbonsäure, 1-Amino-5-chlor-4-(2-hydroyethylamino)-2-nitrobenzol, aromatische Aniline bzw. Phenole mit einem weiteren aromatischen Rest, wie sie in der Formel (Ox5) dargestellt sind 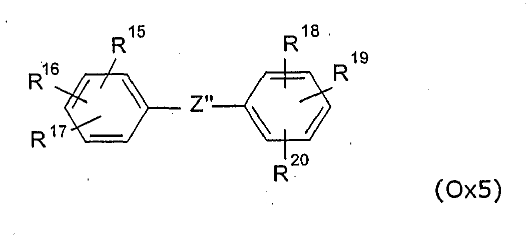 in der
- R¹⁵ für eine Hydroxy- oder eine Aminogruppe, die durch C₁-C₆-Alkyf-, C₁-C₆-Hydroxyalkyl-, C₁-C₆-Alkoxy- oder C₁-C₆-Alkoxy-C₁-C₆-alkylgruppen substituiert sein kann, steht,
- R¹⁶, R¹⁷, R¹⁸, R¹⁹ und R²⁰ unabhängig voneinander für ein Wasserstoffatom, eine Hydroxy- oder eine Aminogruppe, die durch C₁-C₆-Alkyl-, C₁-C₆-Hydroxyalkyl-, C₁-C₆-Alkoxy-, C₁-C₆-Aminoalkyl- oder C₁-C₆-Alkoxy-C₁-C₆-alkylgruppen substituiert sein kann, stehen, und
- Z" für eine direkte Bindung, eine gesättigte oder ungesättigte, ggf. durch Hydroxygruppen substituierte Kohlenstoffkette mit 1 bis 4 Kohlenstoffatomen, eine Carbonyl-, Sulfonyl- oder Iminogruppe, ein Sauerstoff- oder Schwefelatom, oder eine Gruppe mit der Formel (Ox6) 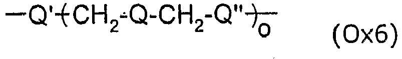
in der
- Q eine direkte Bindung, eine CH₂- oder CHOH-Gruppe bedeutet,
- Q' und Q" unabhängig voneinander für ein Sauerstoffatom, eine NR²¹-Gruppe, worin R²¹ ein Wasserstoffatom, eine C₁-C₆-Alkylgruppe oder C₁-C₆-Hydroxyalkylgruppe, wobei auch beide Gruppen zusammen mit dem Restmolekül einen 5-, 6- oder 7-Ring bilden können, bedeutet, die Gruppe O-(CH₂)ₚ-NH oder NH-(CH₂)ₚ'-O', worin p und p' 2 oder 3 sind, stehen und
- o eine Zahl von 1 bis 4 bedeutet,
wie insbesondere 4,4'-Diaminostilben und dessen Hydrochlorid, 4,4'-Diaminostilben-2,2'-disulfonsäure-mono- oder -di-Na-Salz, 4-Amino-4'-dimethylaminostilben und dessen Hydrochlorid, 4,4'-Diaminodiphenylmethan, 4,4'-Diaminodiphenylsulfid, 4,4'-Diaminodiphenylsulfoxid, 4,4'-Diaminodiphenylamin, 4,4'-Diaminodiphenylamin-2-sulfonsäure, 4,4'-Diaminobenzophenon, 4,4'-Diaminodiphenylether, 3,3',4,4'-Tetraaminodiphenyl, 3,3',4,4'-Tetraamino-benzophenon, 1,3-Bis-(2,4-diaminophenoxy)-propan, 1,8-Bis-(2,5-diaminophenoxy)-3,6-dioxaoctan, 1,3-Bis-(4-aminophenylamino)propan, , 1,3-Bis-(4-aminophenylamino)-2-propanol, 1,3-Bis-[N-(4-aminophenyl)-2-hydroxyethylamino]-2-propanol, N,N-Bis-[2-(4-amino-phenoxy)-ethyl]-methylamin, N-Phenyl-1,4-phenylendiamin und Bis-(5-amino-2-hydroxyphenyl)-methan.

Die stickstoffhaltigen heterozyklischen Verbindungen der Komponente B sind bevorzugt ausgewählt aus der Gruppe bestehend aus 2-Aminopyridin, 3-Aminopyridin, 4-Aminopyridin, 2-Amino-3-hydroxy-pyridin, 2,6-Diamino-pyridin, 2,5-Diamino-pyridin, 2-(Aminoethylamino)-5-aminopyridin, 2,3-Diamino-pyridin, 2-Dimethylamino-5-amino-pyridin, 2-Methylamino-3-amino-6-methoxy-pyridin, 2,3-Diamino-6-methoxy-pyridin, 2,6-Dimethoxy-3,5-diamino-pyridin, 2,4,5-Triamino-pyridin, 2,6-Dihydroxy-3,4-dimethylpyridin, N-[2-(2,4-Diaminophenyl)aminoethyl]-N-(5-amino-2-pyridyl)-amin, N-[2-(4-Aminophenyl)aminoethyl]-N-(5-amino-2-pyridyl)-amin, 2,4-Dihydroxy-5,6-diaminopyrimidin, 4,5,6-Triaminopyrimidin, 4-Hydroxy-2,5,6-triaminopyrimidin, 2-Hydroxy-4,5,6-triaminopyrimidin, 2,4,5,6-Tetraaminopyrimidin, 2-Methylamino-4,5,6-triaminopyrimidin, 2,4-Diaminopyrimidin, 4,5-Diaminopyrimidin, 2-Amino-4-methoxy-6-methyl-pyrimidin, 3,5-Diaminopyrazol, 3,5-Diamino-1,2,4-triazol, 3-Aminopyrazol, 3-Amino-5-hydroxypyrazol, 1-Phenyl-4,5-diaminopyrazol, 1-(2-Hydroxyethyl)-4,5-diaminopyrazol, 1-Phenyl-3-methyl-4,5-diaminopyrazol, 4-Amino-2,3-dimethyl-1-phenyl-3-pyrazolin-5-on (4-Aminoantipyrin), 1-Phenyl-3-methylpyrazol-5-on, 2-Aminochinolin, 3-Aminochinolin, 8-Aminochinolin, 4-Aminochinaldin, 2-Aminonicotinsäure, 6-Aminonicotinsäure, 5-Aminoisochinolin, 5-Aminoindazol, 6-Aminoindazol, 5-Aminobenzimidazol, 7-Aminobenzimidazol, 5-Aminobenzothiazol, 7-Aminobenzothiazol, 2,5-Dihydroxy-4-morpholino-anilin sowie Indol- und Indolinderivaten, wie 4-Aminoindol, 5-Aminoindol, 6-Aminoindol, 7-Aminoindol, 5,6-Dihydroxyindol, 5,6-Dihydroxyindolin und 4-Hydroxyindolin. Weiterhin als heterozyklische Verbindungen können erfindungsgemäß die in der DE-U1-299 08 573 offenbarten Hydroxypyrimidine eingesetzt werden. Die vorgenannten Verbindungen können sowohl in freier Form als auch in Form ihrer physiologisch verträglichen Salze, z. B. als Salze anorganischer Säuren, wie Salz- oder Schwefelsäure, eingesetzt werden.

Die aromatischen Hydroxyverbindungen der Komponente B sind bevorzugt ausgewählt aus der Gruppe bestehend aus 2-Methylresorcin, 4-Mathylresorcin, 5-Methylresorcin, 2,5-Dimethylresorcin, Resorcin, 3-Methoxyphenol, Brenzkatechin, Hydrochinon, Pyrogallol, Phloroglucin, Hydroxyhydrochinon, 2-Methoxyphenol, 3-Methoxyphenol, 4-Methoxyphenol, 3-Diethylamino-phenol, 2-(2-Hydroxyethyl)phenol, 3,4-Methylendioxyphenol, 2,4-Dihydroxybenzoesäure, 3,4-Dihydroxybenzoesäure, 1-(2,4-Dihydroxy-phenyl)essigsäure, 1-(3,4-Dihydroxy-phenyl)essigsäure, Gallussäure, 2,4,6-Trihydroxybenzoesäure, -acetophenon, 2-Chlorresorcin, 4-Chlorresorcin, 1-Naphthol, 1,5-Dihydroxynaphthalin, 2,3-Dihydroxynaphthalin, 2,7-Dihydroxynaphthalin, 6-Dimethylamino-4-hydroxy-2-naphthalinsulfonsäure und 3,6-Dihydroxy-2,7-naphthalinsulfonsäure.

In einer weiteren bevorzugten Ausführungsform liegen die kosmetischen Zubereitungen als Flüssigkeit, vorzugsweise als Dispersion, Emulsion, Lösung oder Gel, besonders bevorzugt mit einer Viskosität von 500 bis 4000 mPas, weiter bevorzugt 5000 bis 35000 mPas, insbesondere von 10000 bis 35000 mPas, im Speziellen von 20000 bis 32000 mPas (Brookfield Viskosimeter LVT-II bei 4 U/min und 20 °C, Spindel 5).

Wie bereits zuvor beschrieben, weisen die kosmetischen Zubereitungen vorzugsweise einen Wassergehalt unterhalb von 20 Gew.-%, vorzugsweise unterhalb von 12 Gew.-%, besonders bevorzugt unterhalb von 8 Gew.-%, weiter bevorzugt unterhalb von 4 Gew.-%, insbesondere unterhalb von 2 Gew.-%, jeweils bezogen auf die gesamte kosmetische Zubereitung, auf.

Die aus dem Stand der Technik bekannten flüssigen Färbesysteme für Keratinmaterialien enthalten als Lösungsmittel nahezu ausschließlich Wasser oder Gemische von Wasser mit niedermolekularen Alkoholen wie Ethanol und/oder Isopropanol. Für die Wahl dieser Lösungsmittel spielen zum Einen physiologische Gesichtspunkte eine Rolle, zum Anderen ist eine Anfärbung des Haarinneren nur bei geeigneten Transportmedien sowie eine Reaktion bei reaktionsfähigen Systemen nur bei einem geeigneten Reaktionsmedium gewährleistet. Diese Bedingungen sind bei Wasser bzw. Wasser-/Alkoholgemischen optimal erfüllt. Die Verwendung der angeführten Lösungsmittel ist allerdings nicht nur mit Vorteilen verbunden. So unterliegen einige Farbstoffe bei Lagerung in wässrigen beziehungsweise wässrigalkoholischen Medien der Hydrolyse beziehungsweise lösen sich nur unzureichend. Diese Nachteile können prinzipiell durch eine Lagerung, z.B. in Pulverform überwunden werden. Aber durch diese Zubereitungsart stellt nicht immer ein optimale Lösung dar. So ist die für eine weitgehende Lösung aller Komponenten notwendige feinteilige Dispergierung oftmals nicht sichergestellt.

In einer weiteren bevorzugten Ausführungsform weisen die Haarfärbemittel oder Haarfärbemittelvorstufen, insbesondere die Komponente A der Oxohaarfärbemittel, eine Wasserlöslichkeit unterhalb von 5 Gew.-%, vorzugsweise unterhalb von 2 Gew.-%, insbesondere unterhalb von 1 Gew.-% auf.

Geeignete schlecht wasserlösliche Haarfärbemittelvorstufen sind aus der deutschen Auslegeschrift DE 2932489, aus der aromatische Aldehyde bekannt sind, und aus der deutschen Patentanmeldung DE 196 30 275, in der Vinyloge, aromatische Aldehyde beschrieben sind, bekannt. In den genannten Druckschriften sind zahlreiche Verbindungen beschrieben, die nur eine begrenzte Wasserlöslichkeit von weniger als 1 g/l (20 °C) aufweisen. Besonders bevorzugt sind die aus der WO 95/24886 bekannten Isatinderivate. Farbstoffe, die ausgewählt sind aus der Gruppe der Isatinderivate oder der aromatischen oder vinylogen Carbonylverbindungen sind besonders bevorzugt, da diese Farbstoffe in Wasser oft schwer löslich sind. Diese Farbstoffvorprodukte werden häufig für die Oxofärbung eingesetzt. Besonders bevorzugte kosmetische Zubereitungen enthalten das 1-Allylisatin, das 1-Diethylaminomethylisatin, das 1-Diethylaminomethylisatin, das 1-Pi-peridinomethylisatin, der 4-Hydroxy-3-methoxyzimtaldehyd, das Glutaconaldehyd-tetra-butylammoniumsalz sowie der 2-(1,3,3,-Trimethyl-2-indolyliden)-acetaldehyd.

Vorzugsweise besitzen die Farbstoffe oder Farbstoffvorprodukte eine gute Öllöslichkeit. Unter öllöslich im Rahmen der vorliegenden Erfindung werden Substanzen verstanden, deren Löslichkeit in Paraffinöl bei 20 °C oberhalb 0,1 Gew.-% liegt.

Es hat sich gezeigt, dass insbesondere für die Herstellung wasserarmer kosmetischer Zubereitung, insbesondere wasserarmer Haarfärbemittel-Zubereitungen zusätzlich Öle eingesetzt werden können. Bevorzugt werden flüssige Ölkomponenten eingesetzt.

Flüssige Ölkomponenten im Sinne der vorliegenden Erfindung sind alle physiologisch verträglichen, bei 20° C flüssigen mineralischen, tierischen, pflanzlichen oder synthetischen Ölkomponenten. Beispiele für solche Ölkomponenten sind z.B. Paraffinöle, Silikonöle, Triglyceridöle, z.B. Klauenöl, Lardöl, Nerzöl, Olivenöl, Sonnenblumenöl, Mandelöl, flüssige Wachsester wie z.B. Spermöl, Jojobaöl, synthetische Ester wie z.B. Glycerin-tricaprylat, n-Hexyllaurat, Isopropylmyristat, 2-Ethylhexyl-stearat, Butyloleat, synthetische Ether wie z.B. Di-n-octylether, synthetische Kohlenwasserstoffe wie z.B. Diisooctyl-cyclohexan, Squalan, synthetische Alkohole wie z.B. 2-Octyldodecanol oder 2-Hexyl-decanol.

Besonders bevorzugt enthalten die kosmetischen Zubereitungen zusätzlich ein Öl ausgewählt aus der Gruppe
a) Mineralöle, vorzugsweise Paraffinöle,
b) pflanzliche Öle, vorzugsweise Sonnenblumenöl, Rapsöl, Sojabohnenöl, Rizinusöl,
c) Silikonöle, vorzugsweise quarternisierte Silikone,
d) Ester von C₁₀-C₃₆-Fettsäuren, vorzugsweise Ester von C₁₄-C₂₈-Fettsäuren und
e) Dialkylether mit mindestens einem Kohlenstoffrest, der 6 oder mehr Kohlenstoffatome trägt.

In einer weiteren bevorzugten Ausführungsform enthalten die kosmetischen Formulierungen vorteilhafterweise Komponenten, die beim Lösen in Wasser eine größere Hydratationswärme freisetzen und aufgrund der Wärmeentwicklung, insbesondere bei Haarfärbemittelzubereitung, zu einem verbesserten Farbaufzug führen. Vorzugsweise enthalten die kosmetischen Zubereitungen eine oder mehrere Komponenten mit einem exothermen Löslichkeitsverhalten in Wasser, vorzugsweise ausgewählt aus der Gruppe
a) Alkali- oder Erdalkalisalze, vorzugsweise Alkali- oder Erdalkalihalogenide und/oder -sulfate, insbesondere Caliumchlorid und/oder Magnesiumsulfat und/oder dehydratisierte Zeolithe und
b) niedermolekulare Polyole, vorzugsweise Glycerin, Propylenglycol oder Polyethylenglycol.

Für die wasserfreien, vorzugsweise ölhaltigen kosmetischen Formulierungen hat sich überraschenderweise gezeigt, dass der für die stabile feinteilige Dispergierung notwendige Viskositätsaufbau von unpolaren oder semipolaren Ölen durch verschiedene Zusätze erreicht werden kann. In einer bevorzugten Ausführungsform der vorliegenden Erfindung weisen die kosmetischen Zubereitungen ein oder mehrere viskositätsregulierende Zusätze auf, die ausgewählt sind aus
a) Ester oder Amide von Di-, Tri-, Tetra- oder Polyolen, insbesondere Dextrin ein oder mehrfach verestert mit Palmitinsäure oder N-Lauroyl-1-glutaminsäure,α,γ-di-n-butylamid,
b) Ester von Di- oder Oligocarbonsäuren, insbesondere Di-behenylfumarsäureester,
c) Schichtsilikaten, vorzugsweise organisch modifizierte, insbesondere hydrophob modifizierte Schichtsilikate,
d) Mono- oder Diglyceride von C₁₂-C₂₂-Fettsäuren
e) Alkali-, Erdalkali- und Aluminiumsalze von Fettsäuren und/oder Hydroxycarbonsäuren, insbesondere die Lithiumsalze von C3-C14-Hydroxycarbonsäuren,
f) Aerosile, vorzugsweise SiO₂ und/oder TiO₂, besonders bevorzugt solche mit einer mittleren Partikelgröße unterhalb von 100 µm, insbesondere unterhalb von 100 µm,
g) Polyole, vorzugsweise Polyethylenglycole und/oder Polypropylenglycole, besonders bevorzugt Polyole mit einem mittleren Molekulargewicht unterhalb von 20000,
h) Dibenzyliden-Sorbitole sowie deren Derivate,
i) Copolymere mit Aminodithiazolen,
j) Pfropfcopolymere von Polyvinylpyridin mit sulfoniertem Polyisobutylen,
k) Vernetzte Polyamine und/oder Polyimine
l) Polymere ausgewählt aus i) Gummi-basierten Blockcopolymeren, ii) Silikonölen mit einer Viskosität oberhalb von 2000 mPas, iii) mikrokristalline Wachse und
m) Ethylen-Vinylacetat-Copolymere.

Besonders bevorzugt als viskositätsregulierende Zusätze für die wasserarmen kosmetischen Formulierungen in den erfindungsgemäßen Portionen haben sich Dibenzylidensorbitole sowie deren Derivate herausgestellt, die in der US 6,338,841 B1 beschrieben werden und deren Inhalt in die Anmeldung mit aufgenommen wird. Weiterhin bevorzugt sind die Copolymere mit Aminodiazolen, wie sie in der US 5,472,627 beschrieben werden und auf deren Inhalt explizit Bezug genommen wird. Weiterhin bevorzugt sind Pfropfcopolymere von Polyvinylpyridin mit sulfoniertem Polyisobutylen wie sie in der US 5,328,960 beschrieben werden und deren Inhalt voll umfänglich in diese Anmeldung aufgenommen wird. Weiterhin bevorzugt sind die quervernetzen Polyamine und/oder Polyimine, wie sie explizit in der WO 01/46373 A1 offenbart werden. Speziell bevorzugt sind ebenfalls die Ester von Di- oder Oligocarbonsäuren insbesondere Dibehenylfumarsäureester, wie sie in der WO 99/51191 offenbart werden. Weitere bevorzugte viskositätsregulierende Zusätze sind Polymere ausgewählt aus I) gummibasierten Blockcopolymeren, II) Siliconölen mit einer Viskosität oberhalb von 2000 mPas, III) mikrokristallinen Wachsen, wie sie in der WO 98/30193 beschrieben werden. Als besonders vorteilhaft für den Viskositätsaufbau in wasserarmen Haarfärbemittelsystemen haben sich die Lithiumsalze von C₃-C₁₄-Hydroxycarbonsäuren herausgestellt, wie sie explizit in der WO 98/11180 beschrieben werden. Vorteilhaft einsetzbar sind ebenfalls Ethylen-Vinylacetat-Copolymere, wie sie in der WO 97/07158 beschrieben werden und deren Inhalt voll umfänglich mit in diese Anmeldung aufgenommen wird.

Die kosmetischen Zubereitungen können darüber hinaus weitere Wirk- und Zusatzhilfsstoffe enthalten.

In vielen Fällen enthalten die Färbemittel mindestens ein Tensid, wobei prinzipiell sowohl anionische als auch zwitterionische, ampholytische, nichtionische und kationische Tenside geeignet sind. Es hat es sich als vorteilhaft erwiesen, die Tenside aus anionischen, zwitterionischen oder nichtionischen Tensiden auszuwählen.

Als anionische Tenside eignen sich in Zubereitungen alle für die Verwendung am menschlichen Körper geeigneten anionischen oberflächenaktiven Stoffe. Diese sind gekennzeichnet durch eine wasserlöslichmachende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ -CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₆-C₂₂-Carbonsäuren, wie Ölsäure, Stearinsäure, Isostearinsäure und Palmitinsäure.

Nichtionogene Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Glycerin,
- C₈-C₂₂-Alkylmono- und -oligoglycoside und deren ethoxylierte Analoga sowie
- Anlagerungsprodukte von 5 bis 60 Mol Ethylenoxid an Rizinusöl und gehärtetes Rizinusöl.

Bevorzugte nichtionische Tenside sind Alkylpolyglykoside der allgemeinen Formel R¹O-(Z)_{X}. Diese Verbindungen sind durch die folgenden Parameter gekennzeichnet.

Der Alkylrest R¹ enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Die erfindungsgemäß verwendbaren Alkylpolyglykoside können beispielsweise nur einen bestimmten Alkylrest R¹ enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.

Besonders bevorzugt sind solche Alkylpolyglykoside; bei denen R¹
- im wesentlichen aus C₈- und C₁₀-Alkylgruppen,
- im wesentlichen aus C₁₂- und C₁₄-Alkylgruppen,
- im wesentlichen aus C₈-C₁₆-Alkylgruppen oder
- im wesentlichen aus C₁₂-C₁₆-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylpolyglykoside mit x-Werten von 1,1 bis 1,6 sind bevorzugt. Ganz besonders bevorzugt sind Alkylglykoside, bei denen x 1,1 bis 1,4 beträgt.

Die Alkylglykoside können neben ihrer Tensidwirkung auch dazu dienen, die Fixierung von Duftkomponenten auf dem Haar zu verbessern. Der Fachmann wird also für den Fall, dass eine über die Dauer der Haarbehandlung hinausgehende Wirkung des Parfümöles auf dem Haar gewünscht wird, bevorzugt zu dieser Substanzklasse als weiterem Inhaltsstoff der erfindungsgemäßen Zubereitungen zurückgreifen.

Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Weiterhin können, insbesondere als Co-Tenside, zwitterionische Tenside verwendet werden. Als zwitterionische Tenside werden solche oberflächenaktive Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammonium-glycinat, N-Acyl-aminopropyl-N,N-dimethyl-ammoniumglycinate, beispielsweise das Kokosacylaminopropyl-dimethylammoniumglycinat, und 2-Alkyl-3-carboxylmethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Ebenfalls insbesondere als Co-Tenside geeignet sind ampholytische Tenside. Unter ampholytischen Tensiden werden solche oberflächenaktiven Verbindungen verstanden, die außer einer C₈-C₁₈-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Erfindungsgemäß werden als kationische Tenside insbesondere solche vom Typ der quartären Ammoniumverbindungen, der Esterquats und der Amidoamine eingesetzt.

Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalze von Fettsäuren mit 1,2-Dihydroxypropyldialkylaminen. Solche Produkte werden beispielsweise unter den Warenzeichen Stepantex®, Dehyquart® und Armocare® vertrieben. Die Produkte Armocare® VGH-70, ein N,N-Bis(2-Palmitoyloxyethyl)dimethylammoniumchlorid, sowie Dehyquart® F-75 und Dehyquart® AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid® S 18 im Handel erhältliche Stearamidopropyl-dimethylamin dar.

Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquaternäre Polydimethylsiloxane, Quaternium-80).

Ein Beispiel für ein als kationisches Tensid einsetzbares quaternäres Zuckerderivat stellt das Handelsprodukt Glucquat®100 dar, gemäß INCI-Nomenklatur ein "Lauryl Methyl Gluceth-10 Hydroxypropyl Dimonium Chloride".

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so dass man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Ferner können die erfindungsgemäßen Färbemittel weitere Wirk-, Hilfs- und Zusatzstoffe, wie beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quaternisierte Celluloseether, Polysiloxane mit quaternären Gruppen, Dimethyldiallylammoniumchlorid-Polymere, Acrylamid-Dimethyldiallyl-ammoniumchlorid-Copolymere, mit Diethylsulfat quaternierte Dimethylamino-ethylmethacrylat-Vinylpyrrolidon-Copolymere, Vinylpyrrolidon-Imidazolinium-methochlorid-Copolymere und quaternierter Polyvinylalkohol,
- zwitterionische und amphotere Polymere wie beispielsweise Acrylamidopropyl-trimethylammoniumchlorid/Acrylat-Copolymere und Octylacrylamid/Methyl-methacrylat/tert-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Malein-säureanhydrid-Copolymere und Acrylsäure/Ethylacrylat/N-tert.Butyl-acrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Maleinsäure und Milchsäure,
- haarkonditionierende Verbindungen wie Phospholipide, beispielsweise Sojalecithin, Ei-Lecitin und Kephaline,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- faserstrukturverbessernde Wirkstoffe, insbesondere Mono-, Di- und Oligosaccharide wie beispielsweise Glucose, Galactose, Fructose, Fruchtzucker und Lactose,
- quaternierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- Substanzen zur Einstellung des pH-Wertes, wie beispielsweise übliche Säuren, insbesondere Genußsäuren und Basen,
- Wirkstoffe wie Allantoin, Pyrrolidoncarbonsäuren und deren Salze sowie Bisabolol,
- Vitamine, Provitamine und Vitaminvorstufen, insbesondere solche der Gruppen A, B₃, B₅, B₆, C, E, F und H,
- Pflanzenextrakte wie die Extrakte aus Grünem Tee, Eichenrinde, Brennessel, Hamamelis, Hopfen, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel,.
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Stabilisierungsmittel für Wassserstoffperoxid und andere Oxidationsmittel,
- Treibmittel wie Propan-Butan-Gemische, N₂O, Dimethylether, CO₂ und Luft,
- Antioxidantien,
enthalten

Bezüglich weiterer fakultativer Komponenten sowie die eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Die eigentliche oxidative Färbung der Fasern kann grundsätzlich mit Luftsauerstoff erfolgen. Bevorzugt wird jedoch ein chemisches Oxidationsmittel eingesetzt, besonders dann, wenn neben der Färbung ein Aufhelleffekt an menschlichem Haar gewünscht ist. Als Oxidationsmittel kommen Persulfate, Chlorite und insbesondere Wasserstoffperoxid oder dessen Anlagerungsprodukte an Harnstoff, Melamin sowie Natriumborat in Frage. Erfindungsgemäß kann aber das Oxidationsfärbemittel auch zusammen mit einem Katalysator auf das Haar aufgebracht werden, der die Oxidation der Farbstoffvorprodukte, z.B. durch Luftsauerstoff, aktiviert. Solche Katalysatoren sind z.B. Metallionen, Iodide, Chinone oder bestimmte Enzyme.

Geeignete Metallionen sind beispielsweise Zn²⁺, Cu²⁺, Fe²⁺, Fe³⁺, Mn²⁺, Mn⁴⁺, Li⁺, Mg²⁺, Ca²⁺ und Al³⁺. Besonders geeignet sind dabei Zn²⁺, Cu²⁺ und Mn²⁺. Die Metallionen können prinzipiell in der Form eines beliebigen, physiologisch verträglichen Salzes oder in Form einer Komplexverbindung eingesetzt werden. Bevorzugte Salze sind die Acetate, Sulfate, Halogenide, Lactate und Tartrate. Durch Verwendung dieser Metallsalze kann sowohl die Ausbildung der Färbung beschleunigt als auch die Farbnuance gezielt beeinflusst werden.

Geeignete Enzyme sind z.B. Peroxidasen, die die Wirkung geringer Mengen an Wasserstoffperoxid deutlich verstärken können. Weiterhin sind solche Enzyme erfindungsgemäß geeignet, die mit Hilfe von Luftsauerstoff die Oxidationsfarbstoffvorprodukte direkt oxidieren, wie beispielsweise die Laccasen, oder *in situ* geringe Mengen Wasserstoffperoxid erzeugen und auf diese Weise die Oxidation der Farbstoffvorprodukte biokatalytisch aktivieren. Besonders geeignete Katalysatoren für die Oxidation der Farbstoffvorläufer sind die sogenannten 2-Elektronen-Oxidoreduktasen in Kombination mit den dafür spezifischen Substraten, z.B.
- Pyranose-Oxidase und z.B. D-Glucose oder Galactose,
- Glucose-Oxidase und D-Glucose,
- Glycerin-Oxidase und Glycerin,
- Pyruvat-Oxidase und Benztraubensäure oder deren Salze,
- Alkohol-Oxidase und Alkohol (MeOH, EtOH),
- Lactat-Oxidase und Milchsäure und deren Salze,
- Tyrosinase-Oxidase und Tyrosin,
- Uricase und Harnsäure oder deren Salze,
- Cholinoxidase und Cholin,
- Aminosäure-Oxidase und Aminosäuren.

Ein weiterer Gegenstand der vorliegenden Erfindung ist ein Verfahren zur Herstellung der erfindungsgemäßen Portionen.

Das erfindungsgemäße Verfahren umfasst die folgenden Schritte:
a) Bereitstellen eines Behälters aus einer wasserlöslichen und/oder wasserdispergierbaren Umhüllung,
b) Befüllen des Behälters mit einer kosmetischen Zubereitung und
c) Versiegeln, vorzugsweise flüssigkeitsdichtes Versiegeln des Behälters
wobei vor dem Schritt a) oder während der Schritte a) bis c) oder nach dem Schritt c) durch Erhitzen und Pressen auf die Außen- und Innenfläche der Umhüllung ein dreidimensionales Muster so aufgebracht wird, dass die Umhüllung einen quadratischen Mittelwert der Rauheit von wenigstens 10 µm aufweist.

Vorzugsweise erfolgt die Herstellung der erfindungsgemäßen Portionen ganz oder teilweise nach einem Tiefziehverfahren, Spritzgussverfahren, Blasformverfahren, Rotary-Die-Verfahren oder insbesondere Schlauchbeutelsiegelverfahren.

Die Schritte a) bis c) werden in einer bevorzugten Ausführungsform mittels eines Tiefziehverfahrens durchgeführt. Dazu wird eine Folie aus einem der zuvor beschriebenen wasserlöslichen und/oder wasserdispergierbaren Polymere über eine oder mehrere Kavität(en) positioniert und anschließend an die Kavitäteninnenwände angelegt, vorzugsweise durch Anlegen eines Vakuums aus Öffnungen in der/den Kavität(en). Die Ausformung des zuvor beschriebene wasserlöslichen und/oder wasserdispergierbaren Tiefziehbehälters kann alternativ auch durch Einpressen/Einstanzen mittels eines Formwerkzeugs erfolgen.

Behälter im Rahmen der vorliegenden Erfindung sind Gegenstände, die befähigt sind stoffliche Dinge aufzunehmen.

Die geformten Behälter werden anschließend mit der kosmetischen Zubereitung befüllt und abschließend mit einer weiteren wasserlöslichen und/oder wasserdispergierbaren Folie, die gleich oder auch verschieden von der Folie sein kann, die den Tiefziehbehälter gebildet hat, entlang des Kavitätenrandes versieglt.

Vorzugsweise wird die Folie während des Tiefziehverfahrens erwärmt, beispielsweise durch Infrarotstrahlung. Das Erwärmen hat sich als vorteilhaft herausgestellt, da sich die Folien besser in die Kavitäten gezogen und an die Kavitätenwandungen angelegt werden können. Bevorzugt erfolgt das Tiefziehverfahren nach dem in der WO-A1-00/55068 beschriebenen Verfahren. In bevorzugten Ausführungsformen sind die Kavitäten domförmig ausgestaltet.

In einer bevorzugten Ausführungsform des Verfahrens weist die in dem Tiefziehverfahren eingesetzte Folie eine Oberfläche auf, die einen quadratischen Mittelwert der Rauheit oberhalb von 10 µm, vorzugsweise zwischen 10 und 100 µm, besonders bevorzugt von 10 bis 50 µm, insbesondere bevorzugt von 30 bis 35 µm aufweist. Vorzugsweise weisen die im Tiefziehverfahren eingesetzten Folien die zuvor für das Hüllmaterial der erfindungsgemäßen Portionen beschriebenen Eigenschaften auf.

In einer weiteren Ausführungsform des Tiefziehverfahrens erhält die Folie durch den Tiefziehvorgang die zuvor beschriebenen Oberflächeneigenschaften. Beispielsweise kann dies durch Tiefziehen einer nicht oder nur unvollständig rauen Folie in eine Kavität mit Wandungen in Matrizenform erfolgen. Der tiefgezogenen Folie wird dadurch eine dreidimensionale makroskopische Struktur aufgeprägt, die der gewünschten Rauheit der Umhüllung der erfindungsgemäßen Portionen entspricht. Ausbilden des Behälters und gleichzeitiges Aufprägen der Struktur kann alternativ auch durch ein Formwerkzeug erfolgen, dass die Folie in die Kavität presst und gleichfalls der Folie die gewünschte Struktur aufprägt.

Ein weiteres bevorzugtes Verfahren für die Schritte a) bis c) des erfindungsgemäßen Verfahrens ist das Rotary-Die-Verfahren.

Das Rotary-Die-Verfahren, ist beispielsweise in der WO 97/35537 zur Herstellung wasserlöslicher Kapseln beschrieben. Die Herstellung der Portionen erfolgt dadurch, dass eine kosmetische Zubereitung über eine Dosiervorrichtung mittels eines Füllkeils lokal zwischen zwei Bänder aus wasserlöslichem und/oder wasserdispergierbarem polymeren Thermoplast, wie zuvor beschrieben, eingespritzt wird, die sich auf zwei parallel zueinander drehbar gelagerten Formwalzen befinden, die an ihren Mantelflächen ringsum Hohlformen aufweisen, deren Gestalt jeweils einer halben herzustellenden Portion entspricht. Die Versiegelung erfolgt durch Druckkontakt der beiden Folienbänder. In einer bevorzugten Ausführungsform wird zur Verbesserung der Versiegelung mindestens eine der wasserlöslichen und/oder wasserdispergierbaren Folienbänder vor dem Formkörperbildungsprozess mit einem Lösungsmittel angelöst. Weiterhin bevorzugt ist die Hitzeversiegelung der beiden Portionshälften. Dazu können vorteilhafterweise die beiden Formwalzen als Elektroden für das dielektrische Verschmelzen der Folien miteinander dienen. Zur Erleichterung des Formbildungsprozesses mittels Einspritzen der kosmetischen Zubereitung kann vorteilhafterweise an den Kavitäten ein Vakuum angelegt werden. Dies führt dazu, dass der Injektionsdruck der Flüssigkeit verringert werden kann und somit die Gefahr der Kontamination des Polymermaterials mit flüssigem Füllgut an den Siegelpositionen herabgesetzt wird. Vorteilhafterweise weisen die Formwalzen im Bereich der Formwalzenstege Aufrauungen auf. Durch die Aufrauung der Formwalzenstege wird die Haftreibung für die Folienbänder aus denen die Portionen hergestellt werden vergrößert.

Über die Gestalt der Hohlformen in den Formwalzen lassen sich Portionen mit beliebigen Geometrien herstellen. Die einzige Vorgabe ist, dass sie eine Spiegelebene aufweisen. Geometrien wie Kugel, Eier, Kuben, Figuren sind im Rahmen dieser Erfindung bevorzugt. Über die Dosiervorrichtung wird eine genau abgemessene Menge der kosmetischen Zubereitung in die sich ausbildende wasserlösliche Portion injiziert. Von besonderem Vorteil ist es, wenn der Injektionsstoß von einem Rückhub der Dosiervorrichtung gefolgt wird. Es wird so ein Nachtropfen oder Fadenziehen des Mittels, was wiederum zu einer Kontamination der wasserlöslichen und/oder wasserdispergierbaren Folie im Bereich der Versiegelungsposition führen kann, vermieden.

Vorzugsweise weisen die Folienbänder, die in das Rotary-Die-Verfahren eingesetzt werden, bereits die zuvor beschriebenen Oberflächeneigenschaften des Hüllmaterials der erfindungsgemäßen Portionen auf. In einer weiteren Ausführungsform können den Folienbändern jedoch die erforderlichen Oberflächeneigenschaften während des Formens aufgeprägt werden. So können beispielsweise die Kavitäteninnenwandungen eine Matrizenstruktur aufweisen, die der Folie bei Kontakt mit der Wandung die erforderlichen Eigenschaften, insbesondere die Rauheit aufprägt. Ein Erwärmen der Kavitäten und/oder der Folienbänder sowie ggf. ein erhöhten Injektionsdruck und/oder ein Vakuum aus Öffnungen der Kavität heraus sind vorteilhaft für den Folienprägevorgang.

Ein weiteres bevorzugtes Verfahren bezüglich der Schritte a) bis c) ist das Blasformverfahren.

### Das Blasformverfahren umfasst die Schritte:

Urformen eines Vorformlings aus einer Blasformmasse basierend auf einem wasserlöslichen und/oder wasserdispergierbaren polymeren Thermoplast, wie zuvor beschrieben, Blasformen des Vorformlings in einer Kavität zu einem Hohlkörper; Füllen des Hohlkörpers mit einer kosmetischen Formulierung und flüssigkeitsdichtes Verschließen der so geformten und befüllten Portion.

Vorteilhafterweise erfolgt die Herstellung derart, dass in einem ersten Schritt mittels Extrudieren ein Vorformling, vorzugsweise in Form eines Schlauchstücks, herstellt, und in einem zweiten Schritt in einem Arbeitszyklus der Hohlkörper, vorzugsweise mittels eines unter Druck stehenden Gases, vorzugsweise Pressluft, geblasen, vorzugsweise zur endgültigen Hohlkörpergeometrie (entsprechend den Bemessungen der Kavität) und mit der kosmetischen Zubereitung gefüllt, flüssigkeitsdicht verschlossen, sowie anschließend entformt wird.

Geeignete Blasformverfahren umfassen Extrusionsblasen, Coextrusionsblasen, Spritz- Streckblasen und Tauchblasen. Die Kavität kann dabei aus mehrteiligen Formteilen aufgebaut sein, bevorzugt ist jedoch eine zweiteilige Kavität. Zur Abtrennung des Vorformlings und/oder zum Verschließen der Portionen wird in einer bevorzugten Ausführungsform eine Klinge verwendet, wie beispielsweise in der WO 01/64421 beschrieben. Besonders bevorzugt ist die Verwendung einer vibrierenden Schneideeinrichtung, wie explizit in der EP 924 047 offenbart. Die Befüll-Öffnung des Hohlkörpers nach dem Befüllen lässt sich weiterhin, vorzugsweise durch Materialschluss, bevorzugt mittels thermischer Behandlung, besonders bevorzugt durch Aufsetzen eines Schmelzkleckses, verschließen.

Die Befüll-Öffnung oder Öffnungen des Hohlkörper lassen sich vorteilhaft durch thermische Behandlung, vorzugsweise durch Verschmelzen der Wandungen, die an die Öffnung angrenzen, insbesondere mittels Klemmbacken, flüssigkeitsdicht verschließen.

Die Außenoberfläche der Umhüllung der mittels Blasformung hergestellten Portion wird während des Blasformens derart modifiziert, dass sie die zuvor beschriebenen vorteilhaften Eigenschaften aufweist. Der Außenfläche der Umhüllung wird ein dreidimensionales Muster aufgeprägt, beispielsweise durch entsprechend matrizenfähig ausgestaltete Kavitäteninnenwände an die sich der Thermoplast anlegt.

Spritzgussverfahren können ebenfalls zur Herstellung der erfindungsgemäßen Portionen herangezogen werden. Hierzu wird zunächst ein Behälter spritzgegossen, der vorteilhafterweise durch die Formwerkzeuge bereits die bevorzugten Oberflächeneigenschaften, wie zuvor beschrieben, aufgeprägt bekommt. Anschließend erfolgt die Befüllung mit der kosmetischen Zubereitung. Das Versiegeln der Spritzgussbehälter aus den wasserlöslichen und/oder wasserdispergierbaren Thermoplasten erfolgt vorzugsweise mit einer bereits geprägten Folie aus wasserlöslichem und/oder wasserdispergierbarem Thermoplast, der gleich oder verschieden bezüglich des Spritzgussbehältermaterials sein kann.

Besonders bevorzugt erfolgt das erfindungsgemäße Herstellungsverfahren nach dem Schlauchbeutelsiegelverfahren. Hierzu wird in einer bevorzugten Ausführungsform eine Folie aus wasserlöslichem und/oder wasserdispergierbarem Thermoplast, wie zuvor beschrieben, mit den zuvor beschriebenen Eigenschaften eingesetzt.

Aus den Folien wird zunächst ein Schlauchstück geformt, das einseitig versiegelt, vorzugsweise flüssigkeitsdicht versiegelt wird, nachfolgend mit einer kosmetischen Zubereitung befüllt und anschließend ein weiteres Mal versiegelt wird.

Die Herstellung von Schlauchbeuteln ist dem Fachmann geläufig. Es hat sich jedoch gezeigt, dass der Einsatz von Folien (Hüllmaterialien) mit den zuvor beschriebenen Oberflächeneigenschaften, besondere Vorteile bei der Versiegelung und der anschließenden Verarbeitbarkeit aufweisen. Insbesondere können die hergestellten Portionen gut zusammen gelagert werden ohne miteinander zu verkleben oder aneinander zu haften. Dies ist ein wichtiger Vorteil bei der industriellen maschinellen Großproduktion der erfindungsgemäßen Portionen. Ohne an die Theorie gebunden zu sein, wird vermutet, dass durch die Rauheit der Oberflächen der erfindungsgemäßen Portionen, die Kontaktfläche zu benachbarten Portionen, beispielsweise in größeren Packungseinheiten, verringert ist. Insbesondere bei der Weiterverarbeitung, beispielsweise das Einlegen der Portionen in einer Umverpackung, treten hier wesentlich weniger Störfälle auf, die durch die Anhaftung der Beutel mit der Umverpackung oder benachbarten Portionen resultieren, auf. Wie bereits zuvor beschrieben, besteht das Hüllmaterial der erfindungsgemäßen Portionen bevorzugt aus einem Polymerfilm eines wasserlöslichen und/oder wasserdispergierbaren Thermoplasten.

Die Umhüllung erhält das aufgeprägte Muster durch Erhitzen und Pressen, vorzugsweise vor dem Befüllen des Behälters mit einer kosmetischen Zubereitung.

Vorzugsweise handelt es sich bei dem Hüllmaterial um Blas- oder insbesondere Gießpolymerfolien, die im erwärmten Zustand mit einer Matrize geprägt werden, um ihnen die vorteilhaften Oberflächeneigenschaften zu verleihen. Vorzugsweise erfolgt der Prägevorgang derart, dass die Folie einseitig mit dem Matrizenwerkzeug gepresst wird, so dass einseitig eine dreidimensionale Struktur aufgeprägt wird, die sich auf der gegenüberliegenden Seite als "negativ" wiederfindet.

Üblicherweise werden die erfindungsgemäßen Portionen in einer Verkaufseinheit (Kit) mit einer oder mehreren weiteren Zubereitungen und/oder Kosmetikapplikationsvorrichtungen vermarktet. Ein weiterer Gegenstand der vorliegenden Erfindung ist daher ein Kit-enthaltend ein oder mehrerer erfindungsgemäße Portionen.

In einer weiteren bevorzugten Ausführungsform enthält das erfindungsgemäße Kit zusätzlich ein oder mehrere Bestandteile ausgewählt aus der Gruppe
a) Behältnis zum Anmischen und Auflösen der Portion und/oder
b) ein oder mehrere Behältnis(se) enthaltend mindestens eine weitere kosmetische Zubereitung, vorzugsweise eine Wasserstoffperoxid-Lösung oder Wasserstoffperoxid-Emulsion oder eine Pflegelotion; und/oder
c) eine Vorrichtung zum Vermischen oder Rühren und/oder
d) ein oder mehrere Sicherheitsmaterialien zur Vermeidung des unerwünschten Inkontakttretens der kosmetischen Zubereitung mit dem menschlichen Körper, vorzugsweise Handschuhe.

In einer besonders bevorzugten Ausführungsform enthält das erfindungsgemäße Kit mindestens eine erfindungsgemäße Portion mit einem Blondierpulver als Zubereitung, und einen Behälter, vorzugsweise eine Kunststoffflasche mit einer wässrigen Wasserstoffperoxidlösung oder Wasserstoffperoxidemulsion oder Wasserstoffperioxdispersion und ggf. zusätzlich eine Pflegelotion.

In einer weiteren bevorzugten Ausführungsform enthält das erfindungsgemäße Kit mindestens ein oder mehrere erfindungsgemäße Portionen, die als kosmetische Zubereitung Blondierpulver enthalten, ein oder mehrere Behältnisse, enthalten eine Wasserstoffperoxidemulsion oder Wasserstoffperoxiddispersion, eine Anrührschale zum Vermischen der Komponenten, einen Rührstab und Sicherheitshandschuhe sowie ggf. eine oder mehrere Strähnenhauben oder Strähnennadeln.

In einer weiteren bevorzugten Ausführungsform der Erfindung enthält das erfindungsgemäße Kit mindestens ein oder mehrere erfindungsgemäße Portionen enthaltend ein Haarfärbemittel, vorzugsweise eine Haarfärbemittelvorstufe, besonders bevorzugt die Komponente A eines Oxofärbemittels. Weiterhin enthält das Kit vorzugsweise zusätzlich ein oder mehrere Portionen enthaltend eine weitere Haarfärbekomponente, vorzugsweise die Komponente B eines Oxofärbemittels sowie ggf. zusätzlich eine Oxidationsmittelzubereitung (C), die entweder wässrig, z.B. im Falle von Wasserstoffperoxid sein kann oder auch in Form in eines wasserfreien Pulvers. z.B. eines Wasserstoffperoxidadukts an Harnstoff (Percarbamid oder an Melamin (Melaminperhydrat) oder in Form einer anderen Perverbindung, z.B. Magnesiumperoxid oder Kaliumpersulfat vorliegen kann.

Ein weiterer Gegenstand ist ein Verfahren zum Blondieren oder Färben karatiner Fasern, insbesondere menschlicher Haare umfassend
a) das Lösen einer erfindungsgemäßen Portion in Wasser oder wasserstoffperoxidhaltiger Emulsion oder Dispersion und
b) Aufbringen der in Schritt a) enthaltenen wässrigen Zubereitung auf die keratinen Fasern, vorzugsweise das menschliche Haar.

Ein weiterer Gegenstand der vorliegenden Erfindung ist die Verwendung einer erfindungsgemäßen Portion zur Behandlung keratiner Fasern, insbesondere menschlicher Haare.

Ein weiterer Gegenstand ist die Verwendung der zuvor beschriebenen wasserlöslichen und/oder wasserdispergierbaren Umhüllung zur Portionierung kosmetischer Zubereitungen. Die Oberfläche der Umhüllung weist, wie zuvor beschrieben, einen quadratischen Mittelwert der Rauheit von wenigstens 10 µm auf. Innen- als auch Außenseite der Umhüllung weisen ein durch Erhitzen und Pressen aufgeprägtes, dreidimensionales Muster auf. Unter Portionierung ist, im Rahmen der vorliegenden Erfindung, das Einteilen von stofflichen Mengen in geeignete handhabbare Größen zu verstehen, z.B., die für den einmaligen Haarfärbe- oder Haarblondiervorgang benötigte Menge. Diese Mengeneinheiten werden mittels des Hüllmaterials zu Portionen verpackt, z.B. zu wasserlöslichen Beuteln oder Kapseln.

Die Verwendung erfolgt im Rahmen dieses Gegenstandes mit einer Umhüllung, die eine dreidimensionale makroskopische Oberfläche aufweist, die mindestens 10 %, bevorzugt mindestens 20 %, weiter bevorzugt mindestens 30 % und 50 % größer als die zweidimensionale geometrische Oberfläche ist.

Ein weiterer Gegenstand der vorliegenden Erfindung ist die Verwendung einer erfindungsgemäßen Portion zum Blondieren oder Färben keratiner Fasern, insbesondere menschlicher Haare.

### Beispiele

### Beispiel 1:

Es wurde eine erfindungsgemäße Portion, enthaltend ein Blondierpulver mit der in Tabelle 1 angegebenen Zusammensetzung, hergestellt.

**Tabelle 1: Blondierpulver**

| Rohstoff | Angabe in Gew.-% |
|---|---|
| Ammoniumpersulfat | 21,5 % |
| Natriumphosphat | 4,0 % |
| Aerosil 200 | 3,0 % |
| Kaliumpersulfat | 33,0 % |
| Britesil® C 20 | 22,0 % |
| Natriumstearat | 8,0 % |
| Ceasit®I | 4,0 % |
| Magnesiumoxid | 2,0 % |
| Magnesiumhydroxidcarbonat | 1,0 % |
| Lanette® E | 1,0 % |
| Idranal® III | 0,5 % |

Es wurden die folgenden Rohstoffe verwendet:

| | |
|---|---|
| Aerosil® 200: | pyrogene Kieselsäure (INCI-Bezeichnung: Silica) (Degussa) |
| Britesil® C20: | Natriumsilicat; Molverhältnis SiO₂:Na₂O= 2,0 |
| Ceasit® I: | Calciumstearat |
| Lanette®E: | Natriumcetylstearylsulfat (ex Cognis) |
| Idranal® III: | Ethylendiamin-N,N,N',N'-tetraessigsäuredinatriumsatz |

Die Rohstoffkomponenten des Blondierpulvers werden vermischt und vermahlen, bis eine mittlere Teilchengröße von 100 µm eingestellt ist.

Das Blondierpulver wird nachfolgend mittels eines Schlauchbeutelsiegelverfahrens in eine wasserlösliche PVA-Polymerfolie (Solublon, Typ SA 20 ex Syntana) verpackt.

Die Umhüllung des Schlauchbeutels weist die folgenden Eigenschaften auf:

| | |
|---|---|
| quadratischer Mittelwert der Rauheit: | 30 µm |
| mittlere Stärke der Folie: | 20 µm |
| Folienmaterial: | teilverseiftes Polyvinylacetat mit einem Verseifungsgrad von 96 %; Gießfolie; mittleres Molekulargewicht: 36000 g/mol |

Außen- und Innenflächen der Polymerfolie sind dreidimensional strukturiert mit einem karoförmigen Muster. Das Muster wird gebildet durch ein Gitter mit quadratischen Vertiefungen, so dass die Gitternetzlinien durch die Ränder der Vertiefungen ausgebildet werden.

Die Tiefe der Vertiefung beträgt 0,12 mm.
Die aufgeprägten Karos weisen einen Durchmesser von 0,6 mm auf.

Die erfindungsgemäße Portion enthält 25 g des vorgenannten Blondierpulvers.

Die Portion wurde nachfolgend in einer Wasserstoffperoxiddispersion mit einer Zusammensetzung gemäß Tabelle 2 aufgelöst, bei 20 °C:

**Tabelle 2: Wasserstoffperoxiddispersion**

| Rohstoffe | Angaben in Gew.-% |
|---|---|
| Lorol® C16 | 3,6 % |
| Eumulgin | 0,9 % |
| Texapon® NSO | 2,25 % |
| Ammoniak (25 %) | 0,65 % |
| Dipicolinsäure | 0,1 % |
| Natriumpyrophosphat | 0,03 % |
| Turpinal® SL | 1,5 % |
| Wasserstoffperoxid | 6 % |
| Wasser | ad 100) |

Es wurden die folgenden Rohstoffkomponenten eingesetzt:

| | |
|---|---|
| Lorol® C16: | C₁₆-Fettalkohol |
| Eumulgin®: | Ceteareth-20 |
| Texapon® NSO: | Natrium-Lauryl-Ethersulfat mit 2 EO |
| Turpinal® SL: | Hydroxethyldiphosphonsäure |

Es zeigte sich, dass sich die erfindungsgemäße Portion ca. 5 mal so schnell auflöst wie die aus dem Stand der Technik bekannten Portionen mit Hüllmaterialien aus glatten wasserlöslichen Folien, vergleichbarer Stärken.

Die erfindungsgemäße Portion befindet sich in einem Kit zusammen mit den folgenden Bestandteilen:
a) eine Anmischschale
b) ein Rührstab
c) eine Kunststoffflasche mit einer Wasserstoffperoxiddispersion gemäß Tabelle 2
d) einen Conditioner

### Beispiel 2

### Rezeptur für die Oxofärbung

### Färbung mit CH-acider Komponenten und aromatischem Aldehyd

**Tabelle 3**

| Komponente 1 | | Komponente 2 | |
|---|---|---|---|
| Paraffinöl dünnfl. | 7,5 g | Cetiol® B | 7,5 g |
| Cremophor® RH 40 | 2,0 g | Dehydol® LS3 | 4,0 g |
| Rheopearl® KL | 0,5 g | Rheopearl® KL | 0,5 g |

Beide Komponenten (1 und 2) wurden auf 80 °C unter Rühren erhitzt. Bei dieser Temperatur bildeten sich in beiden Fällen klare, dünnviskose Flüssigkeiten, die sich beim Abkühlen auf Raumtemperatur zu klaren, mittelviskosen Gelen verdickten.

In Komponente 1 wurden nach dem Abkühlen
- 0,75 g Dimethylaminobenzaldehyd
- 1,2 g Methocel® E4M und
- 0,5 g Arginin
in Komponente 2
- 0,85 g 1,2-Dihydro-1,3,4,6-tetramethyl-2-oxopyridiniumchlorid und
- 3,6 g eines bei Raumtemperatur (20 °C) flüssigen C₈-C₁₀-Fettalkoholgemisches homogen dispergiert.

Es wurden die folgenden Rohstoffe eingesetzt:

| | |
|---|---|
| Cremophor RH 40: | Rizinusöl, hydriert mit 40-45 Ethylenoxideinheiten (INCI-Bezeichnung: PEG-40 Hydrogenated Castor Oil) (BASF) |
| Rheopearl®KL: | Dextrinpalmitat (Miyoshi Kasei) |
| Cetiol® B: | Di-n-butyladipat |
| Dehydol® LS 3: | Laurylalkohol-3 EO (Cognis Deutschland GmbH) |
| Methocel® E 4 M: | Hydroxypropylmethylcellulose |

Zur Herstellung einer erfindungsgemäßen Portion wurden Komponente 1 und Komponente 2 jeweils getrennt in einen wasserlöslichen Schlauchbeutel gefüllt, der die Spezifikation wie in Beispiel 1 aufweist, und anschließend thermisch flüssigkeitsdicht versiegelt.

Portion 1, enthaltend Komponente 1 und Portion 2, enthaltend Komponente 2, wurden in 80 ml Wasser von 40 °C eingerührt. Es entstand eine gut fließfähige Emulsion.

Eine mit dieser Formulierung im Gewichtsverhältnis von 4:1 30 Minuten bei 32 °C ausgefärbte Haarsträhne (Kerling naturweiß) war intensiv magentafarben nuanciert.

### Beispiel 3

### Färbung mit Oxidationsfarbstoffen

**Tabelle 4**

| | |
|---|---|
| Paraffinöl dünnflüssig | 19,50 g |
| Dehydol® LS4 | 0,25 g |
| Gelatinizalion Agent GP-1 | 0,25 g |

Es wurde der folgende Rohstoff eingesetzt:

| | |
|---|---|
| Gelatinizalion Agent GP-1: | N-Lauroyl-1-Glutaminsäure-α,γ-di-n-butylamid (Ajinomoto) |
| Dehydol® LS4: | Laurylalkohol-4 EO |

Die Herstellung des Gels erfolgte wie in Beispiel 2 beschrieben bei 80 °C und Abkühlung auf Raumtemperatur. In das Gel mit der in Tabelle 4 angegebenen Zusammensetzung wurden 1,2 g Tetraaminopyrimidinsulfat und 0,6 g Methylresorcin sowie 2 g Soda und 3 g Trinatriumphosphat homogen dispergiert.

Das Gel wurde analog Beispiel 2 in einen Schlauchbeutel mit dem in Beispiel 1 genannten Hüllmaterial abgefüllt und flüssigkeitsdicht versiegelt.

Es wurde eine 5 g Portion hergestellt die mit 20 g einer kommerziellen 6 %igen Entwickleremulsion (Poly Color Cremehaarfarbe) sowie 20 g einer 2 %igen Natrosol 250 HR-Quellung, in der 0,5 g Ammoniumsulfat gelöst waren, beim Raumtemperatur (20 °C) gemischt wurde.

Mit dieser Emulsion wurde eine blonde Haarsträhne (Kerling naturweiß) gefärbt (30 Minuten, 32 °C). Die Nuancierung der Strähne war ein leuchtendes Rot.

Es wurden die folgenden Rohstoffe eingesetzt:

| | |
|---|---|
| Natrosol 250 HR: | Hydroxyethylcellulose ex Aqualon |
| | Viskosität (1 % in H₂O): 1,5 - 2,5 Pas (20 °C) |
| | Viskosität (2 % in H₂O): 30 Pas (20 °C) |

### Beispiel 4

### Oxofärbung:

### Färbung mit aciden Carbonylverbindungen und aromatischen Aminen

**Tabelle 5**

| Komponente 1 | | Komponente 2 | |
|---|---|---|---|
| Cetiol® 868 (Isooctylstearat) | 2,00 g | Stenol® 1618 | 4,00 g |
| | | Eumulgin® B1 | 0,40 g |
| Paraffinöl, dünnflüssig | 14,00 g | Eumulgin® B2 | 0,40 g |
| | | p-Toluylendiaminsulfat | 0,88 g |
| Cutina® GMS | 2,00 g | Ammoniumsulfat | 0,20 g |
| Dehydol® LS 2 | 2,00 g | Wasser | 34,12 g |
| | | mit Ammoniak auf pH 9 | |

Es wurden die folgenden Rohstoffe eingesetzt:

| | |
|---|---|
| Dehydol® LS 2: | Laurylalkohol-2 EO (Cognis Deutschland GmbH) |
| Eumulgin® B2: | Cetylstearylalkohol mit 20 Mol Ethylenoxid (Cognis Deutschland GmbH) |
| Stenol® 1618: | C₁₆/C₁₈-Fettalkoholgemisch (Cognis Deutschland GmbH) |
| Cutina® GMS: | Glycerinmonostearat (Cognis Deutschland GmbH) |
| Eumulgin® B1: | Cetylstearylalkohol mit 12 Mol Ethylenoxid (Cognis Deutschland GmbH) |

Die Bestandteile der Komponente 1 wurde auf 80 °C erhitzt. In der heißen Mischung wurde 0,6 g N-Allylisatin gelöst. Anschließend wurde unter Rühren auf Raumtemperatur abgekühlt. Die Rezeptur wurde in einen Schlauchbeutel wie in Beispiel 2 verpackt. Nach Auflösen der Portion in Komponente 2 erfolgte die Färbung einer blonden Haarsträhne (Kerling Naturweiß, 30 Minuten, 32 °C). Die Farbe der Strähne war tizianrot.

## Patentansprüche

1. Portion umfassend eine kosmetische Zubereitung und eine wasserlösliche und/oder wasserdispergierbare Umhüllung, **dadurch gekennzeichnet, dass** die Umhüllung die kosmetische Zubereitung umhüllt und die Oberfläche der Umhüllung einen quadratischen Mittelwert der Rauheit von wenigstens 10 µm aufweist, wobei sowohl Innen- als auch Außenseite der Umhüllung ein durch Erhitzen und Pressen aufgeprägtes, dreidimensionales Muster aufweisen.

2. Portion, vorzugsweise nach Anspruch 1, umfassend eine kosmetische Zubereitung und eine wasserlösliche und/oder wasserdispergierbare Umhüllung, **dadurch gekennzeichnet, dass** die Umhüllung die kosmetische Zubereitung umhüllt und die dreidimensionale makroskopische Oberfläche mindestens 10%, bevorzugt mindestens 20 %, weiter bevorzugt zwischen 20% und 100%, äußerst bevorzugt zwischen 30% und 50 % größer als die zweidimensionale geometrische Oberfläche ist.

3. Portion nach einem der Ansprüche 1 oder 2, **dadurch gekennzeichnet, dass** die Außen- und Innenoberfläche der Umhüllung zu mindestens 50 %, vorzugsweise mindestens zu 70 %, weiter bevorzugt mindestens zu 90 % und insbesondere im Wesentlichen vollständig mit einem dreidimensionalen, aufgeprägten Muster versehen sind.

4. Portion nach einem der Ansprüche 1 bis 3, **dadurch gekennzeichnet, dass** die Umhüllung ein regelmäßiges Muster aufweist.

5. Portion nach Anspruch 4, **dadurch gekennzeichnet, dass** das regelmäßige Muster in einer periodisch wiederkehrenden Anordnung von Erhöhungen und Vertiefungen der Umhüllungsoberfläche besteht.

6. Portion nach Anspruch 4 oder 5, **dadurch gekennzeichnet, dass** das aufgeprägte Muster mindestens 4, vorzugsweise mindestens 6, besonders bevorzugt zwischen 8 und 50, weiter bevorzugt zwischen 10 und 25 Vertiefungen oder Erhöhungen pro 1 cm² der zweidimensionalen Oberfläche aufweist.

7. Portion nach einem oder mehreren der vorangehenden Ansprüche, **dadurch gekennzeichnet, dass** die Oberfläche kreisförmige und/oder dreieckige und/oder viereckige und/oder mehreckige Vertiefungen aufweist.

8. Portion nach einem oder mehreren der vorangehenden Ansprüche, **dadurch gekennzeichnet, dass** die Oberfläche quaderförmige, runde, eckige, ovale, sägezahnförmige oder dreieckig spitz zur Oberfläche laufende Erhöhungen aufweist.

9. Portion nach einem der Ansprüche 1 bis 7, **dadurch gekennzeichnet, dass** die Oberfläche der Umhüllung mit einem gitterförmigen oder wabenförmigen dreidimensionalem strukturierten Muster versehen ist.

10. Portion nach Anspruch 9, **dadurch gekennzeichnet, dass** die Gitternetzlinien durch eine Aneinanderreihung von die Vertiefung begrenzenden Rändern gebildet werden.

11. Portion nach einem der Ansprüche 9 oder 10, **dadurch gekennzeichnet, dass** das Verhältnis der durchschnittlichen Breite von Gitternetzlinie zur maximalen Ausdehnung in der Ebene der Vertiefung kleiner 20:1, vorzugsweise kleiner 10:1, besonders bevorzugt kleiner 1:1, weiter bevorzugt kleiner 0,5:1 und insbesondere kleiner 0,25:1 ist.

12. Portion nach einem oder mehreren der vorangehenden Ansprüche, **dadurch gekennzeichnet, dass** die Oberfläche der Umhüllung einen quadratischen Mittelwert der Rauheit aufweist von 10 bis 100 µm, vorzugsweise 10 bis 50 µm, insbesondere von 30 bis 35 µm.

13. Portion nach einem oder mehreren der vorangehenden Ansprüche, **dadurch gekennzeichnet, dass** das Verhältnis von durchschnittlichem Durchmesser der Vertiefung zur Tiefe der Vertiefung unterhalb 20:1, vorzugsweise 10:1 bis 1:10, insbesondere 8:1 bis 1:1, im Speziellen 6:1 bis 4:1 beträgt.

14. Portion nach einem oder mehreren der vorangehenden Ansprüche, **dadurch gekennzeichnet, dass** die mittlere Stärke der Umhüllung 10 bis 100 µm, vorzugsweise 15 bis 50 µm und insbesondere 20 bis 40 µm beträgt.

15. Portion nach einem oder mehreren der vorangehenden Ansprüche, **dadurch gekennzeichnet, dass** die wasserlösliche und/oder wasserdispergierbare Umhüllung ganz oder teilweise besteht aus einem Thermoplasten, der ausgewählt ist aus der Gruppe bestehend aus Polyvinylalkohol (PVA), acetalisiertem Polyvinylalkohol, Polyvinylpyrrolidon, Polyethylenoxid, Gelatine, Cellulose, Stärke und Derivate der vorgenannten Stoffe, und/oder Mischungen der vorgenannten Polymere, wobei Polyvinylalkohol besonders bevorzugt ist.

16. Portion nach einem oder mehreren der vorangehenden Ansprüche, **dadurch gekennzeichnet, dass** die wasserlösliche und/oder wasserdispergierbare Umhüllung zusätzlich Polymere umfasst, die ausgewählt sind aus der Gruppe bestehend aus Acrylsäure-haltigen Polymeren, Polyacrylamiden, Oxazolin- Polymeren, Polystyrolsulfonaten, Polyurethanen, Polyestern, Polyethern und/oder Mischungen der vorgenannten Polymere.

17. Portion nach einem oder mehreren der vorangehenden Ansprüche, **dadurch gekennzeichnet, dass** die wasserlösliche Umhüllung ein acetalisiertes Polyvinylalkohol mit einem Verseifungsgrad 70 bis 100 Mol-%, vorzugsweise von 80 bis 96 Moi%, besonders bevorzugt von 82 bis 94 Mol-% und insbesondere von 85 bis 89 Mol-% umfasst.

18. Portion nach einem oder mehreren der vorangehenden Ansprüche, **dadurch gekennzeichnet, dass** die wasserlösliche Umhüllung ein teilverseifter acetalisierter Polyvinylalkohol mit einem mittleren Molekulargewicht im Bereich von 10000 bis 100000 gmol⁻¹, vorzugsweise von 11000 bis 90000 gmol⁻¹, besonders bevorzugt von 12000 bis 80000 gmol⁻¹ und insbesondere von 13000 bis 70000 gmol⁻¹, im Speziellen von 20000 bis 40000 gmol⁻¹ umfasst.

19. Portion nach einem oder mehreren der vorangehenden Ansprüche, **dadurch gekennzeichnet, dass** die Umhüllung die Form eines Beutels, einer Kapsel, eines Spritzguß- oder Tiefziehformkörpers oder eines Blasformkörpers aufweist.

20. Portion nach einem oder mehreren der vorangehenden Ansprüche, **dadurch gekennzeichnet, dass** die Portion ein Innenvolumen von 5 bis 500 cm³, vorzugsweise von 10 bis 200 cm³, besonders bevorzugt von 20 bis 100 cm³, insbesondere von 30 bis 70 cm³ aufweist.

21. Portion nach einem oder mehreren der vorangehenden Ansprüche, **dadurch gekennzeichnet, dass** sich die Umhüllung in Wasser bei 20 °C innerhalb von weniger als 5 Minuten, vorzugsweise weniger als 4 Minuten, weiter bevorzugt weniger als 3 Minuten, insbesondere zwischen 2 und 0,5 Minuten vollständig auflöst.

22. Portion nach einem oder mehreren der vorangehenden Ansprüche, **dadurch gekennzeichnet, dass** die kosmetische Zubereitung ein Pulver oder eine Paste oder Emulsion oder ein Gel ist.

23. Portion nach einem oder mehreren der vorangehenden Ansprüche, **dadurch gekennzeichnet, dass** die kosmetische Zubereitung ein Haarbehandlungsmittel ist.

24. Portion nach einem oder mehreren der vorangehenden Ansprüche, **dadurch gekennzeichnet, dass** die kosmetische Zubereitung ein Blondiermittel oder ein Haarfärbemittel ist.

25. Portion nach einem oder mehreren der vorangehenden Ansprüche, **dadurch gekennzeichnet, dass** die kosmetische Zubereitung einen Wassergehalt von weniger als 20 Gew.-%, vorzugsweise von weniger als 12 Gew.-%, besonders bevorzugt von weniger als 8 Gew.-%, weiter bevorzugt von weniger als 4 Gew.-%, insbesondere von weniger als 2 Gew.-%, jeweils bezogen auf die gesamte kosmetische Zubereitung, enthält.

26. Portion nach einem oder mehreren der vorangehenden Ansprüche, **dadurch gekennzeichnet, dass** die kosmetische Zubereitung einen oder mehrere viskositätsregulierende Zusätze aufweist, die ausgewählt sind aus der Gruppe bestehend aus
a) Ester oder Amide von Di-, Tri-, Tetra- oder Polyolen, insbesondere Dextrin ein oder mehrfach verestert mit Palmitinsäure oder N-Lauroyl-1-glutaminsäure,α,γ-di-n-butylamid,
b) Ester von Di- oder Oligocarbonsäuren, insbesondere Di-behenylfumarsäureester,
c) Schichtsilikaten, vorzugsweise organisch modifizierte, insbesondere hydrophob modifizierte Schichtsilikate,
d) Mono- oder Diglyceride von C₁₂-C₂₂-Fettsäuren
e) Alkali-, Erdalkali- und Aluminiumsalze von Fettsäuren und/oder Hydroxycarbonsäuren, insbesondere die Lithiumsalze von C₃-C₁₄-Hydroxycarbonsäuren,
f) Aerosile, vorzugsweise SiO₂ und/oder TiO₂, besonders bevorzugt solche mit einer mittleren Partikelgröße unterhalb von 100 µm, insbesondere unterhalb von 100 µm,
g) Polyole, vorzugsweise Polyethylenglycole und/oder Polypropylenglycole, besonders bevorzugt Polyole mit einem mittleren Molekulargewicht unterhalb von 20000,
h) Dibenzyliden-Sorbitole sowie deren Derivate,
i) Copolymere mit Aminodithiazolen,
j) Pfropfcopolymere von Polyvinylpyridin mit sulfoniertem Polyisobutylen,
k) Vernetzte Polyamine und/oder Polyimine
l) Polymere ausgewählt aus i) Gummi-basierten Blockcopolymeren, ii) Silikonölen mit einer Viskosität oberhalb von 2000 mPas, iii) mikrokristalline Wachse und
m) Ethylen-Vinylacetat-Copolymere

27. Portion nach einem oder mehreren der vorangehenden Ansprüche, **dadurch gekennzeichnet, dass** die kosmetische Zubereitung eine oder mehrere Komponenten mit einem exothermen Löslichkeitsverhalten in Wasser aufweist, vorzugsweise ausgewählt aus der Gruppe bestehend aus
a) Alkali- oder Erdalkalisalze, vorzugsweise Alkali- oder Erdalkalihalogenide und/oder - sulfate, insbesondere Calciumchlorid und/oder Magnesiumsulfat und/oder dehydratisierte Zeolithe und
b) Niedermolekulare Polyole, vorzugsweise Glycerin, Propylenglycol oder Polyethylenglycol .

28. Portion nach einem oder mehreren der vorangehenden Ansprüche, **dadurch gekennzeichnet, dass** die kosmetische Zubereitung als Flüssigkeit, vorzugsweise als Dispersion, Emulsion, Lösung oder Gel, besonders bevorzugt mit einer Viskosität von 500 bis 40000 mPas, weiter bevorzugt 5000 bis 35000 mPas, insbesondere von 10000 bis 35000 mPas, im Speziellen von 20000 bis 32000 mPas (Brookfield- Viskosimeter LVT-II bei 4 U/min und 20°C, Spindel 5).

29. Portion nach einem oder mehreren der vorangehenden Ansprüche, **dadurch gekennzeichnet, dass** die kosmetische Zubereitung zusätzlich wenigstens ein Öl enthält, welches ausgewählt ist aus der Gruppe bestehend aus
a) Mineralöle, vorzugsweise Paraffinöl,
b) pflanzlichen Öle, vorzugsweise Sonnenblumenöl, Rapsöl, Sojabohnenöl, Rizinusöl,
c) der Silikonöle, vorzugsweise quaternisierte Silikone,
d) Ester von C₁₀-C₃₆-Fettsäuren, vorzugsweise Ester von C₁₄-C₂₈-Fettsäuren und
e) Dialkylether mit mindestens einem Kohlenstoffrest, der 6 oder mehr Kohlenstoffatome trägt.

30. Portion nach einem oder mehreren der vorangehenden Ansprüche, **dadurch gekennzeichnet, dass** die kosmetische Zubereitung aus Haarfärbemittel ausgewählt ist, bestehend aus der Gruppe der temporären Färbemittel, der semipermanenten Färbemittel und der permanenten Haarfärbemittel, insbesondere ausgewählt ist aus der Gruppe der Oxofärbemittel, Oxidationsfarbstoffe, im Speziellen Entwicklerkomponenten oder Kupplerkomponenten oder Komponente A oder Komponente B eines Oxofärbemittels.

31. Portion nach Anspruch 30, **dadurch gekennzeichnet, dass** die Haarfärbemittel, insbesondere die Komponente A des Oxofärbemittels, eine Wasserlöslichkeit unterhalb von 5 Gew.-%, vorzugsweise unterhalb von 2 Gew.-%, insbesondere unterhalb von 1 Gew.% aufweisen.

32. Portion nach einem oder mehreren der vorangehenden Ansprüche, **dadurch gekennzeichnet, dass** das kosmetische Mittel ein Blondiermittel in Pulverform ist und das Pulver eine mittlere Teilchengröße von weniger als 250 µm, vorzugsweise zwischen 50 und 150 µm aufweist.

33. Verfahren zur Herstellung einer Portion nach einem der Ansprüche 1 bis 32 umfassend die folgenden Schritte:
a) Bereitstellen eines Behälters aus einer wasserlöslichen und/oder wasserdispergierbaren Umhüllung,
b) Befüllen des Behälters mit einer kosmetischen Zubereitung und
c) Versiegeln, vorzugsweise flüssigkeitsdichtes Versiegeln, des Behälters,
wobei vor dem Schritt a) oder während der Schritte a) bis c) oder nach dem Schritt c) durch Erhitzen und Pressen auf die Außen- und Innenfläche der Umhüllung ein dreidimensionales, Muster so aufgebracht wird, dass die Oberfläche der Umhüllung einen quadratischen Mittelwert der Rauheit von wenigstens 10 µm aufweist.

34. Verfahren nach Anspruch 33, **dadurch gekennzeichnet, dass** die Herstellung der portionierten Zubereitung ganz oder teilweise nach einem Tiefziehverfahren, Spritzgussverfahren, Blasformverfahren, Rotary-Die-Verfahren oder insbesondere nach Schlauchbeutelsiegelverfahren erfolgt.

35. Verfahren nach einem der Ansprüche 33 oder 34, **dadurch gekennzeichnet, dass** die Umhüllung das aufgeprägte Muster vor dem Befüllen des Behälters mit einer kosmetischen Zubereitung erhält.

36. Kit enthaltend eine oder mehrere Portionen gemäß einem der Ansprüche 1 bis 32.

37. Kit nach Anspruch 36, enthaltend zusätzlich einen oder mehrere Bestandteile ausgewählt aus der Gruppe:
a) Behältnis zum Anmischen und Auflösen der Portion und/oder
b) ein oder mehrere Behältnis(se) enthaltend mindestens eine weitere kosmetische Zubereitung, vorzugsweise eine Wasserstoffperoxid-Lösung oder Wasserstoffperoxid-Emulsion oder Wasserstoffperoxid-Dispersion oder eine Pflegelotion; und/oder
c) eine Vorrichtung zum Vermischen oder Rühren und/oder
d) ein oder mehrere Sicherheitsmaterialien zur Vermeidung des unerwünschten Inkontakttretens der kosmetischen Zubereitung mit dem menschlichen Körper, vorzugsweise Handschuhe und/oder
e) eine oder mehrere Strähnenhaube(n) oder Strähnen-Nadel(n) und/oder
f) eine oder mehrere Mascarabürste(n) und/oder
g) einen oder mehrere Conditioner.

38. Verfahren zum Blondieren oder Färben keratiner Fasern, insbesondere menschlicher Haare, umfassend
a) das Lösen einer Portion nach einem der Ansprüche 1 bis 32 in Wasser oder Wasserstoffperoxid-haltiger Emulsion oder Dispersion und
b) Aufbringen der in Schritt a) erhaltenen wässerigen Zubereitung auf keratine Fasern, vorzugsweise menschliches Haar.

39. Verwendung einer Portion nach einem der Ansprüche 1 bis 32 zur Behandlung keratiner Fasern, insbesondere menschlicher Haare.

40. Verwendung nach Anspruch 39 **dadurch gekennzeichnet, dass** die Portion zum Blondieren oder Färben keratiner Fasern, insbesondere menschlicher Haare eingesetzt wird.

41. Verwendung einer wasserlöslichen und/oder wasserdispergierbaren Umhüllung zur Portionierung kosmetischer Zubereitungen, **dadurch gekennzeichnet, dass** die Oberfläche der Umhüllung einen quadratischen Mittelwert der Rauheit von wenigstens 10 µm aufweist, wobei sowohl Innen- als auch Außenseite der Umhüllung ein durch Erhitzen und Pressen aufgeprägtes, dreidimensionales Muster aufweisen.

42. Verwendung einer wasserlöslichen und/oder wasserdispergierbaren Umhüllung zur Portionierung kosmetischer Zubereitungen nach Anspruch 41, **dadurch gekennzeichnet, dass** die Umhüllung eine dreidimensionale makroskopische Oberfläche aufweist, die mindestens 10%, bevorzugt mindestens 20 %, weiter bevorzugt zwischen 20% und 100%, äußerst bevorzugt zwischen 30% und 50 % größer als die zweidimensionale geometrische Oberfläche ist.

## Claims

1. A portion comprising a cosmetic preparation and a water-soluble and/or water-dispersible wrapping, **characterized in that** the wrapping encases the cosmetic preparation and the surface of the wrapping has a root-mean-square roughness of at least 10 µm, both the inner and outer faces of the wrapping having a three-dimensional pattern embossed by heating and pressing.

2. A portion, preferably according to claim 1, comprising a cosmetic preparation and a water-soluble and/or water-dispersible wrapping, **characterized in that** the wrapping encases the cosmetic preparation and the three-dimensional macroscopic surface is at least 10%, preferably at least 20%, more preferably between 20% and 100%, extremely preferably between 30% and 50%, larger than the two-dimensional geometric surface.

3. The portion according to one of claims 1 or 2, **characterized in that** the outer and inner surfaces of the wrapping are at least 50%, preferably at least 70%, more preferably at least 90%, and in particular substantially completely, provided with a three-dimensional embossed pattern.

4. The portion according to one of claims 1 to 3, **characterized in that** the wrapping has a regular pattern.

5. The portion according to claim 4, **characterized in that** the regular pattern consists of a periodically repeating arrangement of raised parts and recesses of the wrapping surface.

6. The portion according to claim 4 or 5, **characterized in that** the embossed pattern has at least 4, preferably at least 6, particularly preferably between 8 and 50, more preferably between 10 and 25, recesses or raised parts per 1 cm² of the two-dimensional surface.

7. The portion according to one or more of the preceding claims, **characterized in that** the surface has circular and/or triangular and/or quadrangular and/or polygonal recesses.

8. The portion according to one or more of the preceding claims, **characterized in that** the surface has cuboid, round, square, oval or saw-tooth shaped raised parts or raised parts which taper toward the surface in the manner of a triangle.

9. The portion according to one of claims 1 to 7, **characterized in that** the surface of the wrapping is provided with a lattice-shaped or honeycomb-shaped three-dimensional structured pattern.

10. The portion according to claim 9, **characterized in that** the lattice gridlines are formed by edges adjoining the recess being arranged in series.

11. The portion according to one of claims 9 or 10, **characterized in that** the ratio of the average width of lattice gridlines to the maximum extension in the plane of the recess is less than 20:1, preferably less than 10:1, particularly preferably less than 1:1, more preferably less than 0.5:1, and in particular less than 0.25:1.

12. The portion according to one of more of the preceding claims, **characterized in that** the surface of the wrapping has a root-mean-square roughness of from 10 to 100 µm, preferably from 10 to 50 µm, in particular from 30 to 35 µm.

13. The portion according to one or more of the preceding claims, **characterized in that** the ratio of average diameter of the recess to the depth of the recess is less than 20:1, preferably from 10:1 to 1:10, in particular from 8:1 to 1:1, specifically from 6:1 to 4:1.

14. The portion according to one or more of the preceding claims, **characterized in that** the average strength of the wrapping is from 10 to 100 µm, preferably from 15 to 50 µm, and in particular from 20 to 40 µm.

15. The portion according to one or more of the preceding claims, **characterized in that** the water-soluble and/or water-dispersible wrapping consists, in whole or in part, of a thermoplastic selected from the group consisting of polyvinyl alcohol (PVA), acetalized polyvinyl alcohol, polyvinylpyrrolidone, polyethylene oxide, gelatin, cellulose, starch and derivatives of the aforementioned substances, and/or mixtures of the aforementioned polymers, polyvinyl alcohol being particularly preferred.

16. The portion according to one or more of the preceding claims, **characterized in that** the water-soluble and/or water-dispersible wrapping additionally comprises polymers selected from the group consisting of acrylic acid-containing polymers, polyacrylamides, oxazoline polymers, polystyrene sulfonates, polyurethanes, polyesters, polyethers and/or mixtures of the aforementioned polymers.

17. The portion according to one or more of the preceding claims, **characterized in that** the water-soluble wrapping comprises an acetalized polyvinyl alcohol having a saponification degree of from 70 to 100 mol.%, preferably from 80 to 96 mol.%, particularly preferably from 82 to 94 mol.%, and in particular from 85 to 89 mol.%.

18. The portion according to one or more of the preceding claims, **characterized in that** the water-soluble wrapping comprises a partially saponified acetalized polyvinyl alcohol having an average molecular weight in the range of from 10,000 to 100,000 gmol⁻¹, preferably from 11,000 to 90,000 gmol⁻¹, particularly preferably from 12,000 to 80,000 gmol⁻¹, and in particular from 13,000 to 70,000 gmol⁻¹, specifically from 20,000 to 40,000 gmol⁻¹.

19. The portion according to one or more of the preceding claims, **characterized in that** the wrapping is in the form of a pouch, a capsule, an injection molded body or deep drawing molded body, or a blow molded body.

20. The portion according to one or more of the preceding claims, **characterized in that** the portion has an internal volume of from 5 to 500 cm³, preferably from 10 to 200 cm³, particularly preferably from 20 to 100 cm³, in particular from 30 to 70 cm³.

21. The portion according to one or more of the preceding claims, **characterized in that** the wrapping completely dissolves in water at 20 °C in less than 5 minutes, preferably less than 4 minutes, more preferably less than 3 minutes, in particular between 2 and 0.5 minutes.

22. The portion according to one or more of the preceding claims, **characterized in that** the cosmetic preparation is a powder or a paste or emulsion or a gel.

23. The portion according to one or more of the preceding claims, **characterized in that** the cosmetic preparation is a hair treatment agent.

24. The portion according to one or more of the preceding claims, **characterized in that** the cosmetic preparation is a bleaching agent or a hair dye.

25. The portion according to one or more of the preceding claims, **characterized in that** the cosmetic preparation contains a water content of less than 20 wt.%, preferably of less than 12 wt.%, particularly preferably of less than 8 wt.%, more preferably of less than 4 wt.%, in particular of less than 2 wt.%, in each case based on the total cosmetic preparation.

26. The portion according to one or more of the preceding claims, **characterized in that** the cosmetic preparation has one or more viscosity-regulating additives selected from the group consisting of
a) esters or amides of diols, triols, tetraols or polyols, in particular dextrin that is mono-esterified or poly-esterified with palmitic acid or N-lauroyl-1-glutamic acid,α,γ-di-n-butyl amide,
b) esters of dicarboxylic acids or oligocarboxylic acids, in particular dibehenyl fumaric acid ester,
c) phyllosilicates, preferably organically modified, in particular hydrophobically modified, phyllosilicates,
d) monoglycerides or diglycerides of C₁₂-C₂₂ fatty acids,
e) alkali salts, alkaline-earth salts and aluminum salts of fatty acids and/or hydroxycarboxylic acids, in particular the lithium salts of C₃-C₁₄ hydroxycarboxylic acids,
f) aerosils, preferably SiO₂ and/or TiO₂, particularly preferably those having an average particle size of less than 100 µm, in particular less than 100 µm,
g) polyols, preferably polyethylene glycols and/or polypropylene glycols, particularly preferably polyols having an average molecular weight of less than 20,000,
h) dibenzylidene sorbitols and derivatives thereof,
i) copolymers with aminodithiazoles,
j) graft copolymers of polyvinylpyridine with sulfonated polyisobutylene,
k) crosslinked polyamines and/or polyimines,
l) polymers selected from i) rubber-based block copolymers, ii) silicone oils having a viscosity of greater than 2000 mPas, iii) microcrystalline waxes, and
m) ethylene vinyl acetate copolymers.

27. The portion according to one or more of the preceding claims, **characterized in that** the cosmetic preparation has one or more components having an exothermic solubility behavior in water, preferably selected from the group consisting of
a) alkali salts or alkaline-earth salts, preferably alkali or alkaline-earth halides and/or sulfates, in particular calcium chloride and/or magnesium sulfate and/or dehydrated zeolites and
b) low-molecular-weight polyols, preferably glycerol, propylene glycol or polyethylene glycol.

28. The portion according to one or more of the preceding claims, **characterized in that** the cosmetic preparation is present as a liquid, preferably as a dispersion, emulsion, solution or gel, particularly preferably having a viscosity of from 500 to 40,000 mPas, more preferably from 5000 to 35,000 mPas, in particular from 10,000 to 35,000 mPas, specifically from 20,000 to 32,000 mPas (Brookfield viscometer LVT-II at 4 rpm and 20 °C, spindle 5).

29. The portion according to one or more of the preceding claims, **characterized in that** the cosmetic preparation additionally contains at least one oil selected from the group consisting of
a) mineral oils, preferably paraffin oil,
b) vegetable oils, preferably sunflower oil, rapeseed oil, soybean oil, castor oil,
c) the silicone oils, preferably quaternized silicones,
d) esters of C₁₀-C₃₆ fatty acids, preferably esters of C₁₄-C₂₈ fatty acids and
e) dialkylethers having at least one carbon functional group which carries 6 or more carbon atoms.

30. The portion according to one or more of the preceding claims, **characterized in that** the cosmetic preparation is selected from hair dyes, consisting of the group of temporary dyes, semi-permanent dyes and permanent dyes, in particular selected from the group of oxo dyes, oxidation dyes, specifically developer components or coupler components or component A or component B of an oxo dye.

31. The portion according to claim 30, **characterized in that** the hair dye, in particular the component A of the oxo dye, has a water solubility of less than 5 wt.%, preferably less than 2 wt.%, in particular less than 1 wt.%.

32. The portion according to one or more of the preceding claims, **characterized in that** the cosmetic agent is a bleaching agent in powder form and the powder has an average particle size of less than 250 µm, preferably between 50 and 150 µm.

33. A method for preparing a portion according to one of claims 1 to 32, comprising the following steps:
a) providing a container consisting of a water-soluble and/or water-dispersible wrapping,
b) filling the container with a cosmetic preparation and
c) sealing the container, preferably so as to be liquid-tight,
wherein before step a) or during steps a) to c) or after step c), a three-dimensional pattern is applied to the outer and inner surface of the wrapping by heating and pressing such that the surface of the wrapping has a root-mean-square roughness of at least 10 µm.

34. The method according to claim 33, **characterized in that** the portioned preparation is prepared, in whole or in part, according to a deep-drawing method, injection molding method, blow molding method, rotary die method, or in particular according to a tubular pouch sealing method.

35. The method according to one of claims 33 or 34, **characterized in that** the wrapping receives the embossed pattern before the container is filled with a cosmetic preparation.

36. A kit containing one or more portions according to one of claims 1 to 32.

37. The kit according to claim 36, additionally containing one or more components selected from the group consisting of:
a) a container for blending and dissolving the portion and/or
b) one or more container(s) containing at least one further cosmetic preparation, preferably a hydrogen peroxide solution or hydrogen peroxide emulsion or hydrogen peroxide dispersion or a nourishing lotion; and/or
c) a device for mixing or stirring and/or
d) one or more safety materials for preventing undesired contact between the cosmetic preparation and the human body, preferably gloves and/or
e) one or more streaking cap(s) or streaking needle(s) and/or
f) one or more mascara brush(es) and/or
g) one or more conditioners.

38. A method for bleaching or dyeing keratin fibers, in particular human hair, comprising
a) dissolving a portion according to one of claims 1 to 32 in water or hydrogen peroxide-containing emulsion or dispersion and
b) applying the aqueous preparation obtained in step a) to keratin fibers, preferably human hair.

39. The use of a portion according to one of claims 1 to 32 for treating keratin fibers, in particular human hair.

40. The use according to claim 39, **characterized in that** the portion is used for bleaching or dyeing keratin fibers, in particular human hair.

41. The use of a water-soluble and/or water-dispersible wrapping for portioning cosmetic preparations, **characterized in that** the surface of the wrapping has a root-mean-square roughness of at least 10 µm, both the inner and outer faces of the wrapping having a three-dimensional pattern embossed by heating and pressing.

42. The use of a water-soluble and/or water-dispersible wrapping for portioning cosmetic preparations according to claim 41, **characterized in that** the wrapping has a three-dimensional macroscopic surface which is at least 10%, preferably at least 20%, more preferably between 20% and 100%, extremely preferably between 30% and 50%, larger than the two-dimensional geometric surface.

## Revendications

1. Portion comprenant une préparation cosmétique et une enveloppe hydrosoluble et/ou hydrodispersible, **caractérisée en ce que** l'enveloppe enveloppe la préparation cosmétique et la surface de l'enveloppe présente une rugosité quadratique moyenne d'au moins 10 µm, l'intérieur comme l'extérieur de l'enveloppe présentant un motif tridimensionnel gaufré par le chauffage et le pressage.

2. Portion, de préférence selon la revendication 1, comprenant une préparation cosmétique et une enveloppe hydrosoluble et/ou hydrodispersible, **caractérisée en ce que** l'enveloppe enveloppe la préparation cosmétique et la surface macroscopique tridimensionnelle est plus grande que la surface géométrique bidimensionnelle d'au moins 10%, de préférence d'au moins 20%, plus préférablement de 20 % à 100%, le plus préférablement de 30% à 50%.

3. Portion selon l'une des revendications 1 ou 2, **caractérisée en ce que** les surfaces extérieure et intérieure de l'enveloppe sont pourvues d'un motif tridimensionnel gaufré à raison d'au moins à 50 %, de préférence d'au moins 70 %, plus préférablement d'au moins 90 % et en particulier de manière sensiblement complète.

4. Portion selon l'une des revendications 1 à 3, **caractérisée en ce que** l'enveloppe présente un motif régulier.

5. Portion selon la revendication 4, **caractérisée en ce que** le motif régulier consiste en un agencement périodiquement récurrent d'élévations et de dépressions de la surface de l'enveloppe.

6. Portion selon la revendication 4 ou 5, **caractérisée en ce que** le motif gaufré présente au moins 4, de préférence au moins 6, de manière particulièrement préférée entre 8 et 50, plus préférablement entre 10 et 25 dépressions ou élévations pour 1 cm² de la surface bidimensionnelle.

7. Portion selon l'une ou plusieurs des revendications précédentes, **caractérisée en ce que** la surface présente des dépressions circulaires et/ou triangulaires et/ou carrées et/ou polygonales.

8. Portion selon l'une ou plusieurs des revendications précédentes, **caractérisée en ce que** la surface présente des élévations de forme cubique, ronde, angulaire, ovale, en dents de scie ou triangulaire.

9. Portion selon l'une des revendications 1 à 7, **caractérisée en ce que** la surface de l'enveloppe est pourvue d'un motif structuré tridimensionnel en forme de treillis ou de nid d'abeille.

10. Portion selon la revendication 9, **caractérisée en ce que** les lignes de la forme de treillis sont formées par une juxtaposition de bords délimitant les dépressions.

11. Portion selon l'une des revendications 9 ou 10, **caractérisée en ce que** le rapport de la largeur moyenne de la forme de treillis à l'étendue maximale dans le plan de la dépression est inférieur à 20:1, de préférence inférieur à 10:1, de manière particulièrement préférée inférieur à 1:1 , plus préférablement inférieur à 0,5:1 et en particulier inférieur à 0,25:1.

12. Portion selon l'une ou plusieurs des revendications précédentes, **caractérisée en ce que** la surface de l'enveloppe présente une rugosité quadratique moyenne de 10 à 100 µm, de préférence de 10 à 50 µm, en particulier de 30 à 35 µm.

13. Portion selon l'une ou plusieurs des revendications précédentes, **caractérisée en ce que** le rapport du diamètre moyen de la dépression à la profondeur de la dépression est inférieur à 20:1, de préférence de 10:1 à 1:10, en particulier de 8:1 à 1:1, notamment de 6:1 à 4:1.

14. Portion selon l'une ou plusieurs des revendications précédentes, **caractérisée en ce que** l'épaisseur moyenne de l'enveloppe est de 10 à 100 µm, de préférence de 15 à 50 µm et en particulier de 20 à 40 µm.

15. Portion selon l'une ou plusieurs des revendications précédentes, **caractérisée en ce que** l'enveloppe hydrosoluble et/ou hydrodispersible est entièrement ou partiellement constituée d'un thermoplastique choisi dans le groupe constitué par l'alcool polyvinylique (APV), l'alcool polyvinylique acétalisé, la polyvinylpyrrolidone, l'oxyde de polyéthylène, la gélatine, la cellulose, l'amidon et les dérivés des substances susmentionnées, et/ou des mélanges des polymères susmentionnés, l'alcool polyvinylique étant particulièrement préféré.

16. Portion selon l'une ou plusieurs des revendications précédentes, **caractérisée en ce que** l'enveloppe hydrosoluble et/ou hydrodispersible comprend en outre des polymères choisis dans le groupe constitué par les polymères contenant de l'acide acrylique, les polyacrylamides, les polymères d'oxazoline, les sulfonates de polystyrène, les polyuréthanes, les polyesters, les polyéthers et/ou des mélanges des polymères susmentionnés.

17. Portion selon l'une ou plusieurs des revendications précédentes, **caractérisée en ce que** l'enveloppe hydrosoluble comprend un alcool polyvinylique acétalisé ayant un degré de saponification de 70 à 100% en mole, de préférence de 80 à 96% en mole, de manière particulièrement préférée de 82 à 94% en mole et en particulier de 85 à 89% en mole.

18. Portion selon l'une ou plusieurs des revendications précédentes, **caractérisée en ce que** l'enveloppe hydrosoluble présente un alcool polyvinylique acétalisé partiellement saponifié ayant une masse moléculaire moyenne comprise entre 10 000 et 100 000 gmol⁻¹, de préférence entre 11 000 et 90 000 gmol⁻¹, de manière particulièrement préférée entre 12 000 et 80 000 gmol⁻¹ et en particulier de 13 000 à 70 000 gmol⁻¹, notamment de 20 000 à 40 000 gmol⁻¹.

19. Portion selon l'une ou plusieurs des revendications précédentes, **caractérisée en ce que** l'enveloppe présente la forme d'un sac, d'une capsule, d'un corps moulé par injection ou thermoformé ou d'un corps moulé par soufflage.

20. Portion selon l'une ou plusieurs des revendications précédentes, **caractérisée en ce que** la portion présente un volume interne de 5 à 500 cm³, de préférence de 10 à 200 cm³, de manière particulièrement préférée de 20 à 100 cm³, en particulier de 30 à 70 cm³.

21. Portion selon l'une ou plusieurs des revendications précédentes, **caractérisée en ce que** l'enveloppe se dissout totalement dans l'eau à 20 °C en moins de 5 minutes, de préférence en moins de 4 minutes, plus préférablement en moins de 3 minutes, en particulier entre 2 et 0,5 minutes.

22. Portion selon l'une ou plusieurs des revendications précédentes, **caractérisée en ce que** la préparation cosmétique est une poudre ou une pâte ou une émulsion ou un gel.

23. Portion selon l'une ou plusieurs des revendications précédentes, **caractérisée en ce que** la préparation cosmétique est un agent de traitement des cheveux.

24. Portion selon l'une ou plusieurs des revendications précédentes, **caractérisée en ce que** la préparation cosmétique est un agent d'éclaircissement ou un agent de coloration capillaire.

25. Portion selon l'une ou plusieurs des revendications précédentes, **caractérisée en ce que** la préparation cosmétique contient une teneur en eau inférieure à 20% en poids, de préférence inférieure à 12% en poids, de manière particulièrement préférée inférieure à 8% en poids, plus préférablement inférieure à 4% en poids, en particulier inférieure à 2% en poids, dans chaque cas par rapport à la préparation cosmétique totale.

26. Portion selon l'une ou plusieurs des revendications précédentes, **caractérisée en ce que** la préparation cosmétique présente un ou plusieurs additifs régulateurs de viscosité, choisis dans le groupe constitué par
a) les esters ou les amides de di-, tri-, tétra- ou polyols, en particulier la dextrine estérifiée une ou plusieurs fois avec de l'acide palmitique ou le α,γ-di-n-butylamide d'acide N-lauroyl-1-glutamique,
b) les esters d'acides di- ou oligocarboxyliques, en particulier les esters di-béhényl d'acide fumarique,
c) les phyllosilicates, de préférence organiquement modifiés, en particulier les phyllosilicates modifiés hydrophobiquement,
d) les mono- ou diglycérides d'acides gras C₁₂-C₂₂,
e) Sels alcalins, alcalino-terreux et d'aluminium d'acides gras et/ou d'acides hydroxycarboxyliques, en particulier les sels de lithium d'acides hydroxycarboxyliques C₃-C₁₄,
f) les aérosols, de préférence SiO₂ et/ou TiO₂, de manière particulièrement préférée ceux ayant une granulométrie moyenne inférieure à 100 µm, en particulier inférieure à 100 µm,
g) les polyols, de préférence les polyéthylènes glycols et/ou les polypropylènes glycols, de manière particulièrement préférée les polyols ayant un poids moléculaire moyen inférieur à 20 000,
h) les dibenzylidènes sorbitols et leurs dérivés,
i) les copolymères avec des aminodithiazoles,
j) les copolymères greffés de polyvinylpyridine avec du polyisobutylène sulfoné,
k) les polyamines et/ou les polyimines réticulés,
l) les polymères choisis parmi i) les copolymères séquencés à base de caoutchouc, ii) les huiles de silicone ayant une viscosité supérieure à 2000 mPas, iii) les cires microcristallines et
m) les copolymères d'éthylène-acétate de vinyle

27. Portion selon l'une ou plusieurs des revendications précédentes, **caractérisée en ce que** la préparation cosmétique présente un ou plusieurs composants ayant un comportement de solubilité exothermique dans l'eau, de préférence choisi dans le groupe constitué par
a) les sels alcalins ou alcalino-terreux, de préférence les halogénures et/ou les sulfates alcalins ou alcalino-terreux, en particulier le chlorure de calcium et/ou le sulfate de magnésium et/ou les zéolithes déshydratées et
b) les polyols de bas poids moléculaire, de préférence le glycérol, le propylène glycol ou le polyéthylène glycol.

28. Portion selon l'une ou plusieurs des revendications précédentes, **caractérisée en ce que** la préparation cosmétique est un liquide, de préférence une dispersion, une émulsion, une solution ou un gel, de manière particulièrement préférée avec une viscosité comprise entre 500 et 40 000 mPas, plus préférablement entre 5 000 et 35 000 mPas, en particulier de 10 000 à 35 000 mPas, notamment de 20 000 à 32 000 mPas (viscosimètre Brookfield LVT-II à 4 tr/min et 20°C, broche 5).

29. Portion selon l'une ou plusieurs des revendications précédentes, **caractérisée en ce que** la préparation cosmétique contient en outre au moins une huile choisie dans le groupe constitué par
a) les huiles minérales, de préférence l'huile de paraffine,
b) les huiles végétales, de préférence l'huile de tournesol, l'huile de colza, l'huile de soja, l'huile de ricin,
c) les huiles de silicone, de préférence les silicones quaternisées,
d) les esters d'acides gras C₁₀-C₃₆, de préférence les esters d'acides gras C₁₄-C₂₈ et
e) les éthers dialkyliques ayant au moins un radical carboné portant 6 atomes de carbone ou plus.

30. Portion selon l'une ou plusieurs des revendications précédentes, **caractérisée en ce que** la préparation cosmétique est choisie parmi les colorants capillaires constitués du groupe des agents de coloration provisoires, des agents de coloration semi-permanents et des agents de coloration capillaire permanents, notamment choisis dans le groupe des agents oxydants de coloration, des colorants d'oxydation, notamment des composants révélateurs ou des composants de coupleur ou des composants A ou des composants B d'un agent oxydant de coloration.

31. Portion selon la revendication 30, **caractérisée en ce que** les agents de coloration capillaire, en particulier le composant A de l'agent oxydant de coloration, présentent une solubilité dans l'eau inférieure à 5% en poids, de préférence inférieure à 2% en poids, en particulier inférieure à 1% en poids.

32. Portion selon l'une ou plusieurs des revendications précédentes, **caractérisée en ce que** l'agent cosmétique est un agent d'éclaircissement sous forme de poudre et la poudre présente une granulométrie moyenne inférieure à 250 µm, de préférence comprise entre 50 et 150 µm.

33. Procédé pour la production d'une portion selon l'une quelconque des revendications 1 à 32, comprenant les étapes suivantes :
a) la fourniture d'un récipient d'un enveloppe hydrosoluble et/ou hydrodispersible,
b) le remplissage du récipient avec une préparation cosmétique et
c) l'étanchéification du récipient, de préférence l'étanchéification aux fluides,
dans lequel avant l'étape a) ou pendant les étapes a) à c) ou après l'étape c), par le chauffage et le pressage des surfaces externe et interne de l'enveloppe, un motif tridimensionnel est appliqué de telle sorte que la surface de l'enveloppe présente une rugosité quadratique moyenne d'au moins 10 µm.

34. Procédé selon la revendication 33, **caractérisé en ce que** la production de la préparation proportionnée a lieu totalement ou en partie par un procédé de thermoformage, un procédé d'injection, un procédé de moulage par soufflage, un procédé de matrice de rotation ou en particulier par un procédé de marquage du sac tubulaire.

35. Procédé selon l'une des revendications 33 ou 34, **caractérisé en ce que** l'enveloppe reçoit le motif gaufré avant le remplissage du récipient d'une préparation cosmétique.

36. Kit contenant une ou plusieurs portions selon l'une des revendications 1 à 32.

37. Kit selon la revendication 36, contenant en outre un ou plusieurs composants choisis dans le groupe composé :
du contenant pour mélanger et dissoudre la portion et/ou
a) d'un ou de plusieurs récipient(s) contenant au moins une autre préparation cosmétique, de préférence une solution de peroxyde d'hydrogène ou une émulsion de peroxyde d'hydrogène ou une dispersion de peroxyde d'hydrogène ou une lotion de soin ; et/ou
b) d'un dispositif de mélange ou d'agitation et/ou
c) d'un ou de plusieurs matériau(x) de sécurité pour empêcher l'entrée en contact indésirable de la préparation cosmétique avec le corps humain, de préférence des gants et/ou
d) d'un ou de plusieurs bonnet(s) à mèches ou d'une ou de plusieurs aiguille(s) à mèches et/ou
e) d'une ou de plusieurs brosse(s) à mascara et/ou
f) d'un ou de plusieurs conditionneur(s).

38. Procédé d'éclaircissement ou de coloration des fibres kératiniques, en particulier des cheveux humains, comprenant
a) la dissolution d'une portion selon l'une quelconque des revendications 1 à 32 dans de l'eau ou une émulsion ou une dispersion contenant du peroxyde d'hydrogène et
b) l'application de la préparation aqueuse obtenue à l'étape a) sur les fibres kératiniques, de préférence les cheveux humains.

39. Utilisation d'une portion selon l'une quelconque des revendications 1 à 32 pour le traitement des fibres kératiniques, en particulier des cheveux humains.

40. Utilisation selon la revendication 39, **caractérisée en ce que** la portion pour l'éclaircissement ou la coloration des fibres kératiniques, en particulier des cheveux humains, est utilisée.

41. Utilisation d'une enveloppe hydrosoluble et/ou hydrodispersible pour le portionnement de préparations cosmétiques, **caractérisée en ce que** la surface de l'enveloppe présente une rugosité quadratique moyenne d'au moins 10 µm, l'intérieur comme l'extérieur du boîtier présentant un motif tridimensionnel gaufré par chauffage et par pressage.

42. Utilisation d'un revêtement hydrosoluble et/ou hydrodispersible pour le portionnement de préparations cosmétiques selon la revendication 41, **caractérisée en ce que** l'enveloppe présente une surface macroscopique tridimensionnelle plus grande que la surface géométrique bidimensionnelle d'au moins 10%, de préférence d'au moins 20%, plus préférablement de 20% à 100%, et le plus préférablement de 30% à 50%.
